# EUROPEAN PATENT APPLICATION

(11) **EP 3 978 027 A1**
(43) Date of publication of application: **06.04.2022**
(21) Application number: 20814628.2
(22) Date of filing: 29.05.2020
(51) Int. Cl.: A61K 47/64, A61K 47/61, A61K 31/4188, A61K 9/00, A61P 3/10

(54) **PHYSIOLOGICALLY ACTIVE SUBSTANCE BOUND TO BIOTIN MOIETY, AND COMPOSITION FOR ORAL ADMINISTRATION INCLUDING SAME**

(30) Priority: 31.05.2019 KR 20190064370
(71) Applicant: D&D Pharmatech Inc., Seongnam-si, Gyeonggi-do 13486 (KR)
(72) Inventor: SHIN, Jaehee, Seoul 05610 (KR); JEON, Ok-cheol, Seoul 08786 (KR); PARK, Eun Ji, Seoul 06503 (KR)
(74) Representative: Potter Clarkson
(86) International application number: PCT/KR2020/007053
(87) International publication number: WO 2020/242268

(57) **Abstract**

The present invention relates to: a physiologically active substance to which a biotin moiety is bound; and a composition for oral administration comprising same. A physiologically active substance to which a biotin moiety is bound according to the present invention has the effect of having excellent oral absorption into the body.

<110>     D&D Pharmatech Inc.

<120>     Physiological active materials linked biotin moiety and
          composition for oral administration comprising the same

<130>     PB2020-128

<150>     KR 10-2019-0064370
<151>     2019-05-31

<160>     17

<170>     KoPatentIn 3.0

<210>     1
<211>     29
<212>     PRT
<213>     Artificial Sequence

<220>
<223>     Physiological acitve materials


<220>
<221>     MISC_FEATURE
<222>     (2)
<223>     Xaa is aminoisobutyric acid (Aib)


<400>     1
His Xaa Gln Gly Thr Phe Thr Ser Asp Tyr Ser Lys Tyr Leu Asp Glu
  1               5                  10                  15

Gln Ala Ala Lys Glu Phe Val Gln Trp Leu Met Asn Thr
             20                  25



<210>     2
<211>     30
<212>     PRT
<213>     Artificial Sequence

<220>
<223>     Physiological acitve materials


<400>     2
His Ala Glu Gly Thr Phe Thr Ser Asp Val Ser Ser Tyr Leu Glu Gly
  1               5                  10                  15

Gln Ala Ala Lys Glu Phe Ile Ala Trp Leu Val Lys Gly Arg
             20                  25                  30


<210>     3
<211>     33
<212>     PRT
<213>     Artificial Sequence

<220>
<223>     Physiological acitve materials

<400>    3
His Ala Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp

<210>    4
<211>    42
<212>    PRT
<213>    Artificial Sequence

<220>
<223>    Physiological acitve materials

<400>    4
Tyr Ala Glu Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln
        35                  40

<210>    5
<211>    39
<212>    PRT
<213>    Artificial Sequence

<220>
<223>    Physiological acitve materials

<400>    5
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210>    6
<211>    34
<212>    PRT
<213>    Artificial Sequence

<220>
<223>    Physiological acitve materials

<400>    6
Ser Val Ser Glu Ile Gln Leu Met His Asn Leu Gly Lys His Leu Asn
1               5                   10                  15

Ser Met Glu Arg Val Glu Trp Leu Arg Lys Lys Leu Gln Asp Val His
            20                  25                  30

Asn Phe

<210>   7
<211>   28
<212>   PRT
<213>   Artificial Sequence

<220>
<223>   Physiological acitve materials

<400>   7
His Ser Gln Gly Thr Phe Thr Ser Asp Tyr Ser Lys Tyr Leu Asp Ser
 1               5                  10                  15

Arg Arg Ala Gln Asp Phe Val Gln Trp Leu Met Asn
            20                  25

<210>   8
<211>   30
<212>   PRT
<213>   Artificial Sequence

<220>
<223>   Physiological acitve materials

<220>
<221>   MISC_FEATURE
<222>   (2)
<223>   Xaa is aminoisobutyric acid (Aib)

<400>   8
His Xaa Gln Gly Thr Phe Thr Ser Asp Tyr Ser Lys Tyr Leu Asp Glu
 1               5                  10                  15

Gln Ala Ala Lys Glu Phe Val Gln Trp Leu Met Asn Thr Cys
            20                  25                  30

<210>   9
<211>   31
<212>   PRT
<213>   Artificial Sequence

<220>
<223>   Physiological acitve materials

<400>   9
His Ala Glu Gly Thr Phe Thr Ser Asp Val Ser Ser Tyr Leu Glu Gly
 1               5                  10                  15

Gln Ala Ala Lys Glu Phe Ile Ala Trp Leu Val Lys Gly Arg Cys
            20                  25                  30

<210>    10
<211>    34
<212>    PRT
<213>    Artificial Sequence

<220>
<223>    Physiological acitve materials


<400>    10
His Ala Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp Cys




<210>    11
<211>    43
<212>    PRT
<213>    Artificial Sequence

<220>
<223>    Physiological acitve materials


<400>    11
Tyr Ala Glu Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln Cys
        35                  40


<210>    12
<211>    40
<212>    PRT
<213>    Artificial Sequence

<220>
<223>    Physiological acitve materials


<400>    12
His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser Cys
        35                  40

<210>    13
<211>    35
<212>    PRT
<213>    Artificial Sequence

<220>
<223>    Physiological acitve materials

<400>    13
Ser Val Ser Glu Ile Gln Leu Met His Asn Leu Gly Lys His Leu Asn
1               5                   10                  15

Ser Met Glu Arg Val Glu Trp Leu Arg Lys Lys Leu Gln Asp Val His
            20                  25                  30

Asn Phe Cys
        35

<210>    14
<211>    30
<212>    PRT
<213>    Artificial Sequence

<220>
<223>    Physiological acitve materials

<400>    14
His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr Cys
            20                  25                  30

<210>    15
<211>    21
<212>    PRT
<213>    Artificial Sequence

<220>
<223>    Physiological acitve materials

<400>    15
Gly Ile Val Glu Gln Cys Cys Thr Ser Ile Cys Ser Leu Glu Gln Leu
1               5                   10                  15

Glu Asn Tyr Cys Asn
            20

<210>    16
<211>    30
<212>    PRT
<213>    Artificial Sequence

<220>
<223>    Physiological acitve materials

<400>    16
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr
            20                  25                  30

<210>    17
<211>    21
<212>    PRT
<213>    Artificial Sequence

<220>
<223>    Physiological acitve materials

<400>    17
Gly Ile Val Glu Gln Cys Cys Thr Ser Ile Cys Ser Leu Tyr Gln Leu
1               5                   10                  15

Glu Asn Tyr Cys Asn
            20

## Description

### Technical Field

The present invention relates to a physiologically active substance bonded to a biotin moiety, and a composition for oral administration including the same. More specifically, the present invention relates to a physiologically active substance bonded to a biotin moiety having excellent oral absorption into the body, and a composition for oral administration including the same.

### Background Art

Recently, through economic progress and the accelerated growth of science and technology, diets are becoming westernized and the consumption of high-calorie, high-fat food products is increasing. Accordingly, the population afflicted by diabetes and obesity, etc. due to various metabolic diseases, is growing rapidly.

Diabetes is a disease with various complications and whose management requires a restricted diet that substantially lowers patient quality of life. Awareness of diabetes treatment and management is increasing, and the development of therapeutic agents for improvement or treatment of diabetes is urgent.

Diabetes is categorized as 'Type I' diabetes, which is caused by the inability to produce insulin, or as 'Type II' diabetes, wherein insulin production is normal but insulin resistance causes reduced ability to regulate the metabolism. Type II diabetes and obesity are mutual risk factors to each other. They are causes of metabolic disease and increase the risk of atherosclerosis, a major cause of death for diabetes patients, making them both extremely dangerous diseases.

Recently, glucagon derivatives are attracting attention. Glucagon is produced by the pancreas when blood glucose levels begin to drop due to causes such as drug therapy, illness, hormones or enzyme deficiency. Glucagon signals the liver to decompose glycogen to release glucose, raising blood glucose to normal levels. In addition to its hyperglycemic effect, glucagon has been reported to suppress appetite and activate the hormone-sensitive lipases of adipose cells to promote fat decomposition and exhibit an anti-obesity effect. One derivative of glucagon, glucagon-like peptide-1 (GLP-1), is a substance under development as a therapeutic agent reducing hyperglycemia in diabetes patients and whose functions include promoting insulin synthesis and secretion, inhibiting glucagon secretion, suppressing gastric emptying, promoting use of glucose, and inhibiting food intake.

Exendin-4, made from lizard venom which has approximately 50% amino acid homology with GLP-1, is reported to also activate GLP-1 receptors to reduce hyperglycemia of diabetes patients.

However, problems with peptide and protein drugs when orally administered is their decomposition due to enzyme attack, and low intestinal membrane permeability.

### Detailed Description of the Invention

### Problem to be Solved by the Invention

The purpose of the present invention is to provide a physiologically active substance bonded to a biotin moiety having excellent oral absorption into the body, and a composition for oral administration including the same.

### Means for Solving the Problem

One aspect of the present invention provides a physiologically active substance bonded to a biotin moiety, and a method for preparing the same.

Another aspect of the present invention provides a composition for oral administration comprising a physiologically active substance bonded to a biotin moiety.

Yet another aspect of the present invention provides a pharmaceutical composition comprising a physiologically active substance bonded to a biotin moiety.

Yet another aspect of the present invention provides a pharmaceutical composition for preventing or treating diabetes, the composition comprising a physiologically active substance bonded to a biotin moiety.

Yet another aspect of the present invention provides a pharmaceutical composition for preventing or treating obesity, the composition comprising a physiologically active substance bonded to a biotin moiety.

Yet another aspect of the present invention provides a pharmaceutical composition for preventing or treating osteoporosis, the composition comprising a physiologically active substance bonded to a biotin moiety.

Yet another aspect of the present invention provides a pharmaceutical composition for preventing or treating fatty liver, the composition comprising a physiologically active substance bonded to a biotin moiety.

Yet another aspect of the present invention provides a pharmaceutical composition for preventing or treating irritable bowel syndrome, the composition comprising a physiologically active substance bonded to a biotin moiety.

Yet another aspect of the present invention provides a pharmaceutical composition for preventing or treating neurodegenerative disease, the composition comprising a physiologically active substance bonded to a biotin moiety.

### Effects of the Invention

The physiologically active substance bonded to a biotin moiety according to one embodiment of the present invention may be bonded to a water-soluble biotin to have excellent oral absorption into the body.

The physiologically active substance bonded to a biotin moiety according to one embodiment of the present invention may defend against separation of peptides and other physiologically active substances from enzymes, ultimately promoting permeation of the intestinal membrane by physiologically active substances and their absorption in the intestines.

The physiologically active substance bonded to a biotin moiety according to one embodiment of the present invention, by being bonded to biotin, a type of water-soluble vitamin B7, may be absorbed by active transport through a sodium-dependent multivitamin transporter.

According to one embodiment of the present invention, the biotin moiety may be bonded to a non-active region of a physiologically active substance, and thereby not inhibit the activity of the physiologically active substance.

### Brief Description of the Drawings

FIG. 1 is a purification chromatogram of Embodiment 3 of the present invention.
FIG. 2 is a chromatogram for the final substance of Embodiment 1 through Embodiment 3 of the present invention.
FIG. 3 is a MALDI-TOF mass spectrum of Embodiment 1 through Embodiment 3 of the present invention.
FIG. 4 is a graph illustrating blood concentration of Embodiment 1 through Embodiment 3 by hour following oral administration to a rat.
FIG. 5 is a graph illustrating blood glucose change following glucose administration to each specimen.
FIG. 6 is a graph illustrating blood glucose change after administering glucose to each specimen.
FIG. 7 is a graph illustrating blood glucose change after administering glucose to each specimen.
FIG. 8 is a graph illustrating blood glucose change after administering glucose to each specimen.
FIG. 9 is a purification chromatogram of Embodiment 10 of the present invention.
FIG. 10 is a post-purification chromatogram of Embodiment 10 of the present invention.
FIG. 11 is a reverse phase chromatogram of Embodiment 11 of the present invention.
FIG. 12 is a MALDI-TOF mass spectrum of Embodiment 10 and Embodiment 11 of the present invention.
FIG. 13 is a purification chromatogram of Embodiment 12 and Embodiment 13 of the present invention.
FIG. 14 is a post-purification chromatogram of Embodiment 12 of the present invention.
FIG. 15 is a post-purification chromatogram of Embodiment 13 of the present invention.

### Best Mode(s) for Carrying Out the Invention

In the following, examples and embodiments of the present invention will be described in detail to allow a person having ordinary skill in the art to readily carry out the present invention.

However, the present invention may be carried out in various different forms, and is not limited to the examples and embodiments described herein. Throughout the specification of the present invention, when a certain part is said to "include" a certain element, unless specifically stated to the contrary, this does not exclude other elements but means that other elements may be further included.

The terms "about" and "substantially," etc. used throughout the specification as terms referring to degree are used to refer to numerical values at or near any unique manufacturing or material tolerances stated, and are used to prevent unfair use by an infringer of disclosures mentioning accurate or absolute numerical values provided to aid in the understanding of the present invention. The terms "step wherein" or "step of' used throughout the present invention do not mean "step for."

Throughout the specification of the present invention, the term "combinations thereof' used in a Markush claim refers to a mixture or combination of at least one of a group comprised of the elements stated in the expression of the Markush claim, the mixture or combination including at least one selected from the group comprised of these elements. Throughout the specification of the present invention, "and/or B" means "and B, or A or B."

One aspect of the present invention provides a physiologically active substance bonded to a biotin moiety, and a method for preparing the same. The physiologically active substance bonded to a biotin moiety according to one aspect of the present invention may have excellent oral absorption into the body.

Generally, peptide and protein drugs are highly water soluble and fall under Class 3 of the BCS (Biopharmaceutical Classification System), whose intestinal absorption is limited. Peptide and protein drugs are characterized in that they are highly hydrophilic and have large molecular weights, in that they can be decomposed by low-pH gastric acid, and in that they are attacked by enzymes such as trypsin to have low absorption in the intestines. Peptide and protein drugs generally have an oral bioavailability (BA) of around 0.1%, making them difficult to use as pharmaceutical compositions. Whereas enteric coated capsuled are being used to pass [such drugs] through the stomach to overcome this problem, this technology has the problem of not being able to fundamentally improve peptide and protein absorption.

On the other hand, the pharmaceutically active substance bonded to a biotin moiety according to one embodiment of the present invention, having improved intestinal membrane permeability, may have improved absorption in the intestines.

More specifically, the physiologically active substance bonded to a biotin moiety according to one embodiment of the present invention, by being bonded to biotin, a type of water-soluble vitamin B7, may be absorbed by active transport through a sodium-dependent multivitamin transporter.

In the present invention, "unsubstituted or substituted" means [something] may be unsubstituted or may be substituted. "Substituted" means having one or more substituents, and a substituent refers to a chemical portion covalently bonded or fused to an arbitrary atom in a parent group such as an alkylene or heteroalkylene.

In the present invention, "halo" means fluorine, chlorine, bromine or iodine, etc.

In the present invention, "alkyl" means a monovalent portion obtained by removing a hydrogen atom from a carbon atom of an aliphatic or alicyclic, saturated or unsaturated hydrocarbon compound, examples of which include methyl, ethyl, propyl, butyl, pentyl, hexyl, n-propyl, n-butyl, n-pentyl, isopropyl, isobutyl, sec-butyl, tert-butyl, isopentyl and neopentyl.

In the present invention, "heteroalkyl" is an alkyl including at least one heteroatom, and a heteroatom means an alkyl wherein a heteroatom is located in the position of an arbitrary carbon atom in an alkyl, substituting C, CH, CH₂ or CH₃.

In the present invention, "alkylene" refers to a bivalent portion obtained by removing a hydrogen atom from a carbon atom of an aliphatic or alicyclic, saturated or unsaturated hydrocarbon compound.

In the present invention, "heteroalkylene" refers to an alkylene including at least one heteroatom.

In the present invention, "aryl" refers to a monovalent portion obtained by removing a hydrogen atom from the aromatic ring atoms of an aromatic compound having ring atoms. For example, in "C₅₋₁₀ aryl" the carbon has 5 to 10 ring atoms, and this is the monovalent portion obtained by removing a hydrogen atom from the aromatic ring atoms of an aromatic compound. Example of an aryl include benzene, acenaphthene, fluorene, phenalene, acephenanthrene and groups derived from aceanthrene.

In the present invention, "heteroalyl" is an aryl including at least one heteroatom, for example, pyridine, pyrimidine, benzothiophene, furyl, dioxalanyl, pyrrolyl, oxazolyl, pyridyl, pyridazinyl, pyrimidinyl, isobenzofurane, indole, isoindole, indolizine, indoline, isoindoline, purine, benzodioxane, quinoline, isoquinoline, quinolizine, benzoxazine, benzothiazine, pyridopyridine, quinoxaline, quinazoline, cinnoline, phthalazine, naphthyridine, pteridine, perimidine, pyridoindole, oxanthrene, phenoxathiine, phenazine, and phenoxazine, etc.

In the present invention, "arylene" refers to a bivalent portion obtained by removing a hydrogen atom from the aromatic ring atoms of an aromatic compound having ring atoms.

In the present invention, "heteroarylene" refers to an arylene including at least one heteroatom.

In the present invention, "alkenyl" refers to an alkyl having at least one carbon-carbon double bond, examples of which include vinyl (-CH=CH₂), 1-propenyl (-CH=CHCH₃), isopropenyl, butenyl, pentenyl, and hexenyl.

In the present invention, "alkinyl" refers to an alkyl group having at least one carbon-carbon triple bond, examples of which include ethinyl and 2-propinyl.

According to one embodiment of the present invention, the biotin moiety may be represented by General Formula A below.

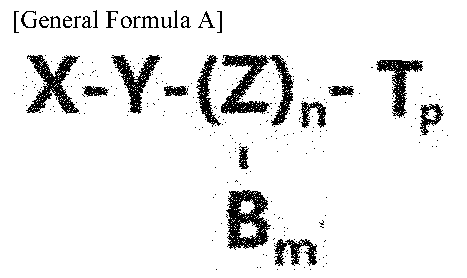

In General Formula A,
X is a functional group capable of bonding to a physiologically active substance,
Y is a spacer,
Z is a bonding unit,
B can be represented by the following Chemical Formula A-1, 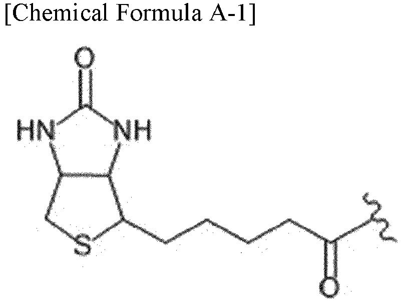
Z is connected to the 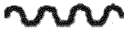 of chemical formula A-1,
T is a terminal group,
m is an integer from 1 to 10,
n is 0 or an integer from 1 to 10, and when n=0, Y is directly bonded to B or T,
and p is an integer of 0 to 1.

In one embodiment of the present invention, in general formula A, X is a functional group capable of bonding with a physiologically active substance. Although not limited hereto, the functional group may include functional groups able to react with a thiol group, carboxyl group and/or amine group, for example maleimide, succinimide, N-hydroxysuccinimide, aldehyde or a carboxyl group.

In one embodiment of the present invention, the functional group X may maintain its structure, or be dropped or modified when bonding to a physiologically active substance.

The Y is a spacer and may have a structure having in-vivo cleavability. For example, whereas it is not limited hereto, Y may correspond to a direct bond, or include in its structure at least one from a group comprised of substituted or unsubstituted C₁₋₅₀ linear alkylene, substituted or unsubstituted C₁₋₅₀ nonlinear alkylene, substituted or unsubstituted C₁₋₅₀ linear heteroalkylene, substituted or unsubstituted C₁₋₅₀ nonlinear heteroalkylene, substituted or unsubstituted C₁₋₅₀ arylene, substituted or unsubstituted C₁₋₅₀ heteroarylene, -O-, -C(O), -C(O)NR-, -C(O)O-, -S-, -NR- or - NOR-, where R may be hydrogen, substituted or unsubstituted C₁₋₅₀ alkyl, substituted or unsubstituted C₁₋₅₀ aryl, or an ethylene glycol repeating unit (-(CH₂CH₂O)ₙ-, where n is an integer of at least 1 and no more than 20).

The Z is a bonding unit capable of bonding to B, and may include, for example, although not limited hereto, an amino acid, polypeptide, alkylene, amine, or polyamidoamine structure.

Although not limited hereto, the amino acid may include lysine, 5-hydroxylysine, 4-oxalicine, 4-thialysine, 4-selenalysine, 4-thiahomolysine, 5,5-dimethyllysine, 5,5-difluorolysine, trans-4-dihydrolysine, 2,6-diamino-4-hexinoic acid, cis-4-dihydrolysine, 6-N-methylysine, diminopimelic acid, ornithine, 3-methylornithine, α-methylornithine, citrulline, homocitrulline, arginine, aspartate, asparagine, glutamate, glutamine, histidine, ornithine, proline, serine, or threonine.

If the n is 0, B or T may bond directly with Y (spacer).

The T is a terminal group, and although not limited hereto, may be, for example, hydrogen or NH2.

If the p is 0, the B may be the terminal.

According to one aspect of the present invention, in General Formula A below, m may be an integer of 1 to 10, and specifically may be an integer or 1 to 8, 1 to 5, and 1 to 4.

In one aspect of the present invention, the X may be selected from a group comprised of maleimide, succinimide, N-hydroxysuccinimide, succinimidyl succinate, succinimidyl glutarate, succinimidyl methyl ester, succinimidyl pentyl ester, Succinimidyl carbonate, p-nitrophenyl carbonate, aldehyde, amine, thiol, oxyamine, iodoacetamide, aminoxyl, hydrazide, hydroxy, propionate, pyridyl, alkyl halide, vinylsulfone, carboxyl, Hydrazide, halogen acetamide, C₂₋₅ alkynyl, C₆₋₂₀ aryldisulfide, C₅₋₂₀ heteroaryldisulfide, isocyanate, thioester, iminoester, and derivatives thereof.

In one specific aspect of the present invention, X is maleimide, N-hydroxysuccinimide, aldehyde or amine.

In one aspect of the present invention, Y is absent, a substituted or unsubstituted linear or branched C₁₋₅₀ alkylene, a substituted or unsubstituted linear or branched C₁₋₅₀ heteroalkylene, substituted or unsubstituted C₆₋₅₀ arylene, or substituted or unsubstituted C₆₋₅₀ heteroarylene, and if substituted, includes at least one selected from a group comprised of =O, -C(O)NH₂, -OH, -COOH, - SH, =NH and -NH₂.

In one aspect of the present invention, Y is a substituted linear or branched C₁₋₅₀ heteroalkylene, and includes at least one -C(O)-.

In one aspect of the present invention, Y is -(C(O))_{q}-(CH₂)ᵣ-(C(O)NHₛ-(CH₂)ᵣ-(OCH₂CH₂)ₜ-(C(O))_{q}-, where q, r, s and t are independently selected, q and S are 0 or 1, r is an integer of 1 to 20, and t is an integer of 0 to 20.

In one aspect of the present invention, Y is -(CH₂)ᵣC(O)NHNH-, where r is an integer of 1 to 20.

In one aspect of the present invention, Y includes -C(O)-.

In one aspect of the present invention, Y includes -C(O)NH-.

In one aspect of the present invention, Z is any one of the following, and each may be independently selected.
A) forms an amino acid or a derivative thereof together with X or separately from X; or
B) is a substituted or unsubstituted linear or nonlinear C₁₋₅₀ heteroalkylene,

And when substituted, includes at least one selected from a group comprised of =O, - C(O)NH₂, -OH, -COOH, -SH, =NH and -NH₂.

In one aspect of the present invention, Z is connected through B and -NH-.

In one aspect of the present invention, Z is a hydrophilic amino acid or a derivative thereof.

Specifically, in one aspect of the present invention, Z may be selected from a group comprised of lysine, arginine, histidine, glutamine, asparagine, threonine, cysteine, serine and derivatives thereof.

In one aspect of the present invention, Z includes at least one glycerol, and at least one polyethylene glycol or a bond thereof.

In one aspect of the present invention, Z includes 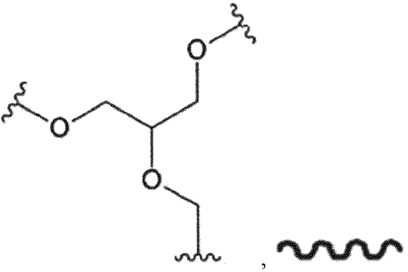 represents a bonding site, at least one 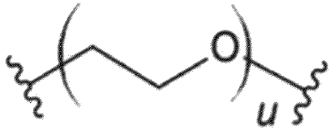 is bonded to the bonding site, and u is an integer of 1 to 20.

In one aspect of the present invention, Z includes 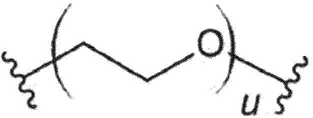 , and - (CH₂)₃NH- is further bonded to the

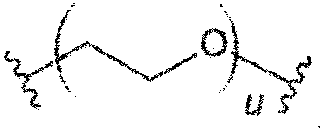

In one aspect of the present invention, T may be selected from a group comprised of amine, C₁₋₈ alkyl, C₁₋₈ alkenyl, halo, hydroxy, thiol, sulfonic acid, carboxyl, phenyl, benzyl, aldehyde, azide, cyanate, isocyanate, thiocyanate, isothiocyanate, nitrile and phosphonic acid.

In a specific aspect of the present invention, T is an amine.

In one aspect of the present invention, the biotin moiety is selected from a group comprised of: 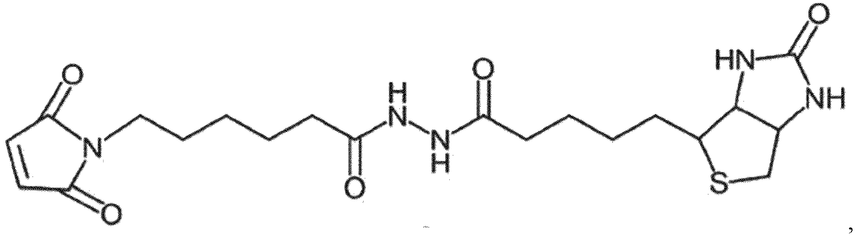 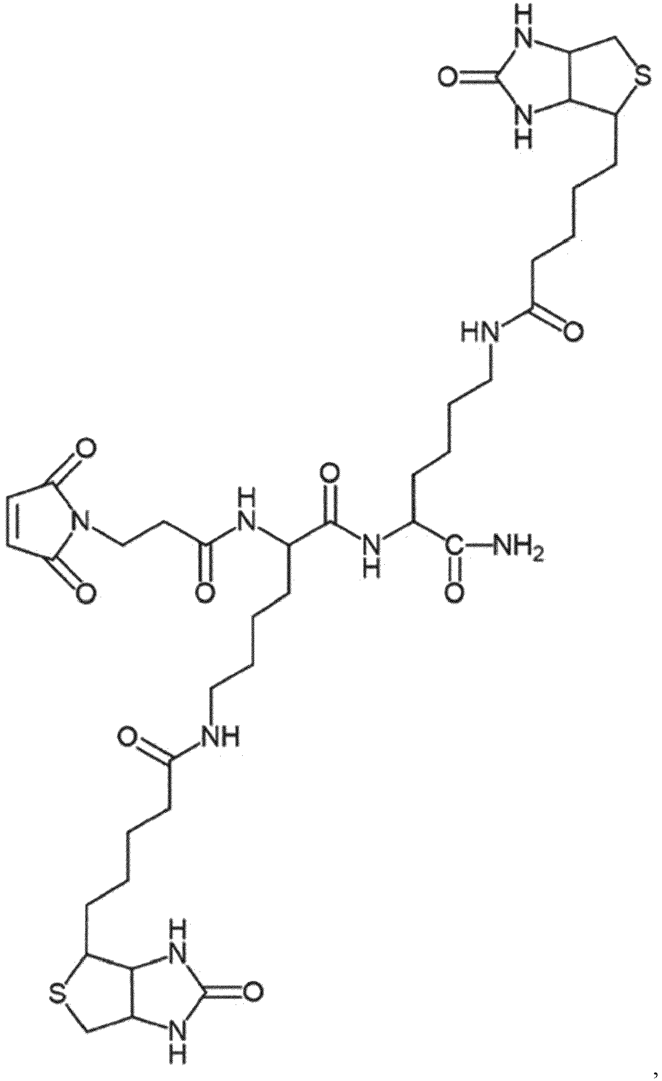 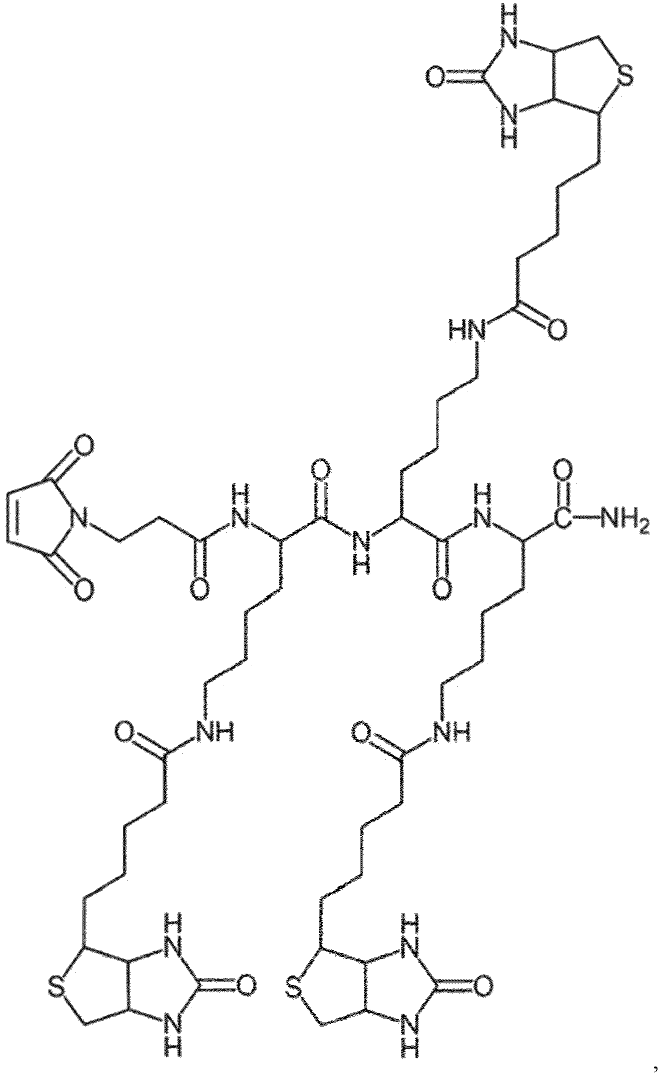 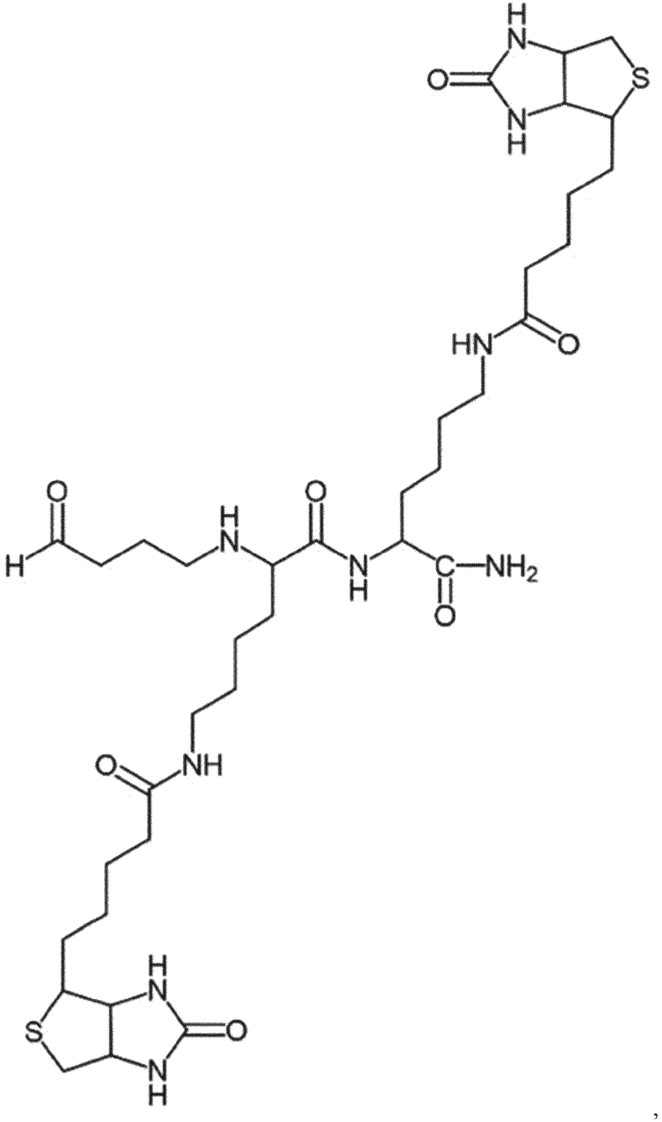 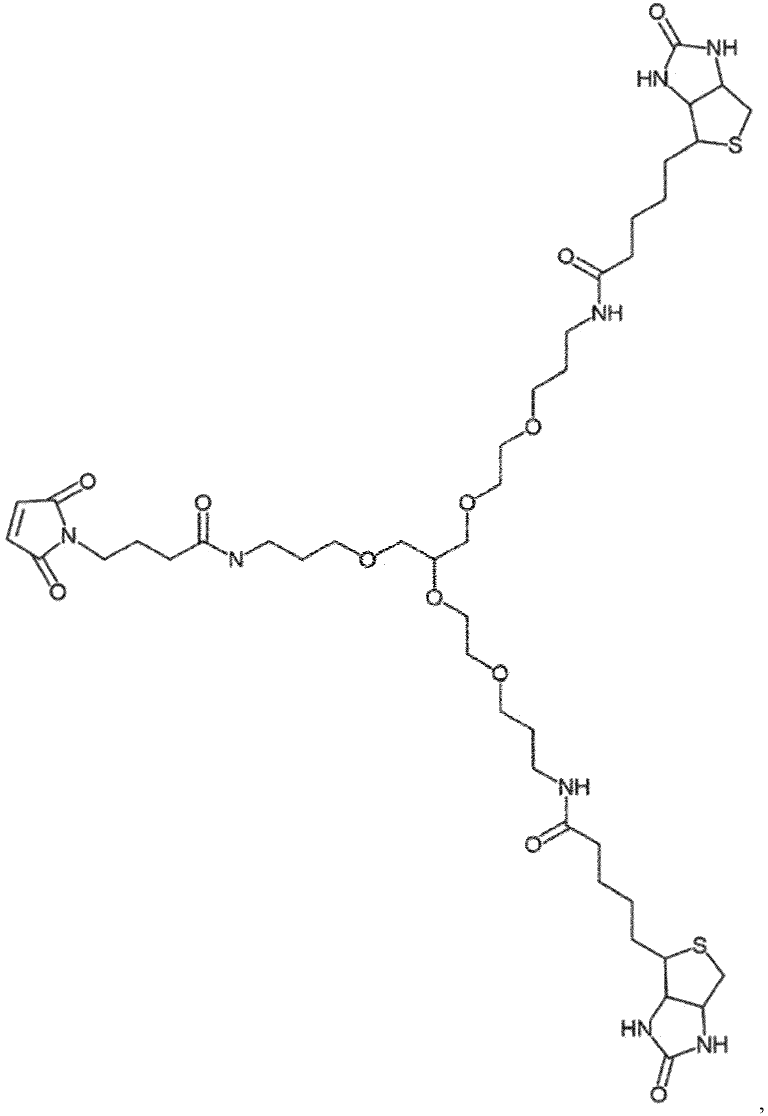 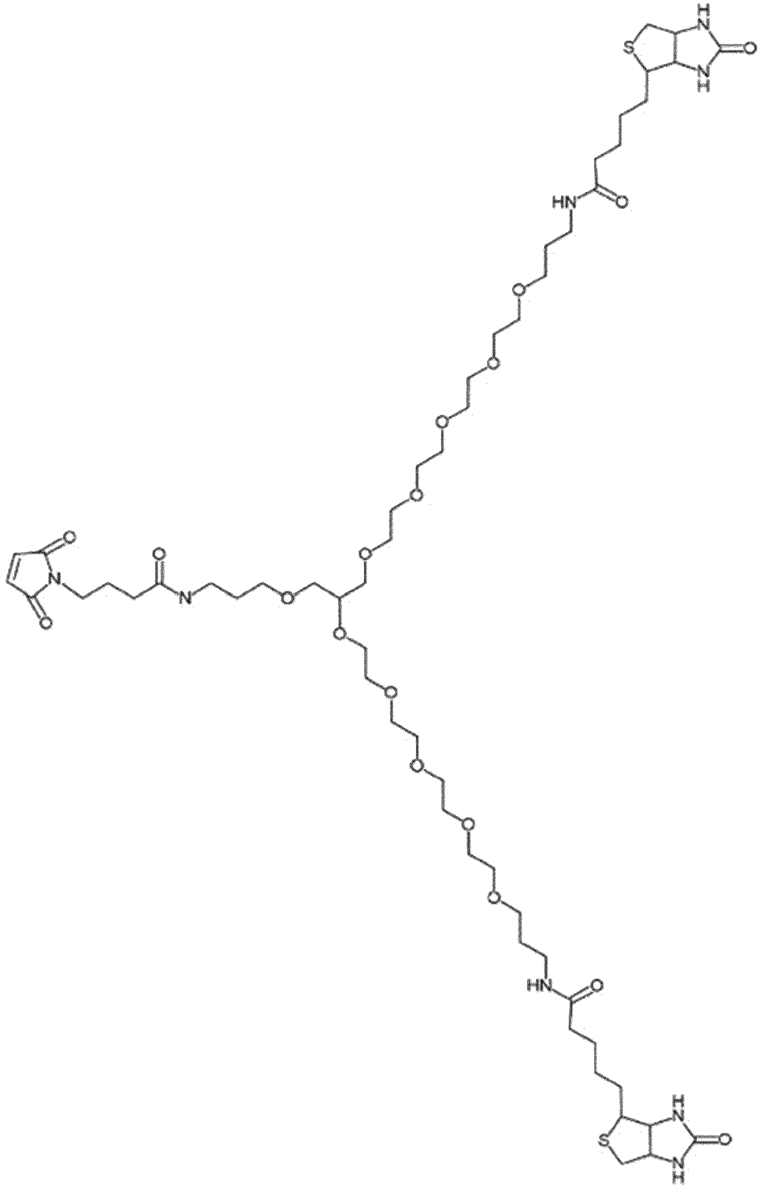 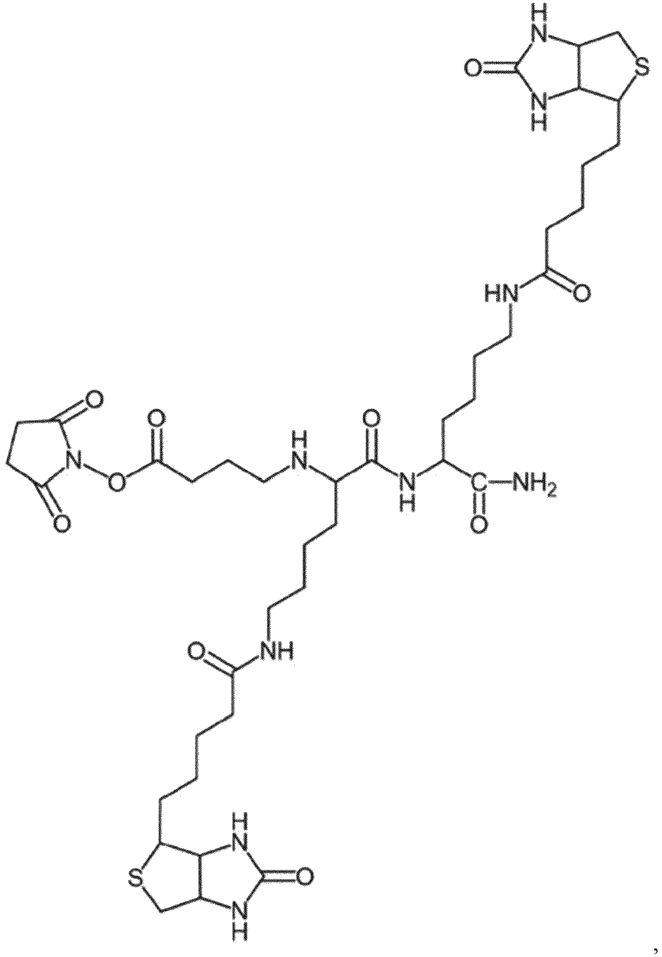 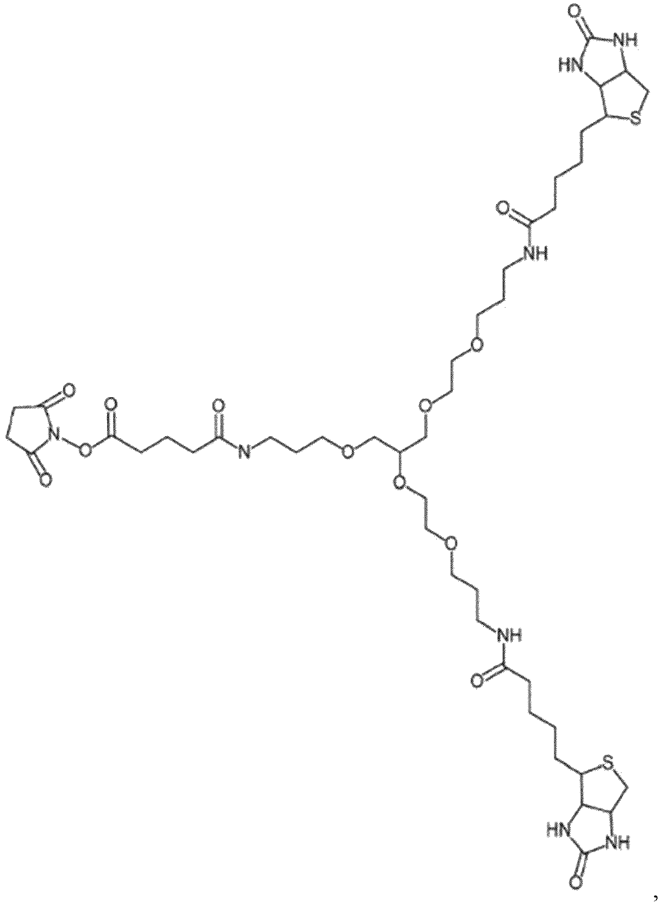 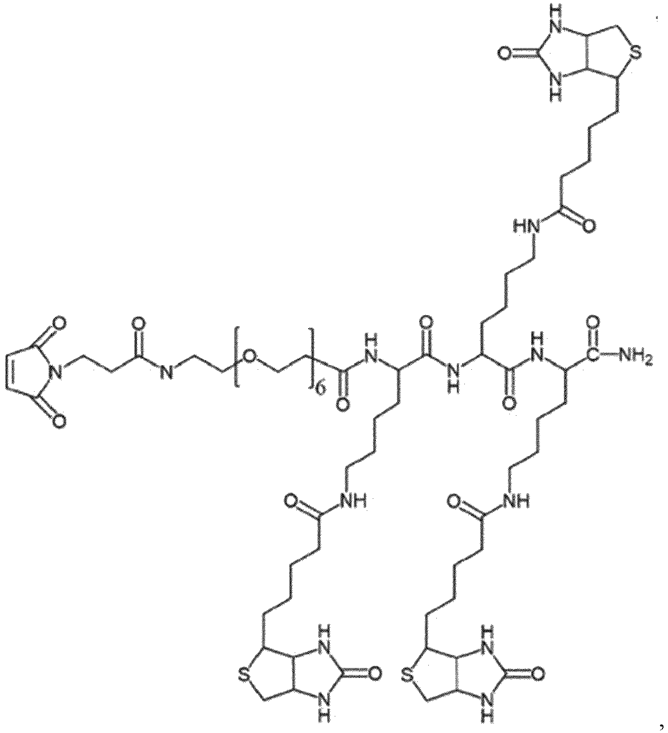 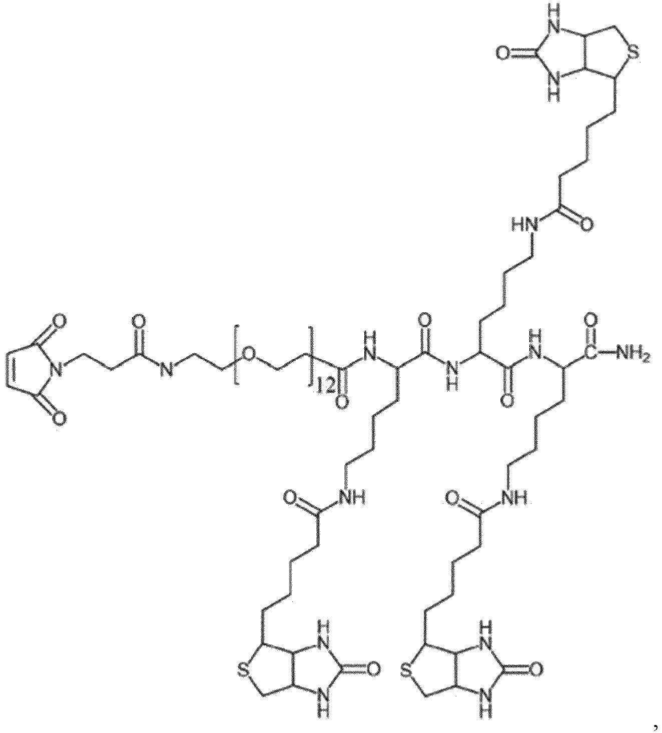 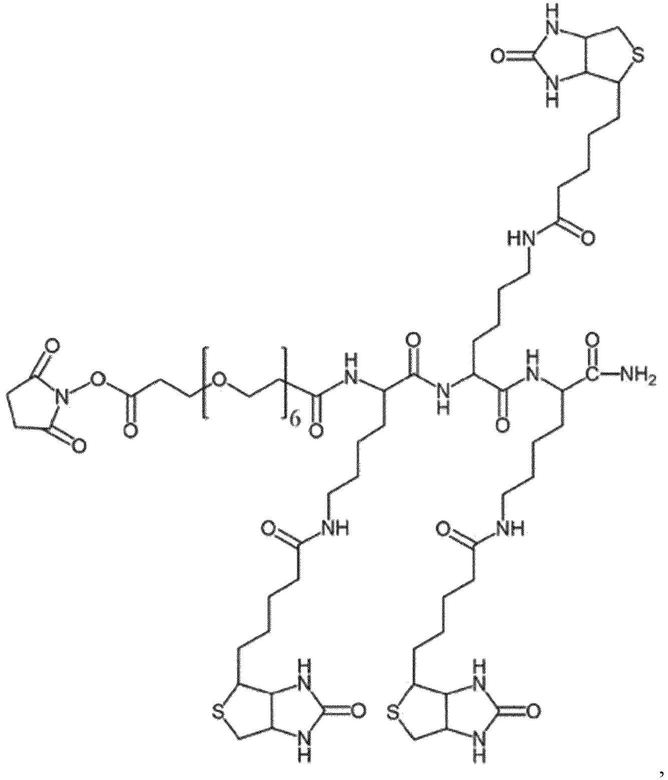 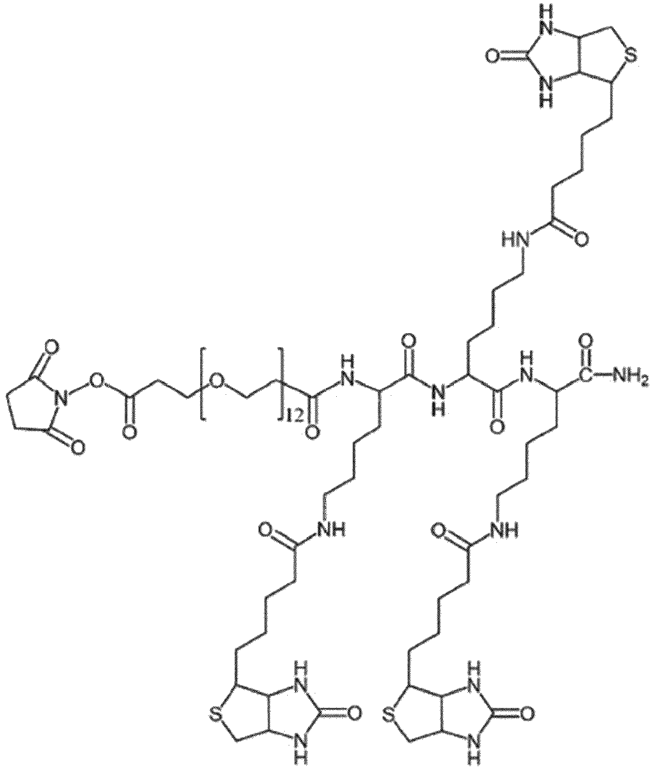 and

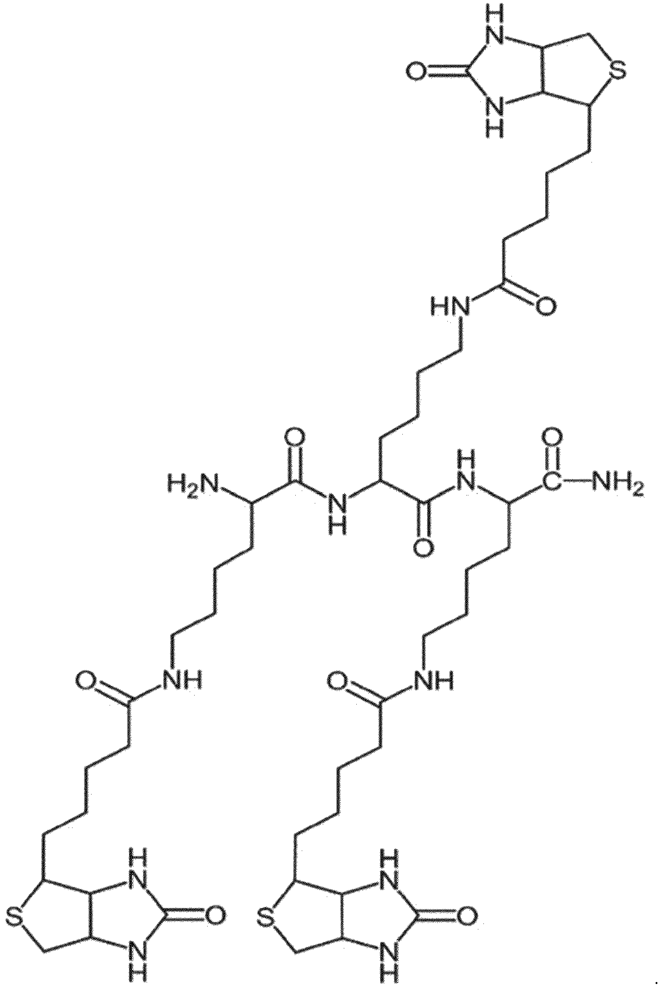

According to one embodiment of the present invention, the biotin moiety and the physiologically active substance may be bonded through various bonds. The bond may be formed by a functional group of the biotin moiety bonding to a functional group of the physiologically active substance, and although not limited hereto, the bond may be, for example, be a thiol-ether bond or amide bond.

In one example, the bond between the biotin moiety and the physiologically active substance may be formed by the method of Reaction Formula 1 below. In Reaction Formula 1 below, 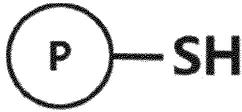 represents a physiologically active substance including a thiol group, and represents a reaction between the biotin moiety including a maleimide group according to one embodiment of the present invention and the thiol group (-SH) of a cysteine residue that exists in a physiologically active substance.

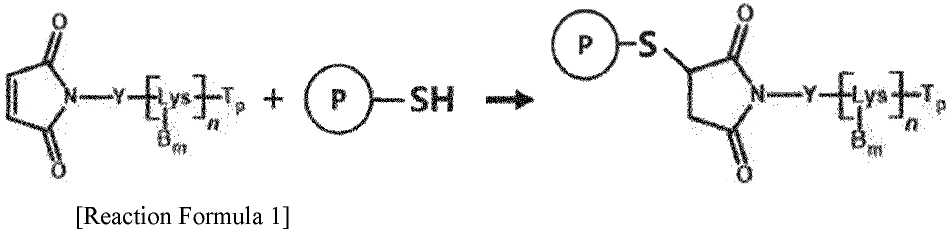

In one example, the bond between the biotin moiety and the physiologically active substance may be formed by the method of Reaction Formula 2 below. In Reaction Formula 2, 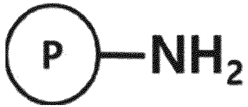 represents a physiologically active substance including an amine group, and represents a reaction between the biotin moiety including N-hydroxysuccinimide according to one embodiment of the present invention and the amine group (-NH₂) that exists in a physiologically active substance.

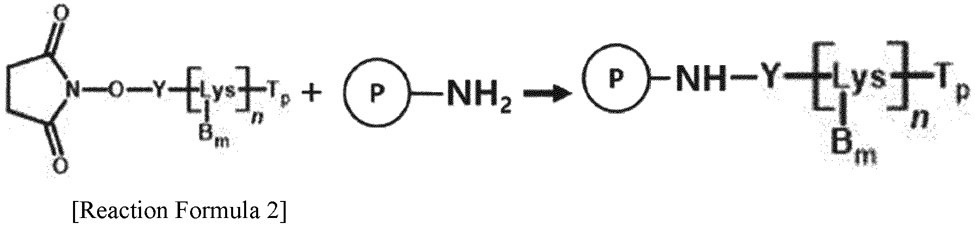

According to one embodiment of the present invention, there may be no particular limit on the physiologically active substance.

In the present invention, a physiologically active substance means a polymer substance which may be administered in the body for a specific purpose.

According to one embodiment of the present invention, the physiologically active substance may be a substance used in a pharmaceutical composition. Whereas it is not limited to these, [the substance] may be a substance used in, for example, the prevention or treatment of diabetes, the prevention or treatment of obesity, the prevention or treatment of osteoporosis, the prevention or treatment of fatty liver, the prevention or treatment of irritable bowel syndrome, or the prevention or treatment of neurodegenerative disease.

According to one embodiment of the present invention, the physiologically active substance, although not limited to these, may be, for example, a polypeptide, a protein, a polysaccharide, or derivatives thereof. The physiologically active substance, although not limited to these, may be, for example, glucagon, GLP-1 (glucagon-like peptide-1), GLP-2 (glucagon-like peptide-2), GIP (glucose-dependent insulinotropic polypeptide), exending-4, insulin, parathyroid hormone, interferon, erythropoietin, calcitonin, serotonin, rituximab, trastzumab, uricase, tissue plasminogen activator, thymoglobin, vaccine, heparin or heparin analogue, antithrombin III, filgrastim, pramlintide acetate, exenatide, eptifibatide, antivenin, IgG, IgM, HGH, thyroxin, coagulation factor VII and VIII, monoclonal antibody, glycolipid acting as a therapeutic agent, and derivatives thereof.

According to one embodiment of the present invention, the biotin moiety may bond to an inactive region of a physiologically active substance.

The biotin moiety may bond to an inactive region of a physiologically active substance to not inhibit the biological activity of the physiologically active substance, and accordingly exhibit biological activity identical to that of or improved over that of the physiologically active substance.

Although not limited to these, the PSA may, for example, may include an exposed -SH group on the inactive region and a biotin moiety may bond to the -SH group. Further, the PSA may include an exposed -NH₃⁺ group or -NH₂ group on the inactive region and a biotin moiety may bond to the exposed -NH₃⁺ group or -NH₂ group. Further, the PSA may include an exposed -N terminal on the inactive region and a biotin moiety may bond to the exposed N- terminal. IN the case of a polypeptide, the PSA may be bonded by a -NH₃+ group, -NH₂ group of a lysine amino acid of the inactive region, or, if the N-terminal of the polypeptide is an inactive region, bonded by the N-terminal, so that activity of the polypeptide is not inhibited.

That is, according to one embodiment of the present invention, the position at which a biotin moiety is bonded to a PSA may be adjusted so that the bond avoids a site exhibiting activity.

According to one embodiment of the present invention, the PSA may be a polypeptide having the amino acid sequence of any one of SEQ. ID. No. 1 through 7 below, or a derivative thereof.
SEQ. ID. NO. 1: H(Aib)QGTFTSDYSKYLDEQAAKEFVQWLMNT
SEQ. ID. NO. 2: HAEGTFTSDVSSYLEGQAAKEFIAWLVKGR
SEQ. ID. NO. 3: HADGSFSDEMNTILDNLAARDFINWLIQTKITD
SEQ. ID. No. 4: YAEGTFISDYSIAMDKIHQQDFVNWLLAQKGKKNDWKHNITQ
SEQ. ID. No. 5: HGEGTFTSDLSKQMEEEAVRLFIEWLKNGGPSSGAPPS
SEQ. ID No. 6: SVSEIQLMHNLGKHLNSMERVEWLRKKLQDVHNF
SEQ. ID. No. 7: HSQGTFTSDYSKYLDSRRAQDFVQWLMN

Further, the PSA may be a protein having the amino acid sequence of SEQ ID. No. 15 and 16 below, or a protein having the amino acid sequence of SEQ. ID. No. 17 and 16, where the protein is bonded through a disulfide bond between the sixth and eleventh cysteine of SEQ. ID. No. 15 or 17, the seventh cysteine of SEQ. ID. No. 15 or 17 and the seventh cysteine of SEQ. ID. No. 16, and the twentieth cysteine of SEQ. ID. No. 15 or 17 and the nineteenth cysteine of SEQ. ID. No. 16.
SEQ. ID. No. 15: GIVEQCCTSICSLEQLENYCN
SEQ. ID. No. 16: FVNQHLCGSHLVEALYLVCGERGFFTPKT
SEQ. ID No. 17: GIVEQCCTSICSLYQLENYCN

According to one embodiment of the present invention, cysteine may be substituted or inserted into the polypeptide to adjust the bonding site with the biotin moiety.

As a non-limiting example, any at least one of the amino acids of an inactive region of the polypeptide selected from a group comprised of the amino acid sequences represented by SEQ. ID. No. 1 through 7 above may be substituted by a cysteine amino acid, or a cysteine amino acid may be inserted thereat. Here, the biotin moiety is bonded to an -SH group of the cysteine amino acid. Further, the polypeptide into which the cysteine amino acid is inserted may be a polypeptide having the amino acid sequence of any one of SEQ. ID. No. 8 through 14.
SEQ. ID. No. 8: H(Aib)QGTFTSDYSKYLDEQAAKEFVQWLMNTC
SEQ. ID. No. 9: HAEGTFTSDVSSYLEGQAAKEFIAWLVKGRC
SEQ. ID. No. 10 : HADGSFSDEMNTILDNLAARDFINWLIQTKITDC
SEQ. ID. No. 11:
   YAEGTFISDYSIAMDKIHQQDFVNWLLAQKGKKNDWKHNITQC
SEQ. ID. No. 12: HGEGTFTSDLSKQMEEEAVRLFIEWLKNGGPSSGAPPPSC
SEQ. ID. No. 13: SVSEIQLMHNLGKHLNSMERVEWLRKKLQDVHNFC
SEQ. ID. No. 14: HSQGTFTSDYSKYLDSRRAQDFVQWLMNTC

According to one embodiment of the present invention, the PSA having a biotin moiety may covalently bond with or form a microsphere with at least one selected from a group comprised of a peptide and non-peptide polymer, fatty acid, cholesterol, antibody, antibody fragment, albumin and fragment thereof, nucleotide, fibronectin, transferrin, FcRn bonding substance, saccharide, elastin, heparin and derivatives thereof.

The non-peptide polymer may be selected from a group comprised of polyethylene glycol (PEG), polypropylene glycol, copolymer of ethylene glycol and propylene glycol, polyoxyethylated polyol, polyvinyl alcohol (PVA), polysaccharide, dextran, polyvinylethyl ether, PLA (polylactic acid), PLGA (polylactic-co-glycolic acid), lipid polymer, chitin, hyaluronic acid and combinations thereof.

Another aspect of the present invention provides a method for preparing a PSA bonded to a biotin moiety, the method comprised of obtaining a biotin moiety; reacting the biotin moiety and a PSA; and, a step of isolating the PSA bonded with the biotin moiety after completion of the reaction.

According to one embodiment of the present invention, in obtaining a biotin moiety, the biotin moiety may be represented by General Formula A above.

According to one embodiment of the present invention, in reacting the mixture, the reaction molar ratio of the biotin moiety to the PSA may be 0.5 or greater. Specifically, the molar ratio of the biotin moiety to the PSA may be 0.5 to 5. An appropriate reaction molar ratio may be selected based on the molecular structure of the biotin moiety, molecular weight, solubility, pH of the reaction solution, reaction temperature, and reaction time, etc.

According to one embodiment of the present invention, the reaction may be carried out using a buffer solution or an organic solvent. There is no particular restriction on the buffer solution or organic solvent, and a buffer solution commonly used in the art may be appropriately selected depending on the structure of the biotin moiety.

In one embodiment of the present invention, the temperature and duration of the reaction step may be appropriate adjusted depending on the characteristics of the biotin moiety and PSA used. Although not limited to these, [the reaction] may be carried out, for example, for at least three hours at 4°C, or for a shorter duration at room temperature. This may be associated with the degree of reactivity of the biotin moiety used. After a suitable reaction time has passed, the reaction may be stopped by lowering the pH of the reaction solution.

According to one embodiment of the present invention, a step of removing unreacted material may be carried out after the reaction step. The method for removing unreacted material may be a method commonly used in the art. Whereas the method is not limited to these, [the unreacted material] may be removed through, for example, dialysis using a suitable buffer solution, for example, PBS (phosphate buffered saline).

According to one embodiment of the present invention, [the method] may include a purification step after the isolating step. The isolation and purification step may be carried out using size-exclusion chromatography, high performance liquid chromatography, or ion exchange chromatography, but is not limited to these.

Another aspect of the present invention provides a composition for oral composition, the composition including the PSA bonded to a biotin moiety described in the above.

The physiologically active substance bonded to a biotin moiety according to one embodiment of the present invention, by being bonded to biotin, a type of water-soluble vitamin B7, may be absorbed by active transport through a sodium-dependent multivitamin transporter, promoting intestinal membrane permeability and absorption in the intestine.

Yet another aspect of the present invention provides a pharmaceutical composition including the PSA bonded to a biotin moiety described in the above. The use of the pharmaceutical composition may be determined depending on the type of the PSA. Further, the pharmaceutical composition may be a composition for oral administration.

According to one embodiment of the present invention, a pharmaceutical composition for preventing or treating diabetes, the composition including a PSA bonded to a biotin moiety, may be provided.

The PSA may be used for preventing or treating diabetes. Although not limited to these, for example, the PSA may be a polypeptide having the amino acid sequence of SEQ. ID. No. 1 to 14, a protein having the amino acid sequence of SEQ. ID. No. 15 and 16, a protein having the amino acid sequence of SEQ. ID. No. 17 and 16, or a derivative thereof.

Further, the protein is bonded through a disulfide bond between the sixth and eleventh cysteine of SEQ. ID. No. 15 or 17, the seventh cysteine of SEQ. ID. No. 15 or 17 and the seventh cysteine of SEQ. ID. No. 16, and the twentieth cysteine of SEQ. ID. No. 15 or 17 and the nineteenth cysteine of SEQ. ID. No. 16.

According to one embodiment of the present invention, a pharmaceutical composition for preventing or treating obesity, the composition including a PSA bonded to a biotin moiety, may be provided.

The PSA may be used for preventing or treating obesity. Although not limited to these, for example, the PSA may be a polypeptide having the amino acid sequence of SEQ. ID. No. 1 to 14, a protein having the amino acid sequence of SEQ. ID. No. 15 and 16, a protein having the amino acid sequence of SEQ. ID. No. 17 and 16, or a derivative thereof.

Further, the protein is bonded through a disulfide bond between the sixth and eleventh cysteine of SEQ. ID. No. 15 or 17, the seventh cysteine of SEQ. ID. No. 15 or 17 and the seventh cysteine of SEQ. ID. No. 16, and the twentieth cysteine of SEQ. ID. No. 15 or 17 and the nineteenth cysteine of SEQ. ID. No. 16.

According to one embodiment of the present invention, a pharmaceutical composition for preventing or treating fatty liver, the composition including a PSA bonded to a biotin moiety, may be provided.

The PSA may be used for preventing or treating fatty liver. Although not limited to these, for example, the PSA may be a polypeptide having the amino acid sequence of SEQ. ID. No. 1 to 14, a protein having the amino acid sequence of SEQ. ID. No. 15 and 16, a protein having the amino acid sequence of SEQ. ID. No. 17 and 16, or a derivative thereof.

Further, the protein is bonded through a disulfide bond between the sixth and eleventh cysteine of SEQ. ID. No. 15 or 17, the seventh cysteine of SEQ. ID. No. 15 or 17 and the seventh cysteine of SEQ. ID. No. 16, and the twentieth cysteine of SEQ. ID. No. 15 or 17 and the nineteenth cysteine of SEQ. ID. No. 16.

According to one embodiment of the present invention, a pharmaceutical composition for preventing or treating irritable bowel syndrome, the composition including a PSA bonded to a biotin moiety, may be provided.

The PSA may be used for preventing or treating irritable bowel syndrome. Although not limited to these, for example, the PSA may be a polypeptide having the amino acid sequence of SEQ. ID. No. 1 to 14, a protein having the amino acid sequence of SEQ. ID. No. 15 and 16, a protein having the amino acid sequence of SEQ. ID. No. 17 and 16, or a derivative thereof.

Further, the protein is bonded through a disulfide bond between the sixth and eleventh cysteine of SEQ. ID. No. 15 or 17, the seventh cysteine of SEQ. ID. No. 15 or 17 and the seventh cysteine of SEQ. ID. No. 16, and the twentieth cysteine of SEQ. ID. No. 15 or 17 and the nineteenth cysteine of SEQ. ID. No. 16.

According to one embodiment of the present invention, a pharmaceutical composition for preventing or treating neurodegenerative disease, the composition including a PSA bonded to a biotin moiety, may be provided.

The PSA may be used for preventing or treating neurodegenerative disease. Although not limited to these, for example, the PSA may be a polypeptide having the amino acid sequence of SEQ. ID. No. 1 to 14, a protein having the amino acid sequence of SEQ. ID. No. 15 and 16, a protein having the amino acid sequence of SEQ. ID. No. 17 and 16, or a derivative thereof.

Further, the protein is bonded through a disulfide bond between the sixth and eleventh cysteine of SEQ. ID. No. 15 or 17, the seventh cysteine of SEQ. ID. No. 15 or 17 and the seventh cysteine of SEQ. ID. No. 16, and the twentieth cysteine of SEQ. ID. No. 15 or 17 and the nineteenth cysteine of SEQ. ID. No. 16.

According to one embodiment of the present invention, the pharmaceutical composition comprising the physiologically active substance bonded to a biotin moiety as an active ingredient may be formulated and administered in various oral and non-oral administration forms, but is not limited to these.

When formulating, commonly used fillers, solubilizing agents, bulking agents, bonding agents, wetting agents, disintegrating agents, surfactants and other diluents and excipients may be used for formulation.

Solid formulations for oral administration include tablets, pills, powders, granules and capsules, and such solid formulations may be formulated mixing at least one excipient, for example starch, calcium carbonate, sucrose or lactose into the compound.

Further, aside from simple excipients, lubricating agents such as magnesium stearate or talc are used. Liquid formulations for oral administration include suspensions, oral liquids, emulsions and syrups, which may include, in addition to the commonly used diluents of water and liquid paraffin, various excipients, for example wetting agents, flavoring agents, sweetening agents and preservatives. Formulations for non-oral administration include sterile aqueous solutions, non-aqueous solvents, suspensions, emulsions, lyophilized drugs and suppositories. As non-aqueous solvents or suspensions, propylene glycol, polyethylene glycol (PEG), plant-based oils such as olive oil, and injectable esters such as ethyl oleate may be used.

Further, calcium or vitamin D3 may be added to improve efficacy as a therapeutic agent for proliferative disease or autoimmune disease.

The range of administration dose of the pharmaceutical composition according to one embodiment of the present invention may be diversified depending on the body weight, age, gender, health status, diet, administration interval, method of administration, excretion rate and severity of illness, but generally may be administered once-a-day or across multiple administrations within a daily effective dose. Further, the effective dose may be administered through multiple administrations every one or two weeks.

In the following, the present invention will be described in detail through embodiments and experimental examples. Provided, that the following embodiments and experimental examples are intended only to exemplify the present invention, and the scope of the present invention is not limited by the following embodiments and experimental examples.

### [Embodiments]

### <Embodiment: Preparation of Biotin Moiety>

### List of Abbreviations

HBTU: 3-[Bis(dimethylamino)methyliumyl]-3H-benzotriazol-1-oxide hexafluorophosphate
DIEA: Ethyldiisopropylamine
HATU: 1-[Bis(dimethylamino)methylene]-1H-1,2,3-triazolo[4,5-b]pyridinium 3-oxide hexafluorophosphate
DIC: Diisopropylcarbodiimide
HOBt: 1-Hydroxybenzotriazole
MBHA: 4-Methylbenzhydrylamine hydrochloride)
Fmoc: 9-Fluorenylmethoxycarbonyl
DMF: dimethylformamide
SPPS: solid phase peptide synthesis
HPLC: high performance liquid chromatography
LCMS: liquid chromatography mass spectrometry

### General SPPS Method

In some cases, solid phase synthesis of peptide may be improved through the use of a group which may be cleaved under acidic conditions, for example, 2-Fmoc-oxy-4-methoxybenzyl or a d-peptide protected in a d-peptide amid bond having a 2,4,6-trimethoxybenzyl. The Fmoc-protected amino acid derivative used was the recommended standard: for example, Fmoc-Ala-OH, Fmoc-Arg(Pbf)-OH, Fmoc-Asn(Trt)-OH, Fmoc-Asp(OtBu)-OH, Fmoc-Cys(Trt)-OH, Fmoc-Gln(Trt)-OH, Fmoc-Glu(OtBu)-OH, Fmoc-Gly-OH, Fmoc-His(Trt)-OH, Fmoc-Ile-OH, Fmoc-Leu-OH, Fmoc-Lys(Boc)-OH, Fmoc-Met-OH, Fmoc-Phe-OH, Fmoc-Pro-OH, Fmoc-Ser(tBu)-OH, Fmoc-Thr(tBu)-OH, Fmoc-Trp(Boc)-OH, Fmoc-Tyr(tBu)-OH, or Fmoc-Val-OH , etc. supplied by Anaspect, Bachem, Iris Biotech or Novabiochem. The N-terminal amino acid was a Boc protected in an alpha amino group. For example, Fmoc-8-amino-3,6-dioxaoctanopic acid, Fmoc-tranexamic acid, Fmoc-isonipecotic acid, Fmoc-Glu-OtBu, Fmoc-Lys(Fmoc)-OH supplied by Anaspec, Bachem, Iris Biotech or Novabiochem was used.

### Peptide Synthesis using SPPS

The peptide may be synthesized using ordinary Fmoc chemistry in a link amid MBHA resin using HBTU/DIEA, HATU/DIEA or DIC/HOBt as a coupling reagent. The reactants and coupling reagents used in synthesis include the following combinations

**[Table 1]**

| # | Reactant | Coupling Reagent |
|---|---|---|
| 1 | Fmoc-Lys (Biotin)-OH (1.5 *eq)* | HBTU (1.42 *eq)* and DIEA (3.0 *eq)* |
| 2 | Fmoc-Lys (Biotin)-OH (2.0 *eq)* | HBTU (1.9 *eq)* and DIEA (4.0*eq*) |
| 3 | 3-(2,5-dioxo-2,5-dihydro-1H-pyrrol-1-yl) propanoic acid (3.0 *eq)* | DIC (3.0 *eq)* and HOBt (6.0 *eq)* |
| 4 | 2,2-dimethyl-4-oxo-3,7,10,13,16,19,22-heptaoxapentacosan-25-oic acid (2.0 *eq)* | HATU (1.9 *eq)* and DIEA (4.0 *eq)* |
| 5 | Fmoc-21-amino-4,7,10,13,16,19-hexaoxaheneicosanoic acid (2.0 *eq)* | HATU (1.9 *eq)* and DIEA (4.0 *eq)* |

An exemplary protocol for the peptide synthesis process using SPSS includes the following. 1) DMF is added to a vessel containing link amide MBHA resin and let expand for 2 hours (sub: 0.68 mmol/g, 1.0mmol, 1.47g or 5mmol, 7.35g, sub: 0.68 mmol/g). 2) 20% piperidine/DMF is added, then mixed for 30 minutes. 3) The solvent of 1)-2) is removed, followed by washing with DMF (30 seconds × 5 times). 4) A reactant (1 of the reactants of #1 to #5) is added, then mixed for 30 seconds, followed by addition of the coupling reagent (1 of the coupling reagents of #1 to #5) and nitrogen bubbling for one hour. 5) 20% piperidine/DMF is added, followed by 30 minutes of mixing.

In the exemplary protocol above of 1) to 5), combinations of the reactants and coupling reagents of #1 to #5 may be used at least once to carry out repeated synthesis. To remove Fmoc, [the mixture] was treated for 30 minutes with 20% piperidine/DMF solution.

### General Process for Peptide Purification and Analysis

Unpurified peptide was dissolved in a suitable mixture of water, TFA and ACN, purified using preparative HPLC, dried and quantified. The conditions for purification using preparative HPLC include those shown in Table 2 below.

**[Table 2]**

| Purification Conditions | |
|---|---|
| Solvent | ACN/H₂O |
| Equipment | SHIMADZU LC-8A, or Gilson GX-281 |
| Mobile Phase | A: H₂O (0.075% TFA in H₂O) |
| | B: CH₃CN |
| Gradient | 15-35%-60 min. Retention time: 42 min, or 20-50%-60 min. Retention time: 45min, or 5-35%-60min. Retention time: 50 min |
| Column | Luna25^{∗}200mm, C18, 10um, 110A+Gemin150^{∗}30mm, C18, 5um, 110A, or Luna50^{∗}25mm, C18, 10um, 100Å+Gemini^{®}250^{∗}50mm, C8, 5um, 110Å |
| Flow Rate | 80mL/Min or 20mL/Min |
| Wavelength | 220/254nm |
| Oven Tem. | Room temperature |

Following purification using preparative HPLC, analytical HPLC or LMS was used to analyze the characteristics of the final product.

Analysis showed that the biotin moieties of Table 3 below were obtained.

**[Table 3]**

| Biotin Moiety | Nomenclature |
|---|---|
| B1 | N-Biotionyl-N'-(6-maleimidohexaonyl)hydrazide |
| B2 | 3 - Maleimidopropionate-Lys(Biotin)-Lys(Biotin)-CONH₂ |
| B3 | 3-Maleimidopropionate-Lys(Biotin)-Lys(Biotin)-Lys(Biotin)-CONH₂ |
| B4 | Propionate-N-hydroxysuccinimide ester-PEG-Lys(Biotin)-Lys(Biotin)-Lys(Biotin)-CONH₂ |
| B5 | 3-Maleimidopropionate-PEG-Lys(Biotin)-Lys(Biotin)-Lys(Biotin)-CONH₂ |

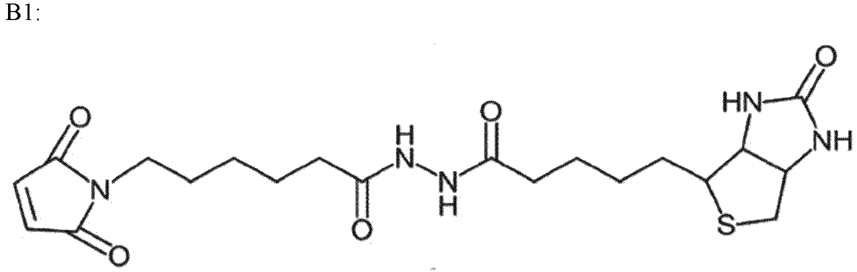

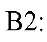

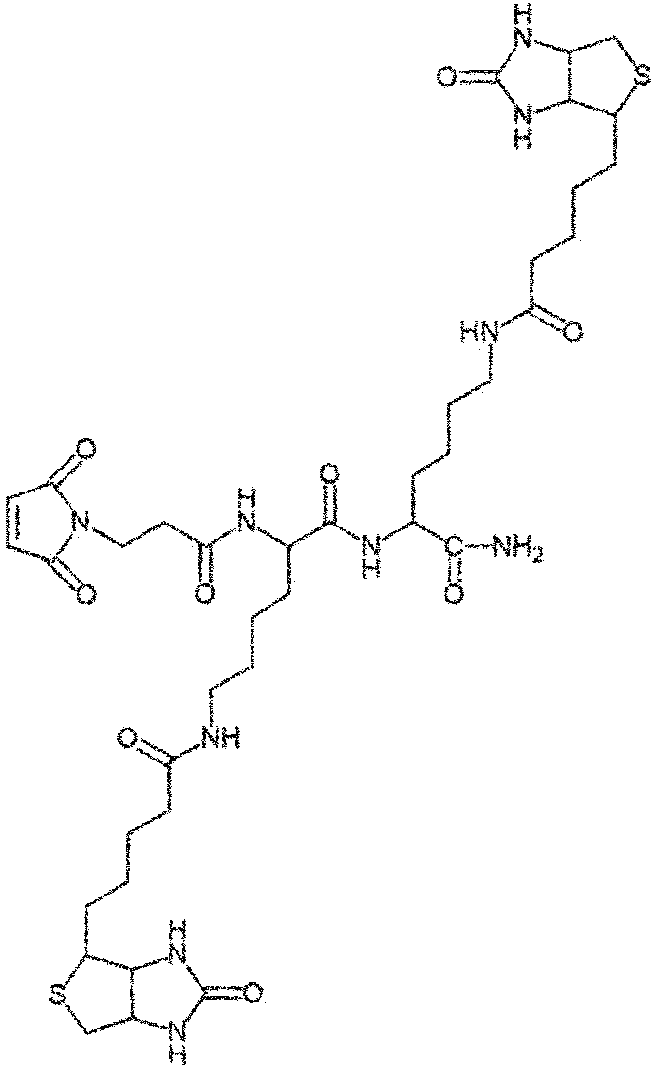

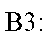

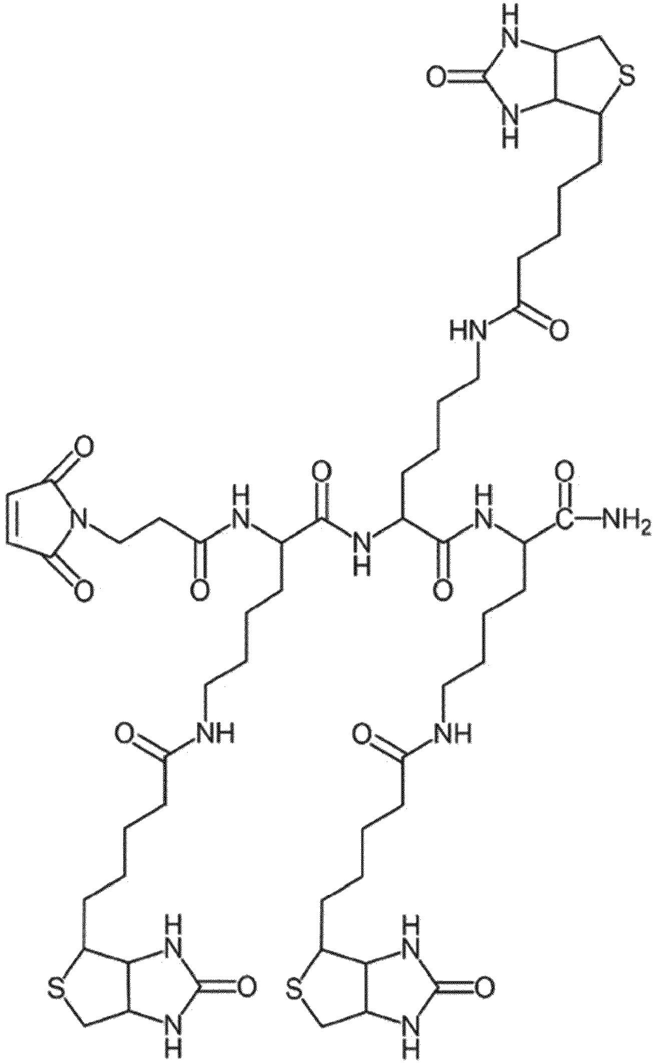

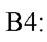

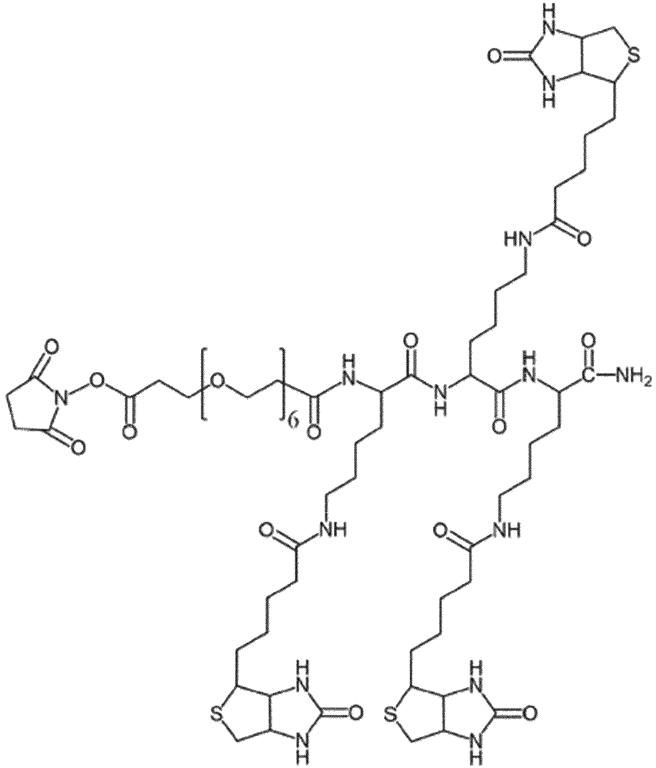

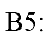

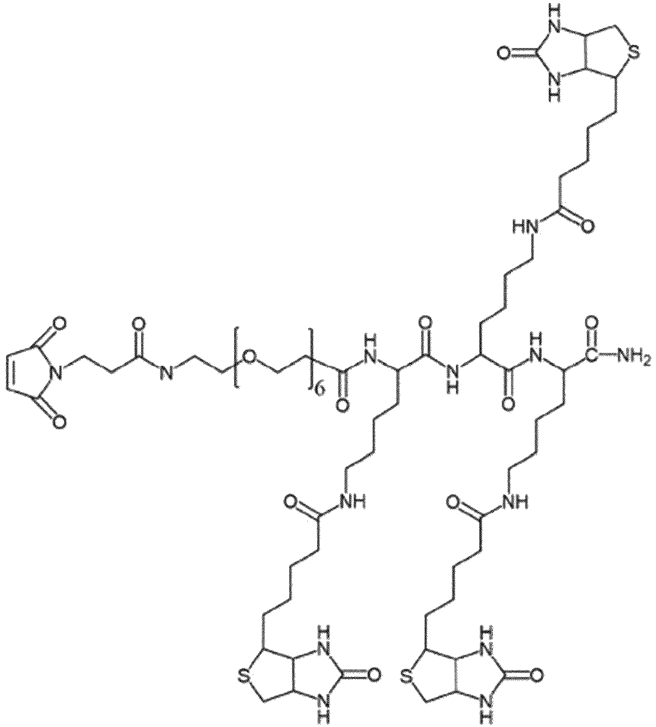

Further, through the peptide synthesis protocol of 1) to 5) using SPSS, and purification and analysis, the following biotin moieties were obtained. In Table 4, X, Y, Z and B are included in the definition of General Formula A of the present specification.

**[Table 4]**

| Biotin Moiety | X | Y | Z | Number of Bs (Biotin) |
|---|---|---|---|---|
| B6 | Aldehyde | propane | Lysine | 2 |
| B7 | Maleimide | butyrate | Glycerol and PEG | 2 |
| B8 | Maleimide | butyrate | Glycerol and PEG | 2 |
| B9 | N-hydroxysuccinimide | butyrate | Lysine | 2 |
| B10 | N-hydroxysuccinimide | glutarate | Glycerol and PEG | 2 |
| B11 | Maleimide | PEG₁₂ | Lysine | 3 |
| B12 | N-hydroxysuccinimide | PEG₁₂ | Lysine | 3 |
| B13 | amine | - | Lysine | 3 |
| B14 | Aldehyde | pentane | Lysine | 2 |
| B15 | Maleimide | adipate | Glycerol and PEG | 2 |
| B16 | Maleimide | suberate | Glycerol and PEG | 2 |
| B17 | Maleimide | sebacate | Glycerol and PEG | 2 |
| B18 | N-hydroxysuccinimide | adipate | Glycerol and PEG | 2 |
| B19 | N-hydroxysuccinimide | suberate | Lysine | 4 |
| B20 | N-hydroxysuccinimide | sebacate | Lysine | 4 |
| B21 | N-hydroxysuccinimide | PEG₆ | Glycerol and PEG | 2 |
| B22 | Succinimidyl carbonate | PEG₆ | Lysine | 2 |
| B23 | Succinimidyl carbonate | PEG₁₂ | Lysine | 3 |
| B24 | Succinimidyl carbonate | pentane | Lysine | 3 |
| B25 | Succinimidyl carbonate | hexane | Lysine | 3 |
| B26 | p-nitrophenyl carbonate | PEG₆ | Lysine | 3 |
| B27 | p-nitrophenyl carbonate | PEG₁₂ | Lysine | 4 |
| B28 | p-nitrophenyl carbonate | propane | Glycerol and PEG | 2 |
| B29 | p-nitrophenyl carbonate | pentane | Glycerol and PEG | 2 |
| B30 | amine | - | Glycerol and PEG | 2 |
| B31 | thiol | butyrate | Lysine | 2 |
| B32 | thiol | glutarate | Lysine | 3 |
| B33 | aminoxy | PEG₆ | Lysine | 3 |
| B34 | iodoacetamide | PEG₆ | Lysine | 3 |

### <Embodiment: Preparation of Physiologically Active Substance Bound to Biotin Moiety>

### <Embodiments 1 through 3>

### 1) Preparation Example

Using DMSO solvent with 0.3% triethylamine (TEA, Sigma) added as the reaction solvent, a mixture of a molar ratio of 1:2 between the polypeptide-biotin moieties stated in Table 5 below with the polypeptide of SEQ. ID. No. 12 and the biotin moieties stated in Table 3 above (B1, B2 and B3 used, respectively; polypeptide of SEQ. ID. No. 12: B1, B2 or B3) was prepared. The mixture was reacted for at least 30 minutes at room temperature, and the reaction was stopped by adding a volume of 1% trifluoroacetic acid identical to the volume of the mixture.

**[Table 5]**

| Item | Polypeptide | Biotin Moiety | Binding Position of the Biotin Moiety |
|---|---|---|---|
| Embodiment 1 | SEQ. ID. No. 12: HGEGTFTSDLSKQMEEEAVRLFIEWLKNGGPSSGAPPPSC | B1 | C40 |
| Embodiment 2 | SEQ. ID. No. 12: HGEGTFTSDLSKQMEEEA VRLFIEWLKNGGPSSGAPPPSC | B2 | C40 |
| Embodiment 3 | SEQ. ID. No. 12: HGEGTFTSDLSKQMEEEAVRLFIEWLKNGGPSSGAPPPSC | B3 | C40 |

### 2) Isolation, purification and confirmation

The reaction products of Embodiments 1 through 3 above were isolated and purified using reverse phase high performance liquid chromatography (hereinafter "HPLC").

The SUPERSIL ODS-1 column (10×250mm, 5 *µ*m, LB Science, South Korea) was used as the column. The mobile phase condition was linearly changed maintaining a flow rate of 4.7m/min using 30-50% solvent B (acetonitrile with 0.1% TFA) and solvent A (distilled water with 0.1% TFA). Monitoring at 280nm in a UV absorptiometer, peaks detected between 12 and 14 minutes were collected. Organic solvent and TFA were evaporated under vacuum from the collected peaks, followed by concentration and purification using an ultracentrifugal filter having an appropriate molecular weight cutoff. The purity of the purified substances was confirmed using HPLC analysis. At a constant temperature near room temperature, analysis was carried out using a Gemini C18 column (4.6x250mm, 5 *µ*m; Phenomenex, CA, USA). Analysis was carried out using the gradient elution method at a flow rate of 1mL/min using a mobile phase comprised of trifluoroacetic acid test solution: acetonitrile mixture (with a mix ratio of 70:30, then 50:50 20 minutes later). UV absorbance was observed at 280nm.

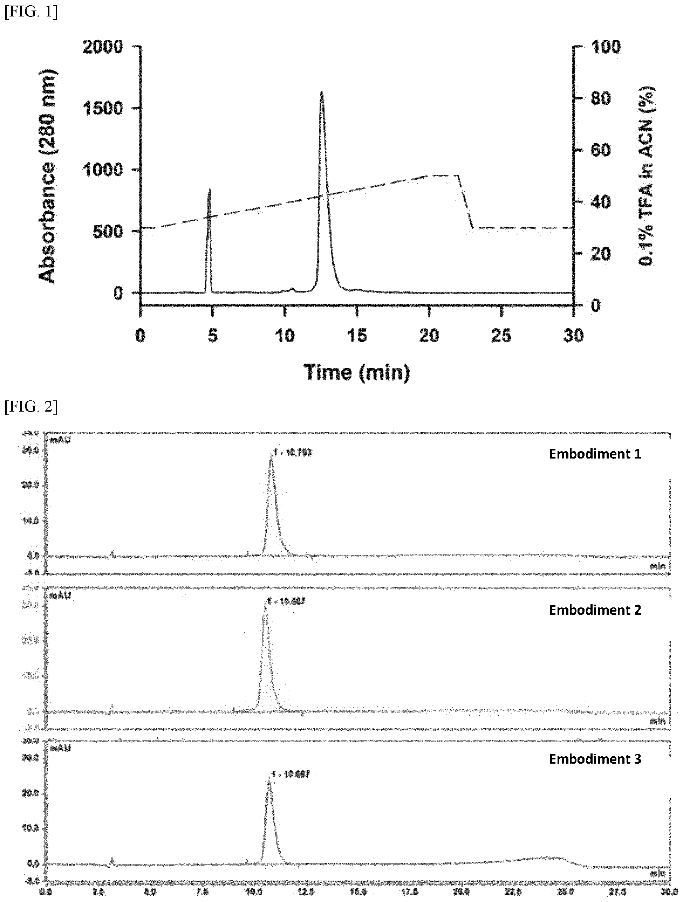

FIG. 1 is a purification chromatogram of Embodiment 3.

FIG. 2 is a chromatogram for the final substance of Embodiment 1 through Embodiment 3.

Under analysis using a UV detector, no peaks other than the polypeptide bonded to a biotin moiety were observed, and expressing the area of the peaks in each chromatogram as percentages, the purity of embodiments 1 through 3 was confirmed to be 99% or greater.

### Molecular Weight Check

The molecular weights of the final substances obtained in Embodiment 1 through Embodiment 3 were measured.

Molecular weight was measured using the MALDI-TOF mass spectrometry method. As the matrix solution, a 50% acetonitrile solution containing 0.1% TFA saturated with CHCA (α-Cyano-4-hydroxycinnamic acid) was used. The mass spectrum was examined in linear mode and reflectron mode, and molecular weight was checked with the concentration of the final substances at 0. 1mg/mL.

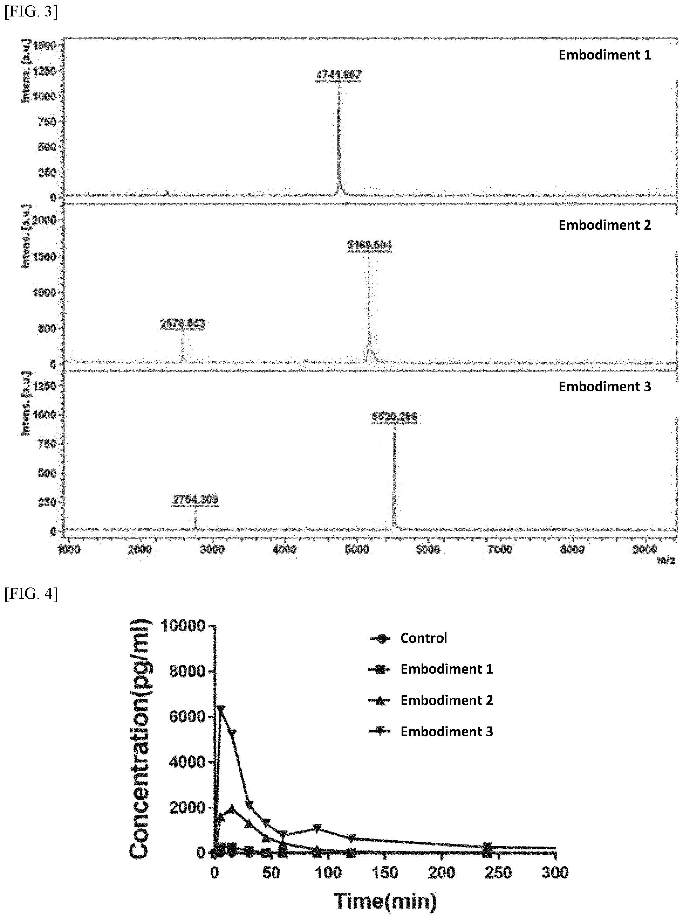

FIG. 3 is a MALDI-TOF mass spectrum of Embodiment 1 through Embodiment 3. Referring to FIG. 3, it was confirmed that the molecular weights of the substances isolated through MALDI-TOF mass spectrometry matched their theoretical molecular weights.

### <Embodiments 4 through 9>

Using the same method as that of Embodiments 1 through 3, polypeptide-biotin moieties were prepared by bonding a biotin moiety to the polypeptides of SEQ. ID. No. 8 and 13, as shown in Table 6 below.

**[Table 6]**

| Item | Polypeptide | Biotin Moiety | Binding Position of the Biotin Moiety |
|---|---|---|---|
| Embodiment 4 | SEQ. ID. No. 8: H(Aib)QGTFTSDYSKYLDEQAAKEFVQWLMNTC | B1 | C30 |
| Embodiment 5 | SEQ. ID. No. 8: H(Aib)QGTFTSDYSKYLDEQAAKEFVQWLMNTC | B2 | C30 |
| Embodiment 6 | SEQ. ID. No. 8: H(Aib)QGTFTSDYSKYLDEQAAKEFVQWLMNTC | B3 | C30 |
| Embodiment 7 | SEQ. ID. No. 13: SVSEIQLMHNLGKHLNSMERVEWLRKKLQDVHNFC | B1 | C35 |
| Embodiment 8 | SEQ. ID. No. 13: SVSEIQLMHNLGKHLNSMERVEWLRKKLQDVHNFC | B2 | C35 |
| Embodiment 9 | SEQ. ID. No. 13: SVSEIQLMHNLGKHLNSMERVEWLRKKLQDVHNFC | B3 | C35 |

### [Evaluation]

### Reaction Rate and Yield

Reaction rates were found by comparing the HLPC peak areas of Embodiments 1 through 3 under HPLC analysis of the mixtures with biotin moiety, based on the amount of polypeptide added in the initial mixture-obtaining step.

Yield of the present invention was found by quantifying the amount of final purified substance based on the amount of polypeptide added in the initial mixture-obtaining step. The results are shown in Table 7 below.

**[Table 7]**

| | Embodiments 1 through 3 |
|---|---|
| Binding Position | C40 |
| Reaction Rate | >99% |
| Yield | 80% |

### Measuring Oral Absorption Rate of Embodiments 1 through 3

To confirm oral absorption rate, pharmaceutical behavior was compared.

The specimens were intravenously and orally administered in amounts of 100 and 500 *µ*g/kg, respectively to experimental rats (SD rat) with a body weight of approximately 200g, then blood serum was collected and enzyme-linked immunosorbent assays were carried out to measure change in blood drug concentration over time. Blood samples were collected from the jugular vein. Results were calculated into averages, and a polypeptide having the amino acid sequence of SEQ. ID. No. 5 was used as the control. In the results of the experiment, oral absorption rate was found to be improved over the control by approximately 257 times for Embodiment 1, 270 times for Embodiment 2, and approximately 557 times for Embodiment 3.

FIG. 4 is a graph illustrating blood concentration of Embodiment 1 through Embodiment 3 by hour following oral administration to a rat. As shown in FIG. 4, Embodiments 1 through 3 were confirmed to exhibit outstanding oral absorption compared to a control.

### Measuring Blood Glucose Regulation Ability of Embodiments 1 through 3

To confirm glucose tolerance, Embodiments 1 through 3 in an oral GLP-1 agonist dosage form were orally administered to mice, and blood glucose regulation efficacy was measured through an intraperitoneal glucose tolerance test (IPGTT).

To measure peritoneal glucose tolerance in an animal model, 100 *µℓ* of specimen (1 *µ*g/mouse, SEQ. ID. No. 1) was orally administered at -60 minutes to 9-week-old male mice (C57BL/6), followed by intraperitoneal injection of 200 *µℓ* glucose (2g/kg) and observation of blood glucose changes in blood collected from the tail vein at -60, 0, 20, 40, 60, 90 and 120 minutes. For Control 1, a polypeptide having the amino acid sequence of SEQ. ID. No. 5 was subcutaneously administered, and for Control 2 the same was orally administered.

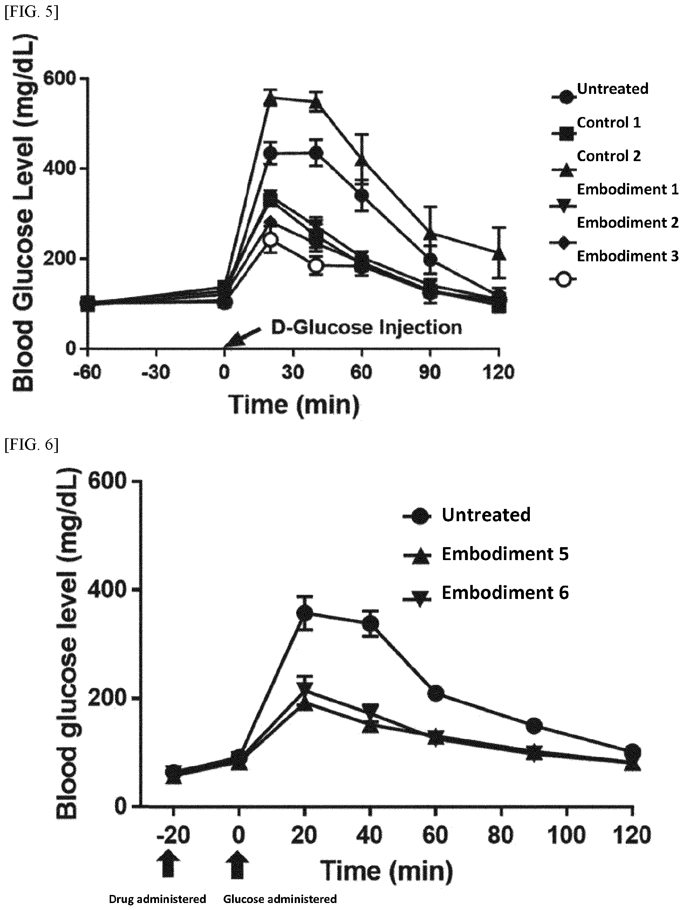

FIG. 5 is a graph illustrating blood glucose change following glucose administration to each specimen. As shown in FIG. 5, Embodiments 1 through 3 were confirmed to have glucose regulating ability.

### Measuring Blood Glucose Regulation Ability of Embodiment 5 and Embodiment 6

To confirm glucose tolerance, Embodiments 5 and 6 were orally administered to mice, and blood glucose regulation efficacy was measured through an intraperitoneal glucose tolerance test.

To measure peritoneal glucose tolerance in an animal model, 100 *µℓ* of specimen (500 *µ*g/kg, SEQ. ID. No. 2) was orally administered at -20 minutes to 9-week-old male mice (C57BL/6), followed by intraperitoneal injection of 200 *µℓ* glucose (2g/kg) and observation of blood glucose changes in blood collected from the tail vein at -20, 0, 20, 40, 60, 90 and 120 minutes.

FIG. 6 is a graph illustrating blood glucose change after administering glucose to each specimen. As shown in FIG. 6, Embodiments 5 and 6 were confirmed to have glucose regulating ability.

### Measuring Oral Absorption Rate of Embodiment 8 and Embodiment 9

The oral absorption rates of Embodiments 8 and 9 were measured. The specimens were administered by subcutaneous injection and orally in amounts of 20 *µ*g/kg to experimental rats (SD rat) with a body weight of approximately 200g, then blood serum was isolated and enzyme-linked immunosorbent assays were carried out to measure change in blood drug concentration over time. Blood samples were collected from the jugular vein. The results of measurement are as shown in Table 8 below. A polypeptide having the amino acid sequence of SEQ. ID. No. 6 was used as the control.

**[Table 8]**

| | Bioavailability Compared to Subcutaneous Administration (%) |
|---|---|
| Control, 20 *µ*g/kg, subcutaneous injection | - |
| Embodiment 8, 20 *µ*g/kg, oral administration | 7.8 |
| Embodiment 9, 20 *µ*g/kg, oral administration | 4.9 |

As shown in the above, the polypeptide bonded to a biotin moiety according to one embodiment of the present invention was confirmed to have a high absorption rate in the intestines.

### <Embodiment 10>

Using biotin moiety B4 from Table 3 and the polypeptide having the amino acid sequence of SEQ. ID. No. 5 in Table 9 below, a polypeptide bonded to a biotin moiety was prepared.

**[Table 9]**

| Item | Polypeptide | Biotin Moiety | Binding Position of Biotin Moiety |
|---|---|---|---|
| Embodiment 10 | SEQ. ID. No. 5: HGEGTFTSDLSKQMEEEA VRLFIEWLKNGGPSSGAPPPS | B4 | K27 |

### 1) Preparation example

Using DMSO solvent with 0.3% triethylamine (TEA, Sigma) added as the reaction solvent, a mixture of a molar ratio of 1:4 between the polypeptide of SEQ. ID. No. 5 and B4 in Table 3 above was prepared. The mixture was reacted for two hours at room temperature, and the reaction was stopped by adding a volume of 1% trifluoroacetic acid identical to the volume of the mixture.

### 2) Isolation, purification and confirmation

The reaction product of Embodiment 10 was isolated and purified using reverse phase HPLC.

The SUPERSIL ODS-1 column (10×250mm, 5 *µ*m, LB Science, South Korea) was used as the column. The mobile phase condition was linearly changed maintaining a flow rate of 4.7m/min using 30-50% solvent B (acetonitrile with 0.1% TFA) and solvent A (distilled water with 0.1% TFA). Monitoring at 280nm in a UV absorptiometer, the peaks detected at 12.7 minutes were collected. Organic solvent and TFA were evaporated under vacuum from the collected peaks, followed by concentration and purification using an ultracentrifugal filter having an appropriate molecular weight cutoff. The purity of the purified substances was confirmed using HPLC analysis. At a constant temperature near room temperature, analysis was carried out using a Gemini C18 column (4.6x250mm, 5 *µ*m; Phenomenex, CA, USA). Analysis was carried out at a flow rate of 1mL/min using a mobile phase comprised of trifluoroacetic acid test solution: acetonitrile mixture (with a mix ratio of 70:30, then 50:50 20 minutes later). UV absorbance was observed at 280nm.

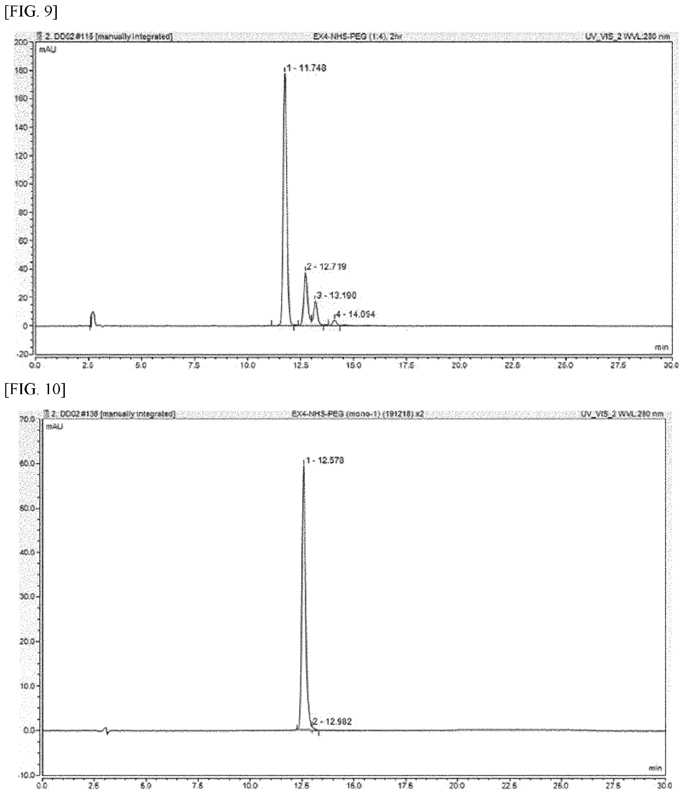

FIG. 9 is a purification chromatogram of Embodiment 10 of the present invention. Unreacted polypeptide of SEQ. ID. No. 1 was detected at 11.7 minutes, and the substance of Embodiment 10 bonded to a biotin moiety was detected at 12.7 minutes.

FIG. 10 is a post-purification chromatogram of Embodiment 10 of the present invention. A single peak of polypeptide bonded to biotin moiety was confirmed, and other peaks were not observed. Expressing the area of the peak in the chromatogram as percentages, the purity was confirmed to be 95% or greater.

### 3) Confirming molecular weight

The molecular weight of the final substance obtained in Embodiment 10 was measured.

Molecular weight was measured using the MALDI-TOF mass spectrometry method. As the matrix solution, a 50% acetonitrile solution containing 0.1% TFA saturated with CHCA (α-Cyano-4-hydroxycinnamic acid) was used. The mass spectrum was examined in linear mode and reflectron mode.

### <Embodiment 11>

Polypeptides bonded to biotin moieties were prepared using biotin moiety B5 from Table 3 and the polypeptide having the amino acid sequence of SEQ. ID. No. 12 in Table 10 below.

**[Table 10]**

| Item | Polypeptide | Biotin Moiety | Binding Positio n of Biotin Moiety |
|---|---|---|---|
| Embodiment 11 | SEQ. ID. No. 12: HGEGTFTSDLSKQMEEEAVRLFIEWLKNGGPSSGAPPPSC | B5 | C40 |

### 1) Preparation example

Using DMSO solvent with 0.3% triethylamine (TEA, Sigma) added as the reaction solvent, a mixture of a molar ratio of 1:2 between the polypeptide of SEQ. ID. No. 12 and B5 in Table 3 above was prepared. The mixture was reacted for 30 minutes at room temperature, and the reaction was stopped by adding a volume of 1% trifluoroacetic acid identical to the volume of the mixture.

### 2) Isolation, purification and confirmation

The reaction product of Embodiment 10 was isolated and purified using reverse phase HPLC.

The SUPERSIL ODS-1 column (10×250mm, 5 *µ*m, LB Science, South Korea) was used as the column. The mobile phase condition was linearly changed maintaining a flow rate of 4.7m/min using 30-50% solvent B (acetonitrile with 0.1% TFA) and solvent A (distilled water with 0.1% TFA). Monitoring at 280nm in a UV absorptiometer, the peaks detected at 11 minutes to 13 minutes were collected. Organic solvent and TFA were evaporated under vacuum from the collected peaks, followed by concentration and purification using an ultracentrifugal filter having an appropriate molecular weight cutoff. The purity of the purified substances was confirmed using HPLC analysis. At a constant temperature near room temperature, analysis was carried out using a Gemini C18 column (4.6x250mm, 5 *µ*m; Phenomenex, CA, USA). Analysis was carried out at a flow rate of 1mL/min using a mobile phase comprised of trifluoroacetic acid test solution: acetonitrile mixture (with a mix ratio of 70:30, then 50:50 20 minutes later). UV absorbance was observed at 280nm.

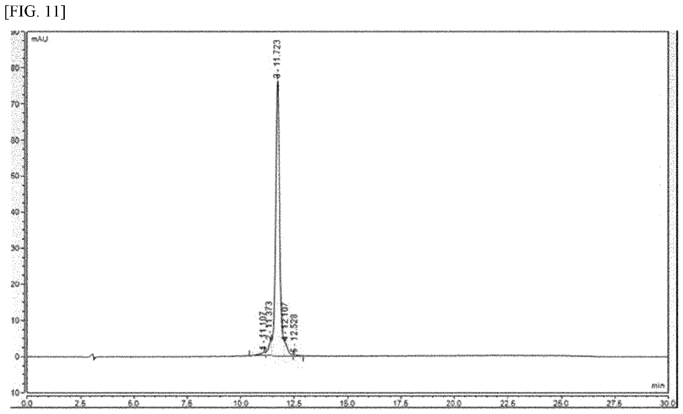

FIG. 11 is a reverse phase chromatogram of Embodiment 11 of the present invention. A single peak of polypeptide bonded to biotin moiety was confirmed, and other peaks were not observed. Expressing the area of the peak in the chromatogram as percentages, the purity was confirmed to be 90% or greater.

### 3) Confirming molecular weight

The molecular weight of the final substance obtained in Embodiment 11 was measured.

Molecular weight was measured using the MALDI-TOF mass spectrometry method. As the matrix solution, a 50% acetonitrile solution containing 0.1% TFA saturated with CHCA (α-Cyano-4-hydroxycinnamic acid) was used. The mass spectrum was examined in linear mode and reflectron mode.

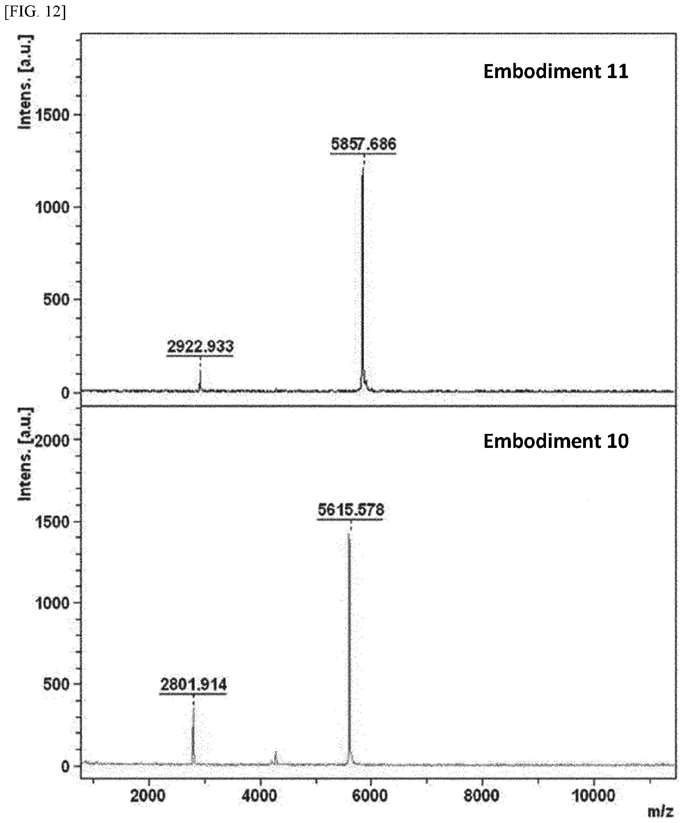

FIG. 12 is a MALDI-TOF mass spectrum of Embodiment 10 and Embodiment 11 of the present invention. Referring to FIG. 12, MALDI-TOF mass spectrometry confirmed that the molecular weights of the isolated substances matched their theoretical molecular weights.

### <Embodiments 12 and 13>

Polypeptides bonded to biotin moieties were prepared using biotin moiety B4 from Table 3 and the polypeptides having the amino acid sequences of SEQ. ID. No. 15 and 16 in Table 11 below.

**[Table 11]**

| Item | Polypeptide | Biotin Moiety | Binding Position of Biotin Moiety |
|---|---|---|---|
| Embodiment 12 | SEQ. ID. No. 15: GIVEQCCTSICSLEQLENYCN (Chain A) SEQ. ID. No. 16: FVNQHLCGSHLVEALYLVCGERGFFYTPKT (Chain B) Protein with disulfide bonds between C6 of Chain A - C11 of Chain A, C7 of Chain A - C7 of Chain B, and C20 of Chain A - C19 of Chain B | B4 | K29 of Chain B |
| Embodiment 13 | SEQ. ID. No. 15: GIVEQCCTSICSLEQLENYCN (Chain A) SEQ. ID. No. 16: FVNQHLCGSHLVEALYLVCGERGFFYTPKT (Chain B) Protein with disulfide bonds between C6 of Chain A - C11 of Chain A, C7 of Chain A - C7 of Chain B, and C20 of Chain A - C19 of Chain B | B4 | F1 of Chain B and K29 of Chain B |

### 1) Preparation example

Using DMSO solvent with 0.3% triethylamine (TEA, Sigma) added as the reaction solvent, a mixture of a molar ratio of 1:16 between the polypeptides of SEQ. ID. No. 15 and 16 and B4 in Table 3 above was prepared. The mixture was reacted for two hours at room temperature, and the reaction was stopped by adding a 30% acetonitrile/distilled water solution containing 1% trifluoroacetic acid identical to the volume of the mixture.

### 2) Isolation, purification and confirmation

The reaction product of Embodiment 12 and 13 was isolated and purified using reverse phase HPLC.

The SUPERSIL ODS-1 column (10×250mm, 5 *µ*m, LB Science, South Korea) was used as the column. The mobile phase condition was linearly changed maintaining a flow rate of 4.7m/min using 30-40% solvent B (acetonitrile with 0.1% TFA) and solvent A (distilled water with 0.1% TFA). Monitoring at 280nm in a UV absorptiometer, the peaks detected at 12.7 minutes were collected. Organic solvent and TFA were evaporated under vacuum from the collected peaks, followed by concentration and purification using an ultracentrifugal filter having an appropriate molecular weight cutoff. The purity of the purified substances was confirmed using HPLC analysis. At a constant temperature near room temperature, analysis was carried out using a Gemini C18 column (4.6x250mm, 5 *µ*m; Phenomenex, CA, USA). Analysis was carried out at a flow rate of 1mL/min using a mobile phase comprised of trifluoroacetic acid test solution: acetonitrile mixture (with a mix ratio of 70:30, then 50:50 20 minutes later). UV absorbance was observed at 280nm.

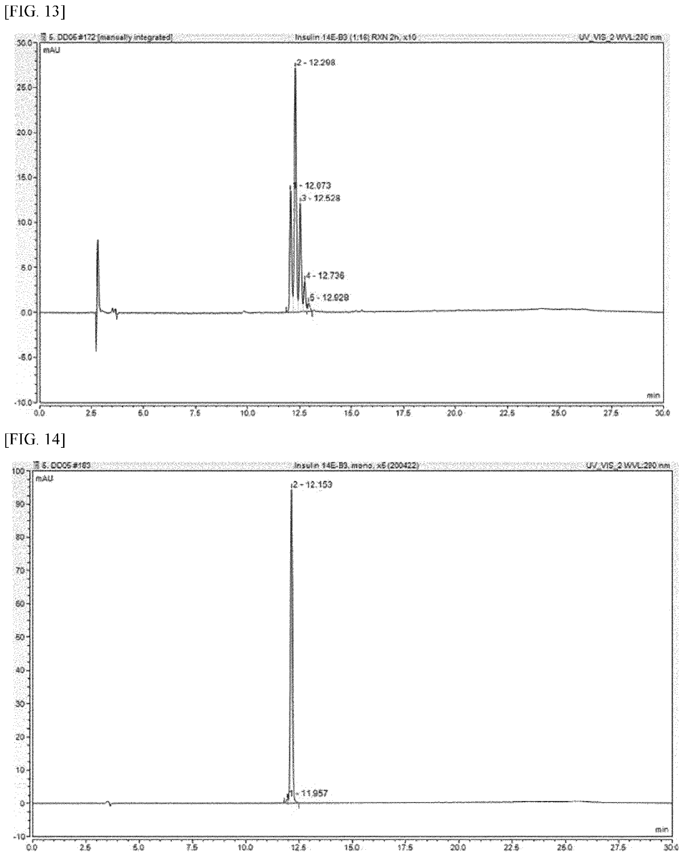

FIG. 13 is a purification chromatogram of Embodiment 12 and Embodiment 13 of the present invention. Unreacted polypeptide of SEQ. ID. No. 7 was detected at 12.1 minutes, and the substance of Embodiment 12 bonded to biotin moiety was detected at 12.3 minutes, and the substance of Embodiment 13 was detected at 12.5 minutes.

FIG. 14 is a post-purification chromatogram of Embodiment 12 of the present invention. A single peak of polypeptide bonded to biotin moiety was confirmed, and other peaks were not observed. Expressing the area of the peak in the chromatogram as percentages, the purity was confirmed to be 95% or greater.

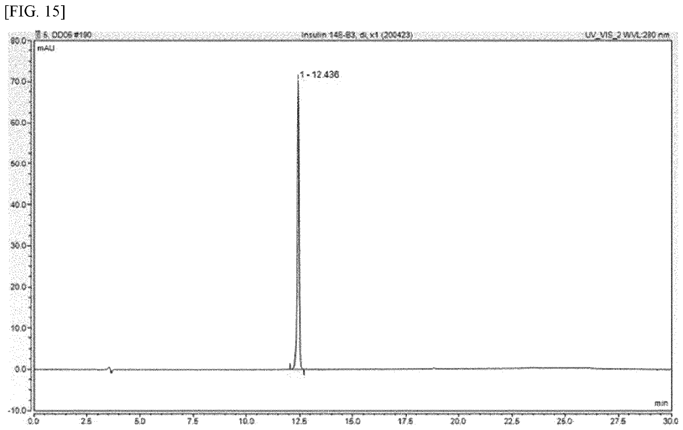

FIG. 15 is a post-purification chromatogram of Embodiment 13 of the present invention. A single peak of polypeptide bonded to biotin moiety was confirmed, and other peaks were not observed. Expressing the area of the peak in the chromatogram as percentages, the purity was confirmed to be 95% or greater.

### 3) Confirming molecular weight

The molecular weight of the final substance obtained in Embodiment 12 and 13 was measured.

Molecular weight was measured using the MALDI-TOF mass spectrometry method. As the matrix solution, a 50% acetonitrile solution containing 0.1% TFA saturated with CHCA (α-Cyano-4-hydroxycinnamic acid) was used. The mass spectrum was examined in linear mode and reflectron mode.

### [Evaluation]

### Measuring Blood Glucose Regulation Ability of Embodiments 10 and 11

To confirm glucose tolerance, Embodiments 10 and 11 in an oral GLP-1 agonist dosage form were orally administered to mice, and blood glucose regulation efficacy was measured through an intraperitoneal glucose tolerance test (IPGTT).

To measure peritoneal glucose tolerance in an animal model, 100 *µℓ* of the polypeptide bonded to a biotin moiety prepared in Embodiments 10 and 11 (10 *µ*g/mouse, SEQ. ID. No. 1) was orally administered at -20 minutes to 9-week-old male mice (C57BL/6), followed by intraperitoneal injection of 200 *µℓ* glucose (2g/kg) and observation of blood glucose changes in blood collected from the tail vein at -20 0, 0, 20, 40, 60, 90 and 120 minutes. For the control, exendin-4 comprised of the amino acid sequence represented by SEQ. ID. No. 5 was orally administered.

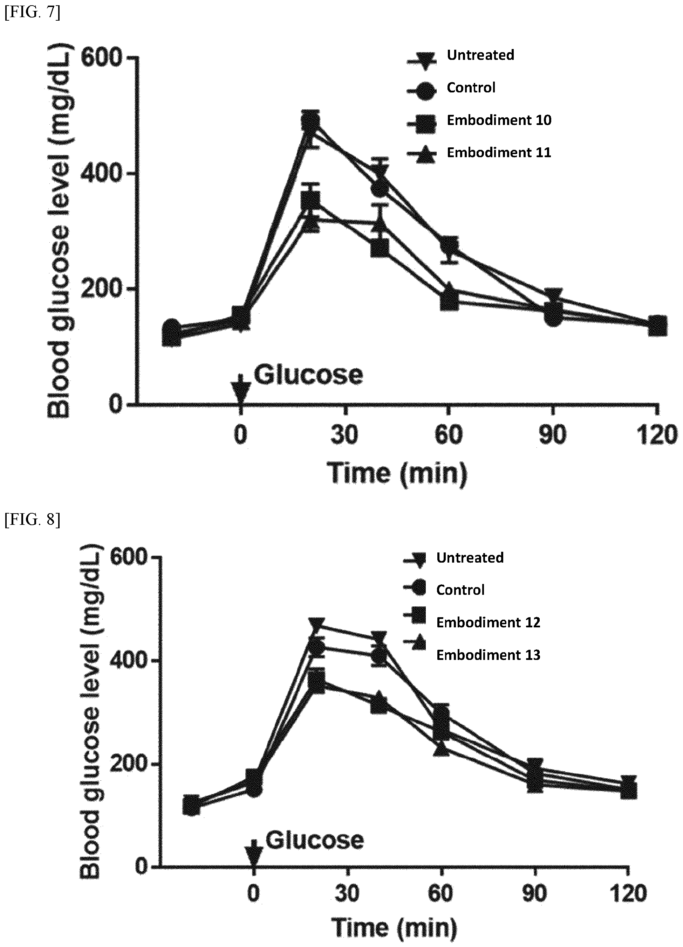

FIG. 7 is a graph illustrating blood glucose change after administering glucose to each specimen. As shown in FIG. 7, Embodiments 10 through 11 were confirmed to have blood glucose regulating ability.

### Measuring Biological Activity of Embodiments 12 and 13

The biological activity of the polypeptide before and after bonding of the biotin moiety was compared. Further, biological activity according to the bonding position of the biotin moiety was compared.

Specifically, biological activity was measured using the PathHunter^{®} Insulin Bioassay Kit (DiscoverX, #93-0466Y3-00007).

PathHunter U2OS INSRb Bioassay cells were distributed across a 96-well plate, then cultured for 24 hours in AssayComplete^{™} Cell Plating Reagent 5(CP5). Then, the respective drugs were added at 20 *µ*ℓ at concentrations of 300, 60, 20, 6.67, 2.22, 0.74, 0.25, 0.05 and 0.01. After three hours of culturing, 10 *µ*ℓ of detection reagent 1 was added and reacted for 15 minutes, followed by addition of 40 *µℓ* of detection reagent 2 and 60 minutes reaction. Then, luminescence was measured using a 96-well microplate reader. A protein comprised of a chain A having the amino acid sequence of SEQ. ID. No. 15 and a chain B having the amino acid sequence of SEQ. ID. No. 16 was used as the control. Here, the protein has disulfide bonds between C6 of Chain A - C11 of Chain A, C7 of Chain A - C7 of Chain B, and C20 of Chain A - C19 of Chain B.

**[Table 12]**

| | EC₅₀ (ng/mL) |
|---|---|
| Control | 4.06 |
| Embodiment 12 | 19.58 |
| Embodiment 13 | 55.31 |

### Measuring Blood Glucose Regulation Ability of Embodiments 12 and 13

To confirm glucose tolerance, Embodiments 12 and 13 in an oral protein dosage form were orally administered to mice, and blood glucose regulation efficacy was measured through an intraperitoneal glucose tolerance test.

To measure peritoneal glucose tolerance in an animal model, 100 *µℓ* of the polypeptide bonded to biotin moiety prepared in Embodiments 12 and 13 (10 *µ*g/mouse) was orally administered at -20 minutes to 9-week-old male mice (C57BL/6), followed by intraperitoneal injection of 200 *µ*ℓ glucose (2g/kg) and observation of blood glucose changes in blood collected from the tail vein at -20, 0, 20, 40, 60, 90 and 120 minutes. Meanwhile, a protein comprised of a chain A having the amino acid sequence of SEQ. ID. No. 15 and a chain B having the amino acid sequence of SEQ. ID. No. 16 was used as the control.

FIG. 8 is a graph illustrating blood glucose change after administering glucose to each specimen. As shown in FIG. 8, Embodiments 12 through 13 were confirmed to have glucose regulating ability.

A person skilled in the art may be aware of or confirm through everyday experimentation a multiplicity of equivalents to the specific examples of the present invention stated in the present specification. Such equivalents are intended as being included in the appended claims. The foregoing description of the present invention is meant to be exemplary, and a person having ordinary skill in the technical field to which the present invention belongs will be able to understand that the present invention is readily modifiable into other specific forms without changing the technical idea of the present invention or essential characteristics thereof. Therefore, the embodiments described in the above shall be understood to be exemplary and non-limiting in all aspects. For example, respective component elements described as combined may be carried out separately, and likewise component elements described as separate may be carried out in combined form. All modified or changed forms deducible from the meaning and scope of the appended claims and their equivalent concepts may be interpreted as being included in the scope of the present invention.

### Industrial applicability

The present invention is a physiologically active substance bonded to a biotin moiety having excellent oral absorption into the body, and a composition for oral administration including the same, and a physiologically active substance bonded to a biotin moiety can be prepared by preparing a biotin moiety using a method such as SPSS and mixing the same with a physiologically active substance. Further, the present invention has the benefits of being able to defend against decomposition of the physiologically active substance due to bonding of a biotin moiety, and ultimately promoting permeation of the intestinal membrane by physiologically active substances and their absorption in the intestines.

## Claims

1. A physiologically active substance bonded to a biotin moiety, wherein the biotin moiety is bonded to an inactive region of the physiologically active substance.

2. The physiologically active substance bonded to a biotin moiety of Claim 1, wherein the biotin moiety is represented by General Formula A below: 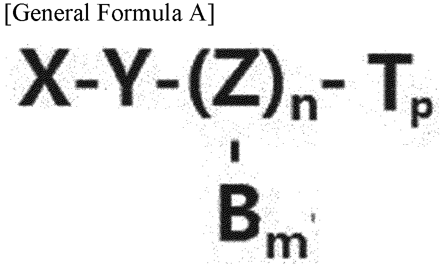 in General Formula A,
X is a functional group capable of bonding to a physiologically active substance, Y is a spacer,
Z is a bonding unit,
B can be represented by the following Chemical Formula A-1, 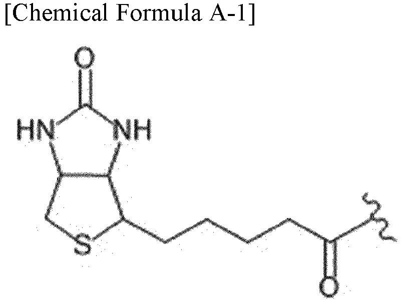
Z is connected to the 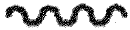 of chemical formula A-1,
T is a terminal group,
m is an integer from 1 to 10,
n is 0 or an integer from 1 to 10, and when n=0, Y is directly bonded to B or T, and
p is an integer of 0 to 1.

3. The physiologically active substance bonded to a biotin moiety of Claim 1, wherein the biotin moiety is bonded to a physiologically active substance selected from a group comprised of polypeptide, protein and polysaccharide.

4. The physiologically active substance bonded to a biotin moiety of Claim 1, wherein the physiologically active substance includes an exposed -SH group, and a biotin moiety bonds to the -SH group.

5. The physiologically active substance bonded to a biotin moiety of Claim 1, wherein the physiologically active substance includes an exposed -NH₃⁺ group or - NH2 group, and a biotin moiety bonds to the -NH₃⁺ group or -NH₂ group.

6. The physiologically active substance bonded to a biotin moiety of Claim 1, wherein the physiologically active substances includes an N-terminal, and a biotin moiety bonds to the N-terminal.

7. The physiologically active substance bonded to a biotin moiety of Claim 1, wherein the physiologically active substance is selected from a group comprised of glucagon, GLP-1 (glucagon-like peptide-1), GLP-2 (glucagon-like peptide-2), GIP (glucose-dependent insulinotropic polypeptide), exending-4, insulin, parathyroid hormone, interferon, erythropoietin, calcitonin, serotonin, rituximab, trastzumab, uricase, tissue plasminogen activator, thymoglobin, vaccine, heparin or heparin analogue, antithrombin III, filgrastim, pramlintide acetate, exenatide, eptifibatide, antivenin, IgG, IgM, HGH, thyroxin, coagulation factor VII and VIII, monoclonal antibody, glycolipid acting as a therapeutic agent, and derivatives thereof.

8. The physiologically active substance bonded to a biotin moiety of Claim 1, wherein the physiologically active substance is a polypeptide selected from a group comprised of the amino acid sequences represented by SEQ. ID. No. 1 through SEQ. ID. No. 7; a protein comprised of amino acid sequences represented by SEQ. ID. No. 15 and SEQ. ID. No. 16; or a protein comprised of amino acid sequences represented by SEQ. ID. No. 17 and SEQ. ID. No. 16.

9. The physiologically active substance bonded to a biotin moiety of Claim 8, wherein the protein having the amino acid sequence of SEQ ID. No. 15 and SEQ. ID. No. 16 or SEQ ID. No. 17 and SEQ. ID. No. 16 is a protein bonded through a disulfide bond between the sixth and eleventh cysteine of SEQ. ID. No. 15 or SEQ. ID. No. 17, the seventh cysteine of SEQ. ID. No. 15 or SEQ. ID. No. 17 and the seventh cysteine of SEQ. ID. No. 16, and the twentieth cysteine of SEQ. ID. No. 15 or SEQ. ID. No. 17 and the nineteenth cysteine of SEQ. ID. No. 16.

10. The physiologically active substance bonded to a biotin moiety of Claim 5 or Claim 6, wherein a biotin moiety bonds to the -NH⁺₃ group, -NH₂ group or N-terminal of a lysine amino acid of the polypeptide.

11. The physiologically active substance bonded to a biotin moiety of Claim 1, wherein the physiologically active substance is a polypeptide wherein any at least one of the amino acids of an inactive region of a polypeptide selected from a group comprised of the amino acid sequences represented by SEQ. ID. No. 1 through 7 has been substituted by a cysteine amino acid or a cysteine amino acid is inserted thereat, and a biotin moiety bonds to an -SH group of the cysteine amino acid.

12. The physiologically active substance bonded to a biotin moiety of Claim 10, wherein the physiologically active substance is a polypeptide selected from a group comprised of amino acid sequences represented by SEQ. ID. No. 8 through 14.

13. The physiologically active substance bonded to a biotin moiety of Claim 2, wherein the X is selected from a group comprised of maleimide, succinimide, N-hydroxysuccinimide, succinimidyl succinate, succinimidyl glutarate, succinimidyl methyl ester, succinimidyl pentyl ester, Succinimidyl carbonate, p-nitrophenyl carbonate, aldehyde, amine, thiol, oxyamine, iodoacetamide, aminoxyl, hydrazide, hydroxy, propionate, pyridyl, alkyl halide, vinylsulfone, carboxyl, Hydrazide, halogen acetamide, C₂₋₅ alkynyl, C₆₋₂₀ aryldisulfide, C₅₋₂₀ heteroaryldisulfide, isocyanate, thioester, iminoester, and derivatives thereof.

14. The physiologically active substance bonded to a biotin moiety of Claim 2, wherein the X is maleimide, N-hydroxysuccinimide, aldehyde or amine.

15. The physiologically active substance bonded to a biotin moiety of Claim 2,
wherein the Y is absent, a substituted or unsubstituted linear or branched C₁₋₅₀ alkylene, a substituted or unsubstituted linear or branched C₁₋₅₀ heteroalkylene, substituted or unsubstituted C₆₋₅₀ arylene, or substituted or unsubstituted C₆₋₅₀ heteroarylene,
and if substituted, includes at least one selected from a group comprised of =O, - C(O)NH₂, -OH, -COOH, -SH, =NH and -NH₂.

16. The physiologically active substance bonded to a biotin moiety of Claim 2, wherein the Y is a substituted linear or branched C₁₋₅₀ heteroalkylene, and includes at least one -C(O)-.

17. The physiologically active substance bonded to a biotin moiety of Claim 2,
wherein the Y is -(C(O))_{q}-(CH₂)ᵣ-(C(O)NHₛ-(CH₂)ᵣ-(OCH₂CH₂)ₜ-(C(O))_{q}-,
q, r, s and t are independently selected,
q and S are 0 or 1,
r is an integer of 1 to 20,
and t is an integer of 0 to 20.

18. The physiologically active substance bonded to a biotin moiety of Claim 2, wherein the Y is -(CH₂)ᵣC(O)NHNH- and r is an integer of 1 to 20.

19. The physiologically active substance bonded to a biotin moiety of Claim 2, wherein the Y includes -C(O)-.

20. The physiologically active substance bonded to a biotin moiety of Claim 2, wherein the Y includes -C(O)NH-.

21. The physiologically active substance bonded to a biotin moiety of Claim 2, wherein the Z is any one of the following, and each may be independently selected:
A) forms an amino acid or a derivative thereof together with X or separately from X; or
B) is a substituted or unsubstituted linear or nonlinear C₁₋₅₀ heteroalkylene, and when substituted, includes at least one selected from a group comprised of =O, -C(O)NH₂, - OH, -COOH, -SH, =NH and -NH₂.

22. The physiologically active substance bonded to a biotin moiety of Claim 2, wherein the Z is connected through B and -NH-.

23. The physiologically active substance bonded to a biotin moiety of Claim 2, wherein Z is a hydrophilic amino acid or a derivative thereof.

24. The physiologically active substance bonded to a biotin moiety of Claim 2, wherein the Z is selected from a group comprised of lysine, arginine, histidine, glutamine, asparagine, threonine, cysteine, serine and derivatives thereof.

25. The physiologically active substance bonded to a biotin moiety of Claim 2, wherein the Z includes at least one glycerol, and at least one polyethylene glycol or a bond thereof.

26. The physiologically active substance bonded to a biotin moiety of Claim 2, wherein the Z includes site, 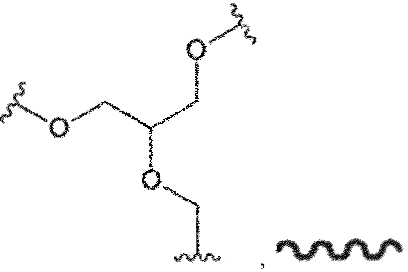 represents a binding at least one 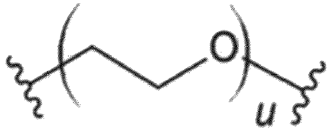 is bonded to the binding site, and u is an integer of 1 to 20.

27. The physiologically active substance bonded to a biotin moiety of Claim 26, wherein -(CH₂)₃NH- is further bonded to the

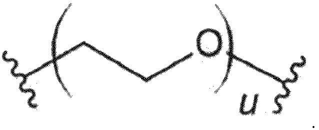

28. The physiologically active substance bonded to a biotin moiety of Claim 2, wherein the T is selected from a group comprised of amine, C₁₋₈ alkyl, C₁₋₈ alkenyl, halo, hydroxy, thiol, sulfonic acid, carboxyl, phenyl, benzyl, aldehyde, azide, cyanate, isocyanate, thiocyanate, isothiocyanate, nitrile and phosphonic acid.

29. The physiologically active substance bonded to a biotin moiety of Claim 2, wherein the T is an amine.

30. The physiologically active substance bonded to a biotin moiety of Claim 1, wherein the biotin moiety is selected from a group comprised of:

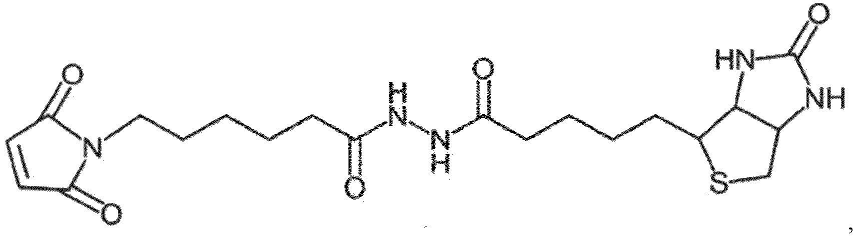

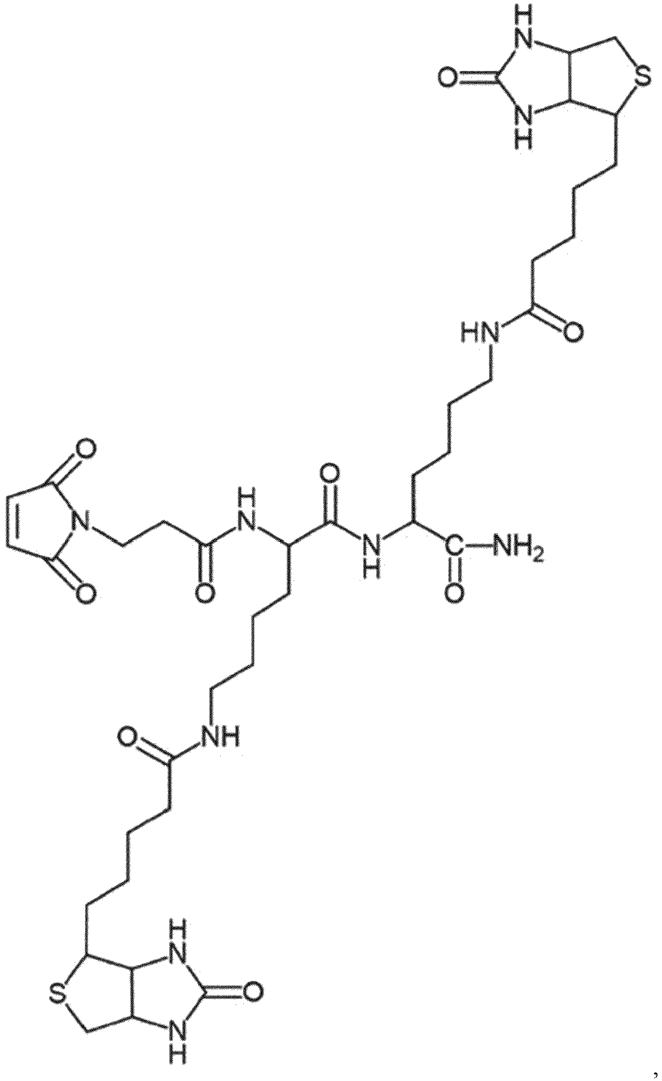

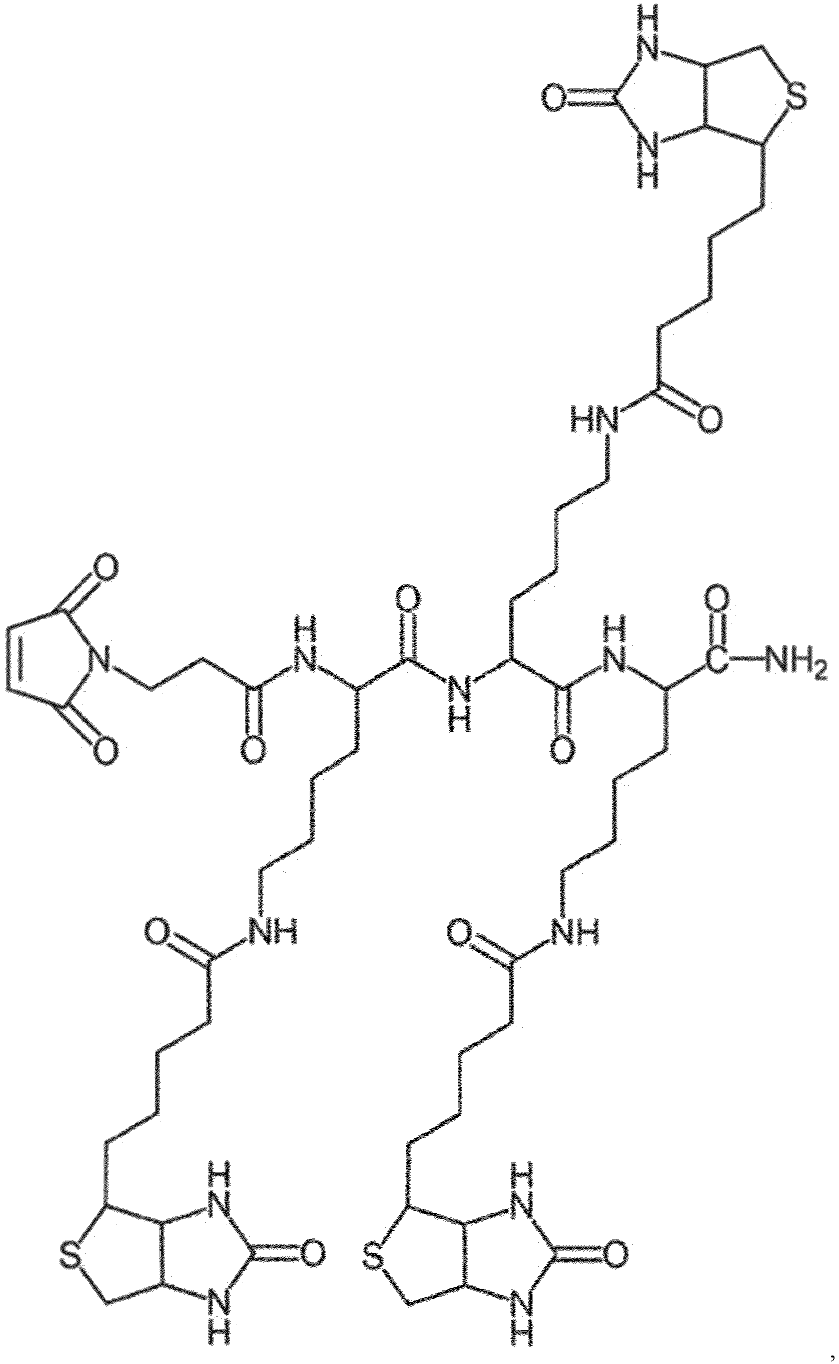

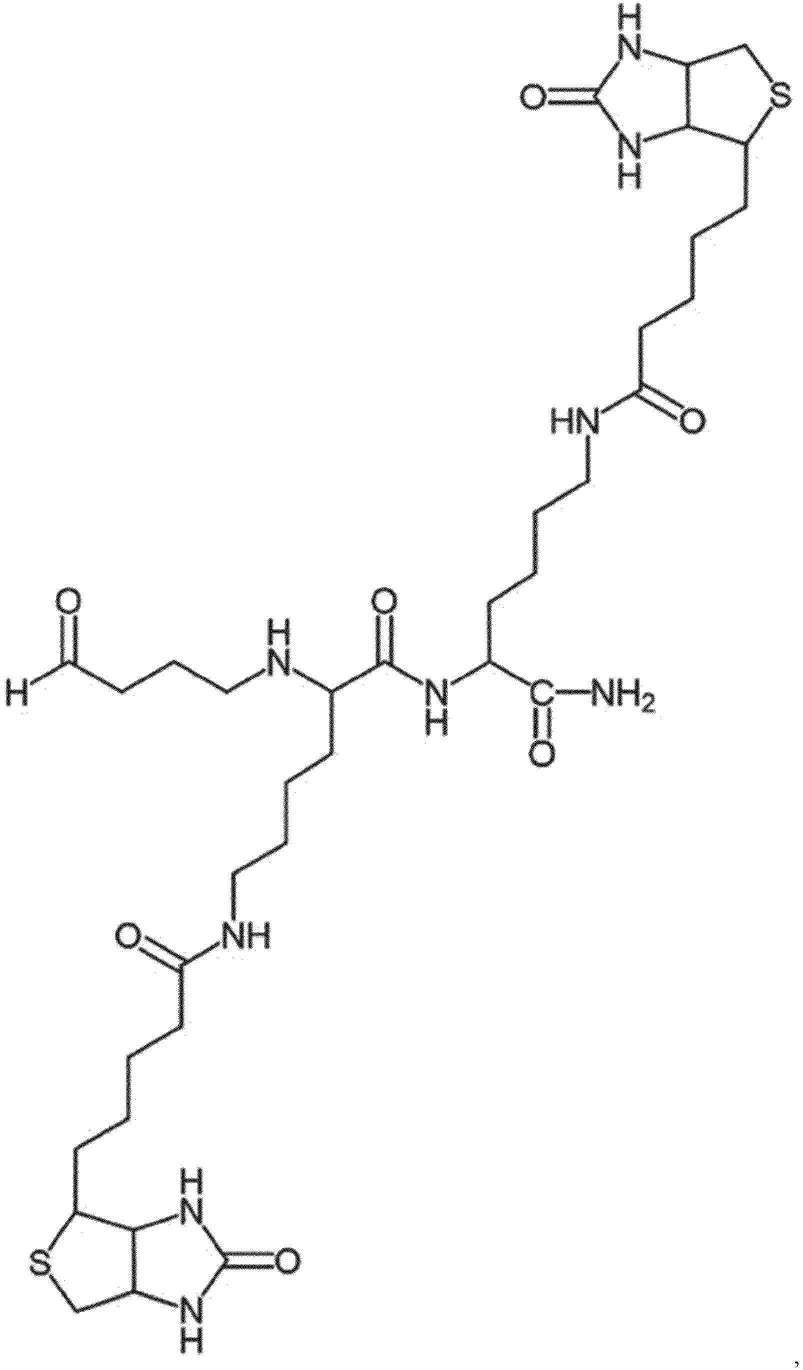

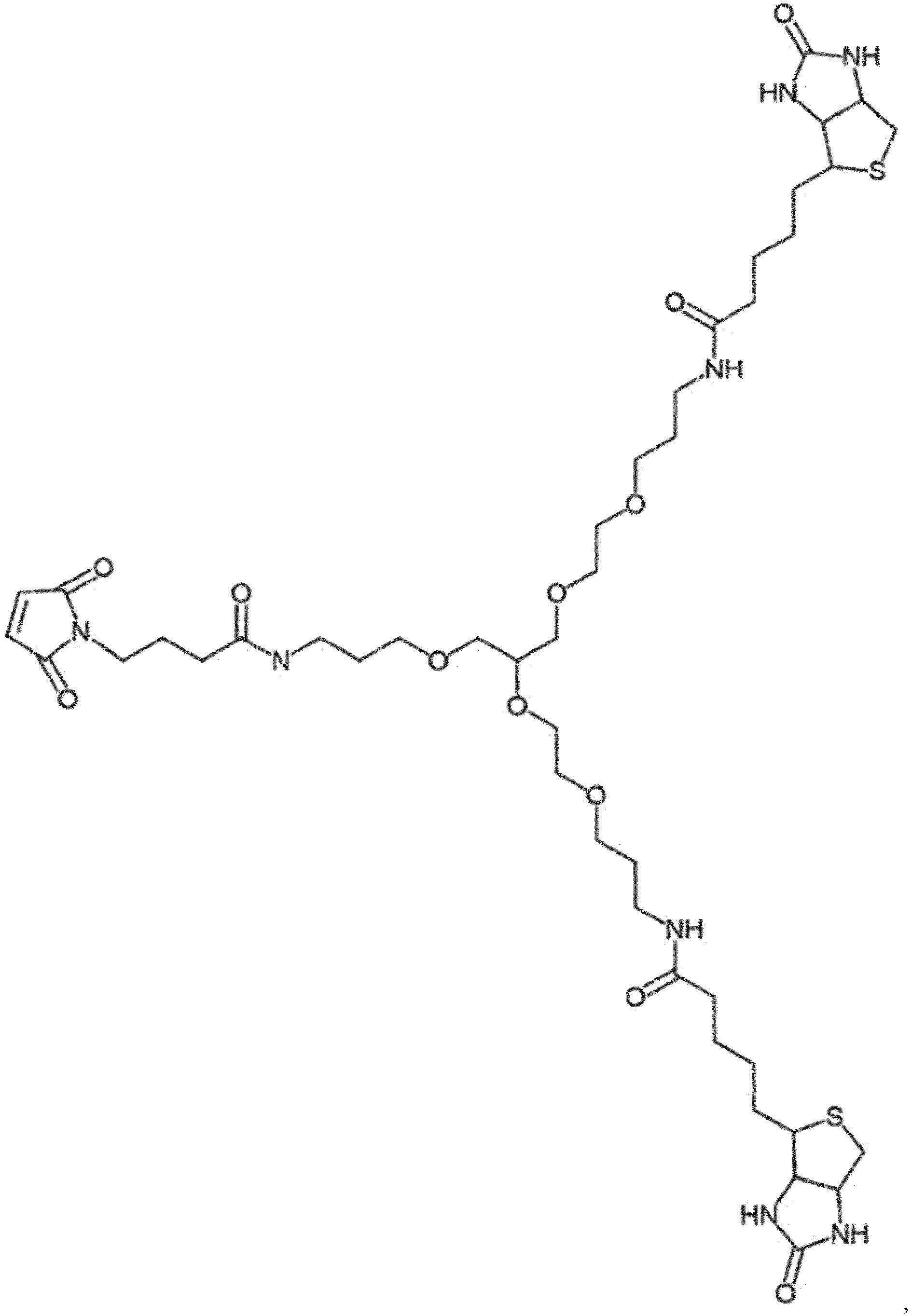

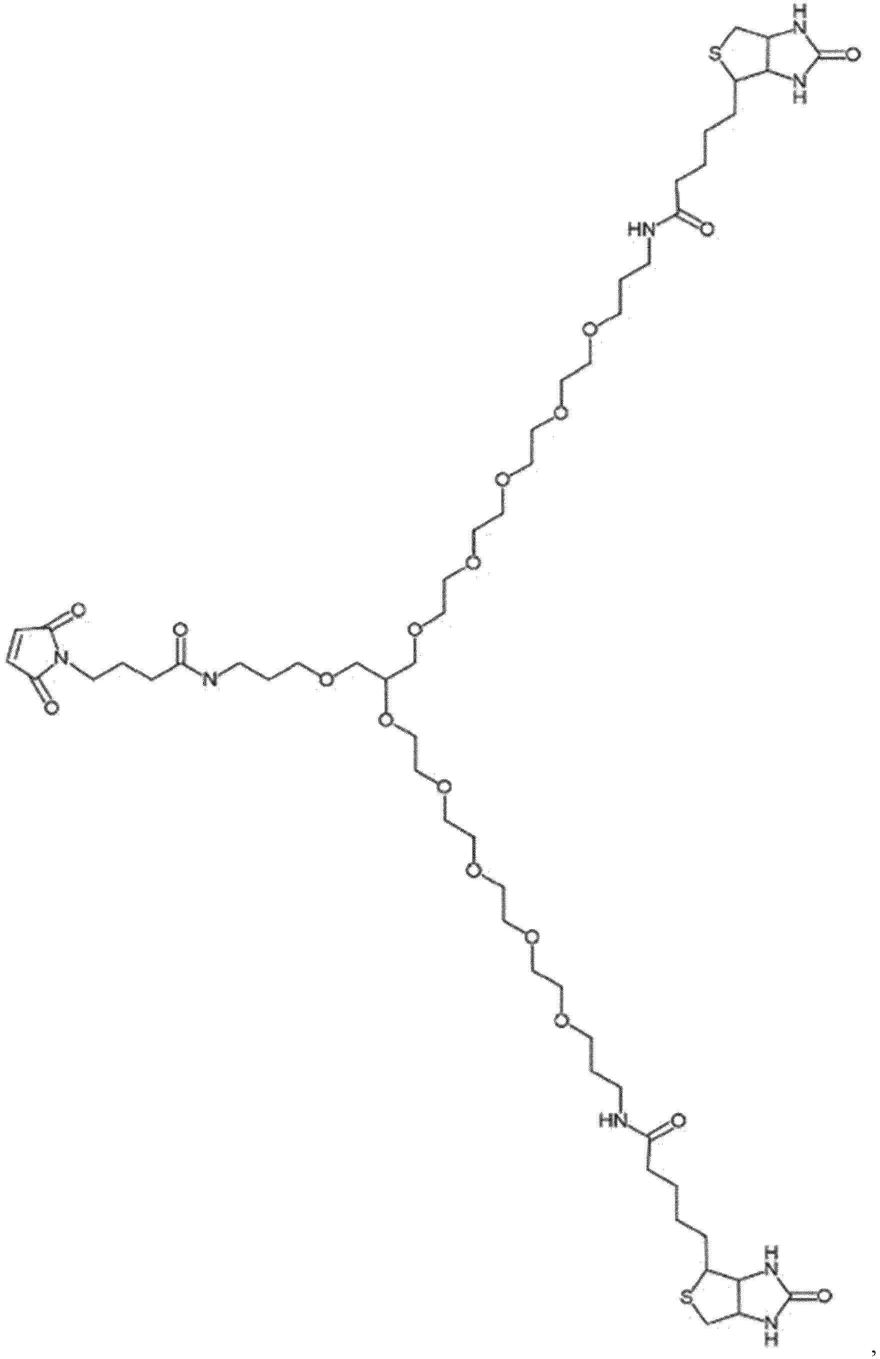

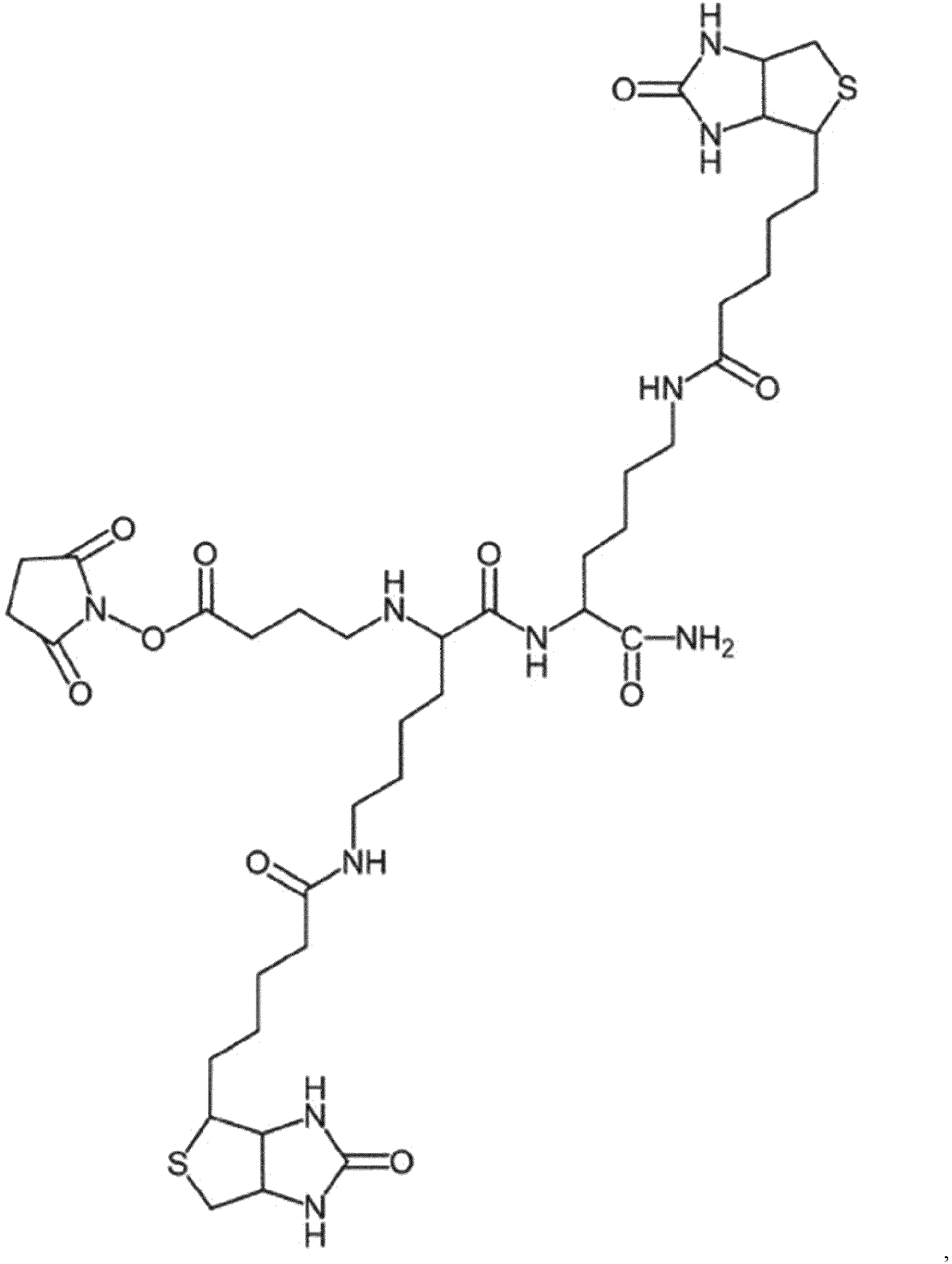

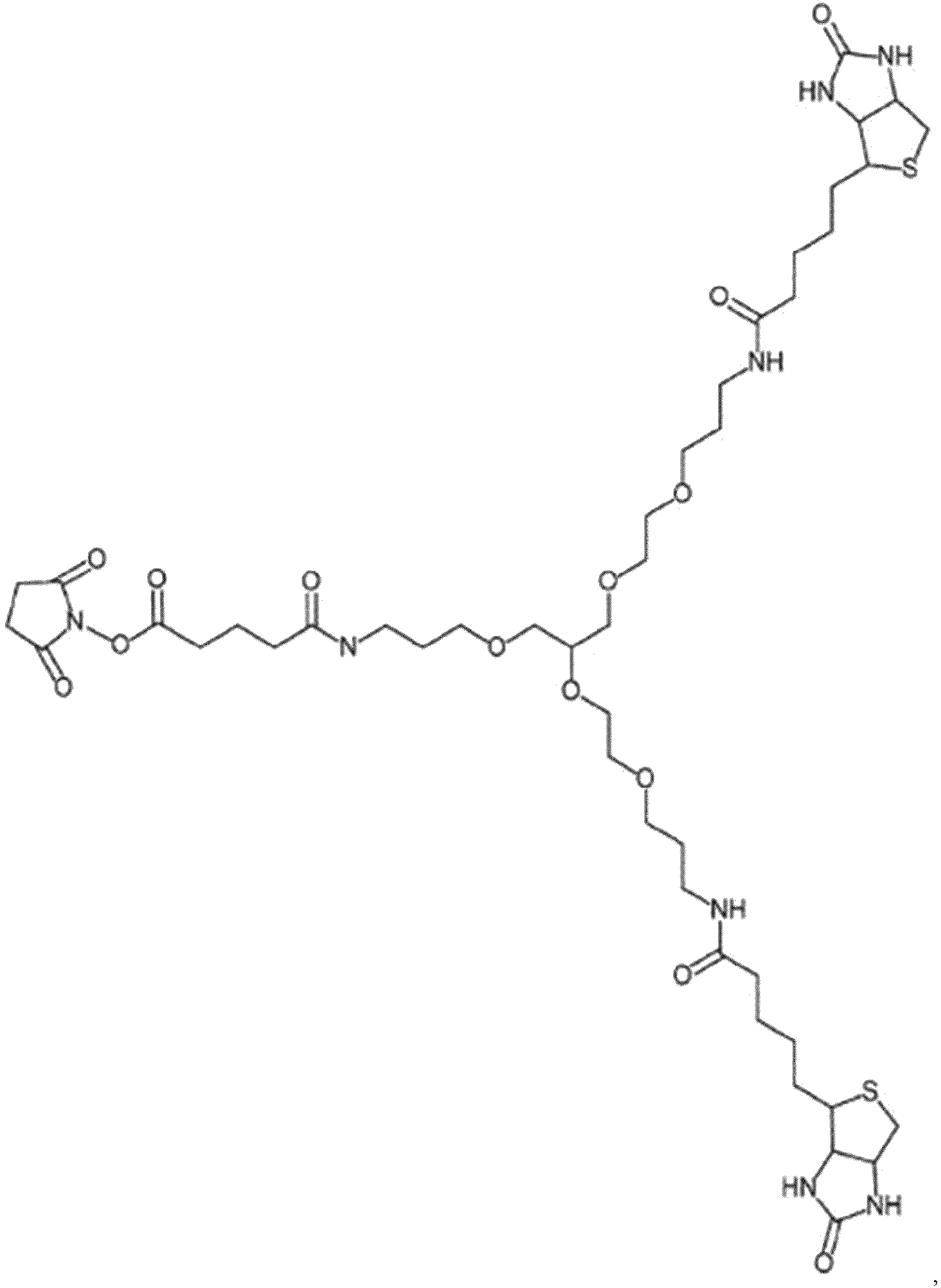

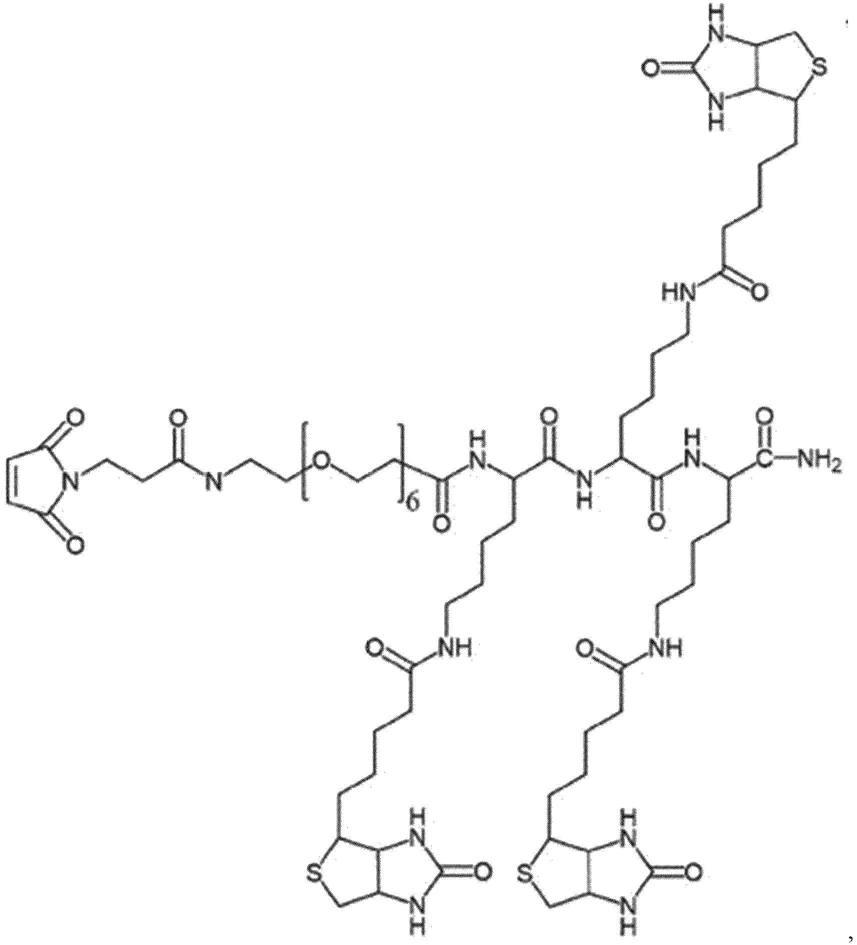

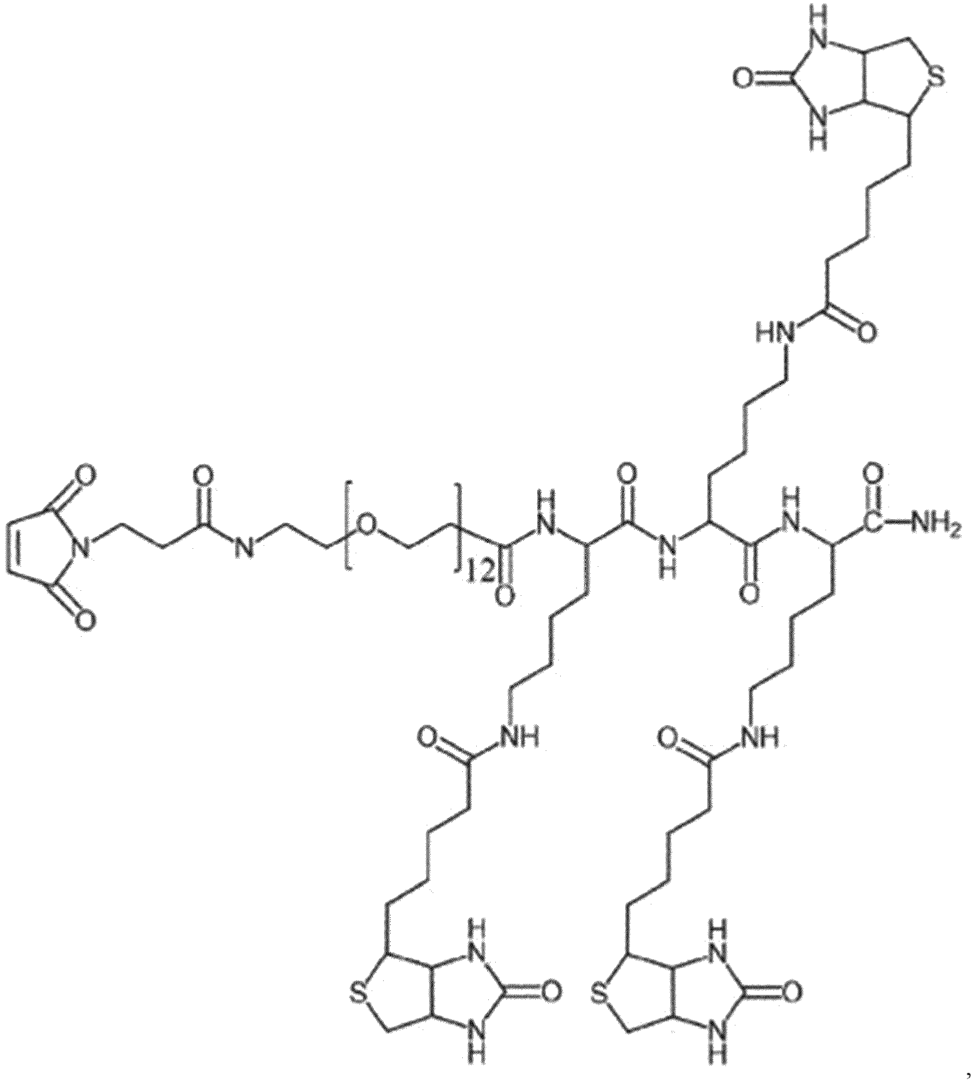

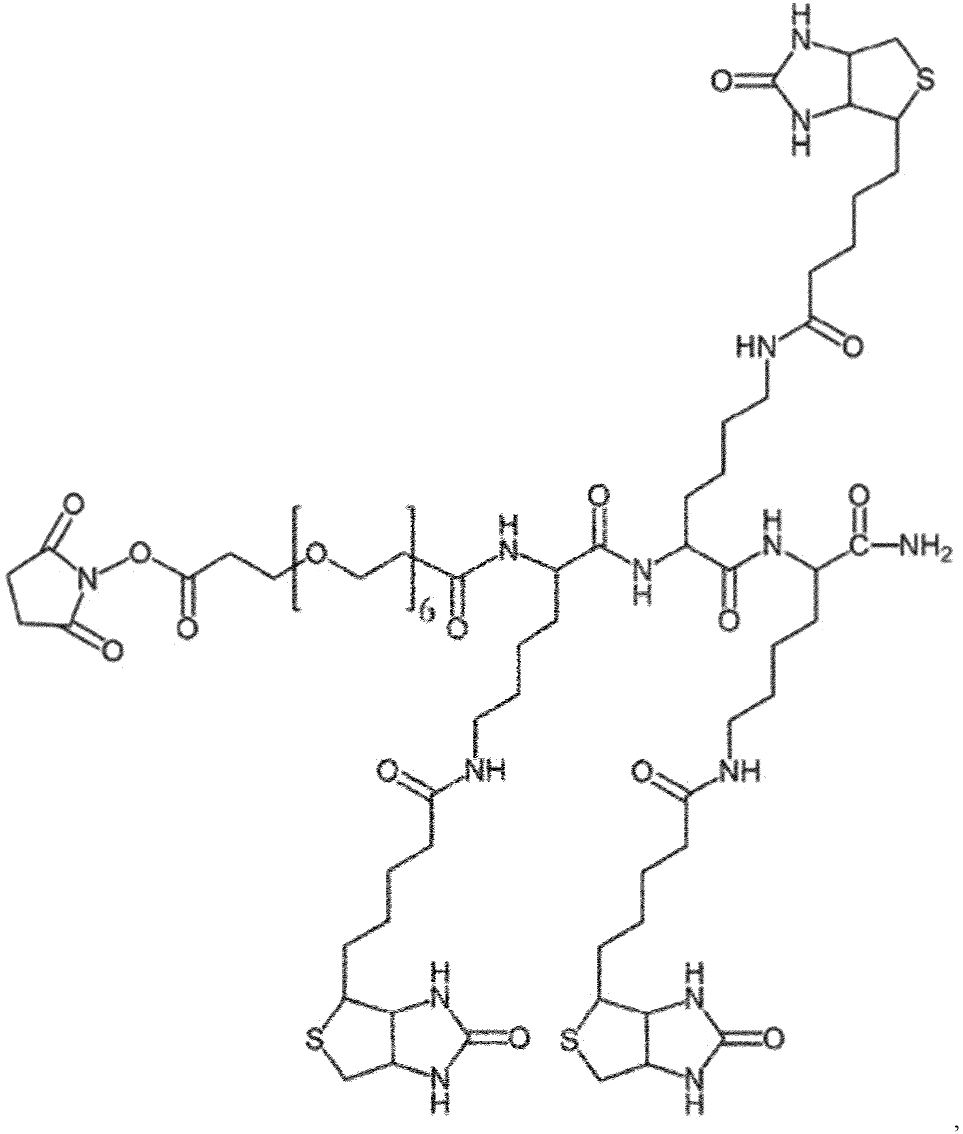

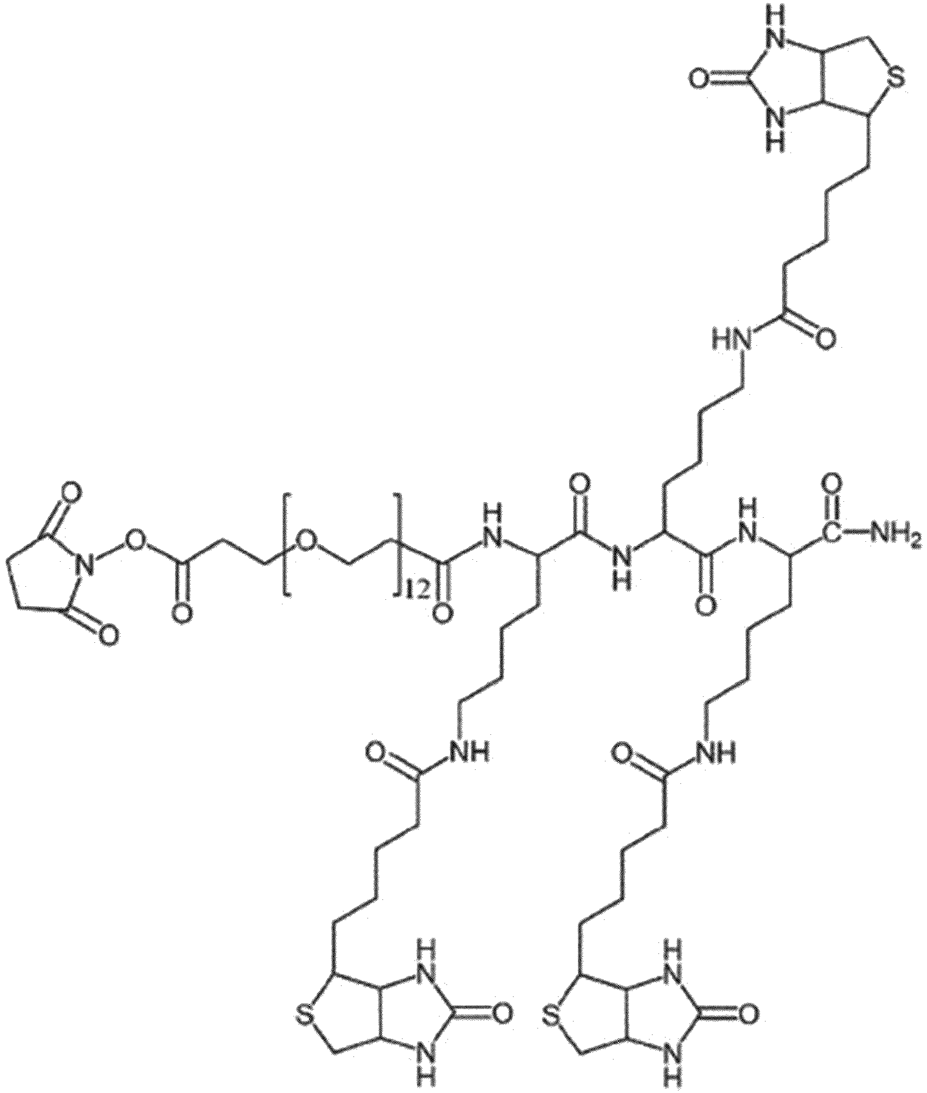

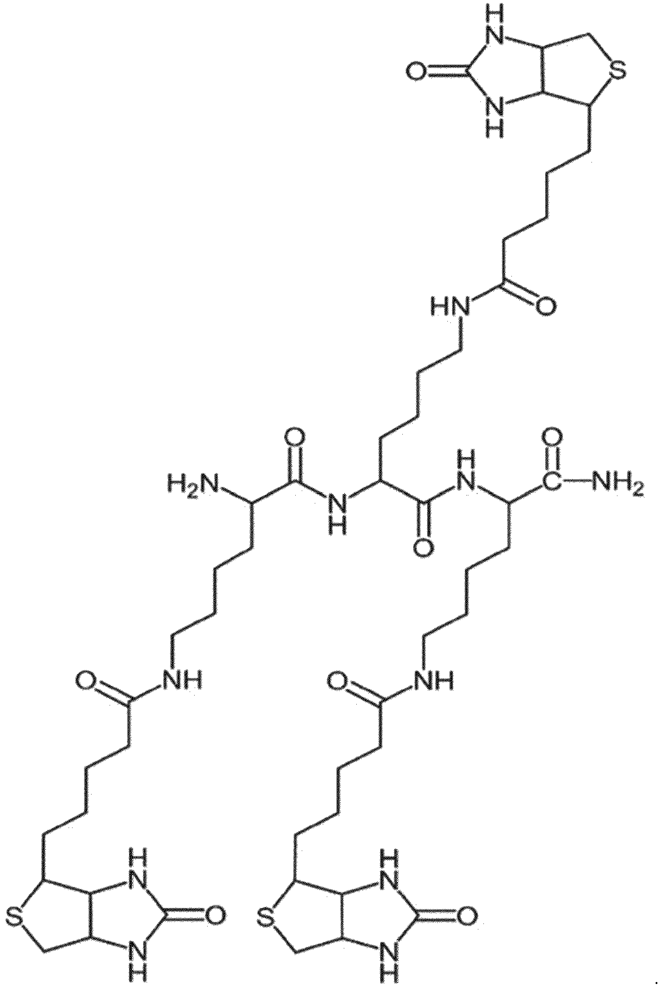

31. A composition orally bioavailable in mammals, the composition comprising the physiologically active substance according to any one of Claims 1 through 30.

32. A kit, comprising:
a. the physiologically active substance bonded to a biotin moiety according to any one of Claims 1 through 30; and,
b. instructional data for administering the physiologically active substance to a human.

33. A method for treating illness in a human, the method including administering to a human the physiologically active substance bonded to a biotin moiety according to any one of Claims 1 through 30.

34. A biotin moiety represented by General Formula A below: 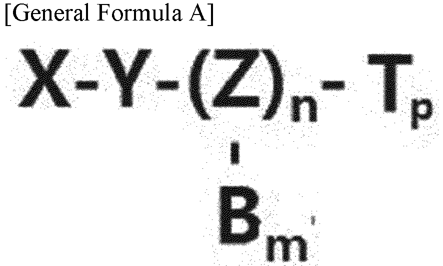 In General Formula A,
X is a functional group capable of bonding to a physiologically active substance, Y is a spacer,
Z is a bonding unit,
B can be represented by the following Chemical Formula A-1, 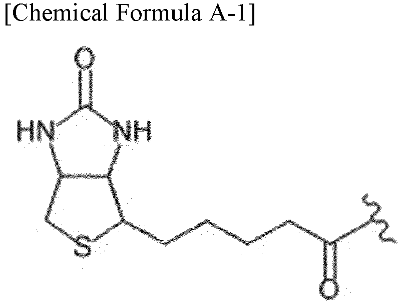
Z is connected to the 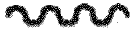 of chemical formula A-1,
T is a terminal group,
m is an integer from 1 to 10,
n is 0 or an integer from 1 to 10, and when n=0, Y is directly bonded to B or T,
and p is an integer of 0 to 1.

35. The biotin moiety of Claim 34, wherein X is selected from a group comprised of maleimide, succinimide, N-hydroxysuccinimide, succinimidyl succinate, succinimidyl glutarate, succinimidyl methyl ester, succinimidyl pentyl ester, Succinimidyl carbonate, p-nitrophenyl carbonate, aldehyde, amine, thiol, oxyamine, iodoacetamide, aminoxyl, hydrazide, hydroxy, propionate, pyridyl, alkyl halide, vinylsulfone, carboxyl, Hydrazide, halogen acetamide, C₂₋₅ alkynyl, C₆₋₂₀ aryldisulfide, C₅₋₂₀ heteroaryldisulfide, isocyanate, thioester, iminoester, and derivatives thereof.

36. The biotin moiety of Claim 34, wherein the X is maleimide, N-hydroxysuccinimide, aldehyde or amine.

37. The biotin moiety of Claim 34,
wherein the Y is absent, a substituted or unsubstituted linear or branched C₁₋₅₀ alkylene, a substituted or unsubstituted linear or branched C₁₋₅₀ heteroalkylene, substituted or unsubstituted C₆₋₅₀ arylene, or substituted or unsubstituted C₆₋₅₀ heteroarylene,
and if substituted, includes at least one selected from a group comprised of =O, - C(O)NH₂, -OH, -COOH, -SH, =NH and -NH₂.

38. The biotin moiety of Claim 34, wherein the Y is a substituted linear or branched C₁₋₅₀ heteroalkylene, and includes at least one -C(O)-.

39. The biotin moiety of Claim 34,
wherein the Y is -(C(O))_{q}-(CH₂)ᵣ-(C(O)NHₛ-(CH₂)ᵣ-(OCH₂CH₂)ₜ-(C(O))_{q}-,
q, r, s and t are independently selected,
q and S are 0 or 1,
r is an integer of 1 to 20,
and t is an integer of 0 to 20.

40. The biotin moiety of Claim 34, wherein the Y is -(CH₂)ᵣC(O)NHNH- and r is an integer of 1 to 20.

41. The biotin moiety of Claim 34, wherein the Y includes -C(O)-.

42. The biotin moiety of Claim 34, wherein the Y includes -C(O)NH-.

43. The biotin moiety of Claim 34, wherein the Zis any one of the following, and each may be independently selected:
A) forms an amino acid or a derivative thereof together with X or separately from X; or
B) is a substituted or unsubstituted linear or nonlinear C₁₋₅₀ heteroalkylene, and when substituted, includes at least one selected from a group comprised of =O, -C(O)NH₂, - OH, -COOH, -SH, =NH and -NH₂.

44. The biotin moiety of Claim 34,
wherein the Z is connected through B and -NH-.

45. The biotin moiety of Claim 34,
wherein the Z is a hydrophilic amino acid or a derivative thereof.

46. The biotin moiety of Claim 34,
wherein the Z is selected from a group comprised of lysine, arginine, histidine, glutamine, asparagine, threonine, cysteine, serine and derivatives thereof.

47. The biotin moiety of Claim 34,
wherein the Z includes at least one glycerol, and at least one polyethylene glycol or a bond thereof.

48. The biotin moiety of Claim 34, wherein the Z includes site, 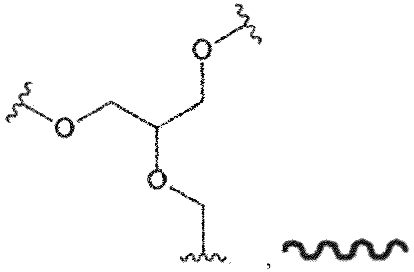 represents a binding at least one 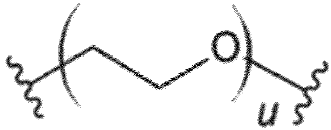 is bonded to the binding site, and u is an integer of 1 to 20.

49. The biotin moiety of Claim 34, wherein the -(CH₂)₃NH- is further bonded to the

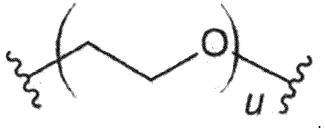

50. The biotin moiety of Claim 34, wherein the T is selected from a group comprised of
amine, C₁₋₈ alkyl, C₁₋₈ alkenyl, halo, hydroxy, thiol, sulfonic acid, carboxyl, phenyl, benzyl, aldehyde, azide, cyanate, isocyanate, thiocyanate, isothiocyanate, nitrile and phosphonic acid.

51. The biotin moiety of Claim 34, wherein the T is an amine.

52. The biotin moiety of Claim 34, selected from a group comprised of the following chemical formulae:

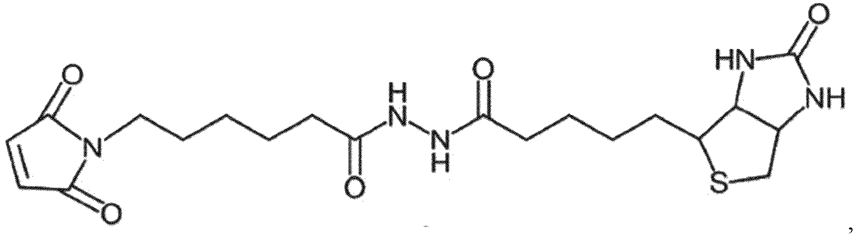

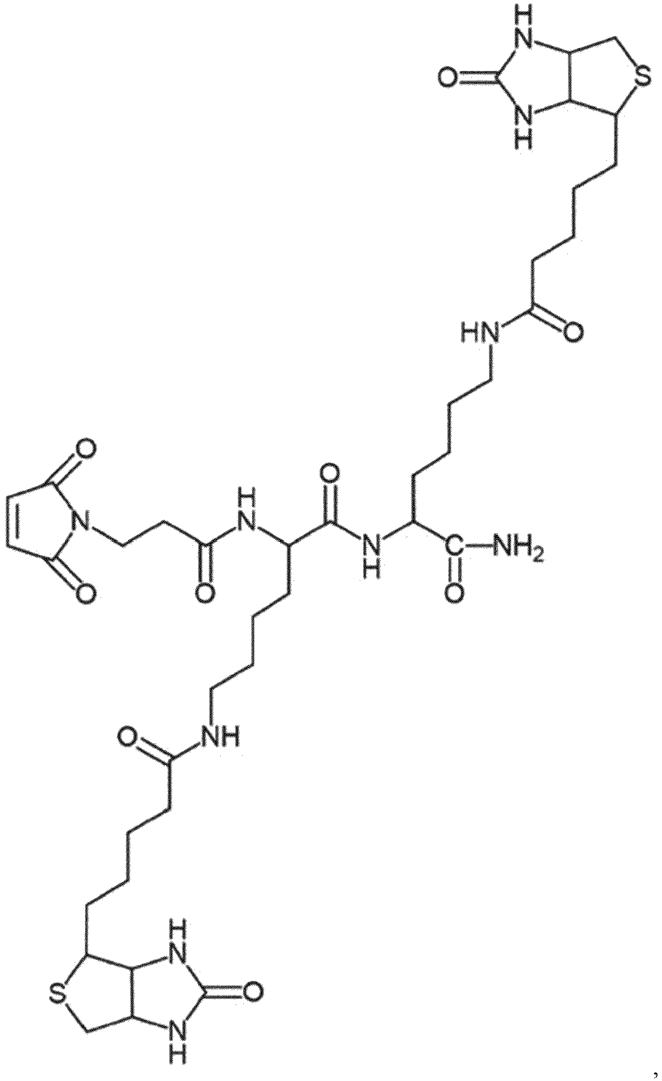

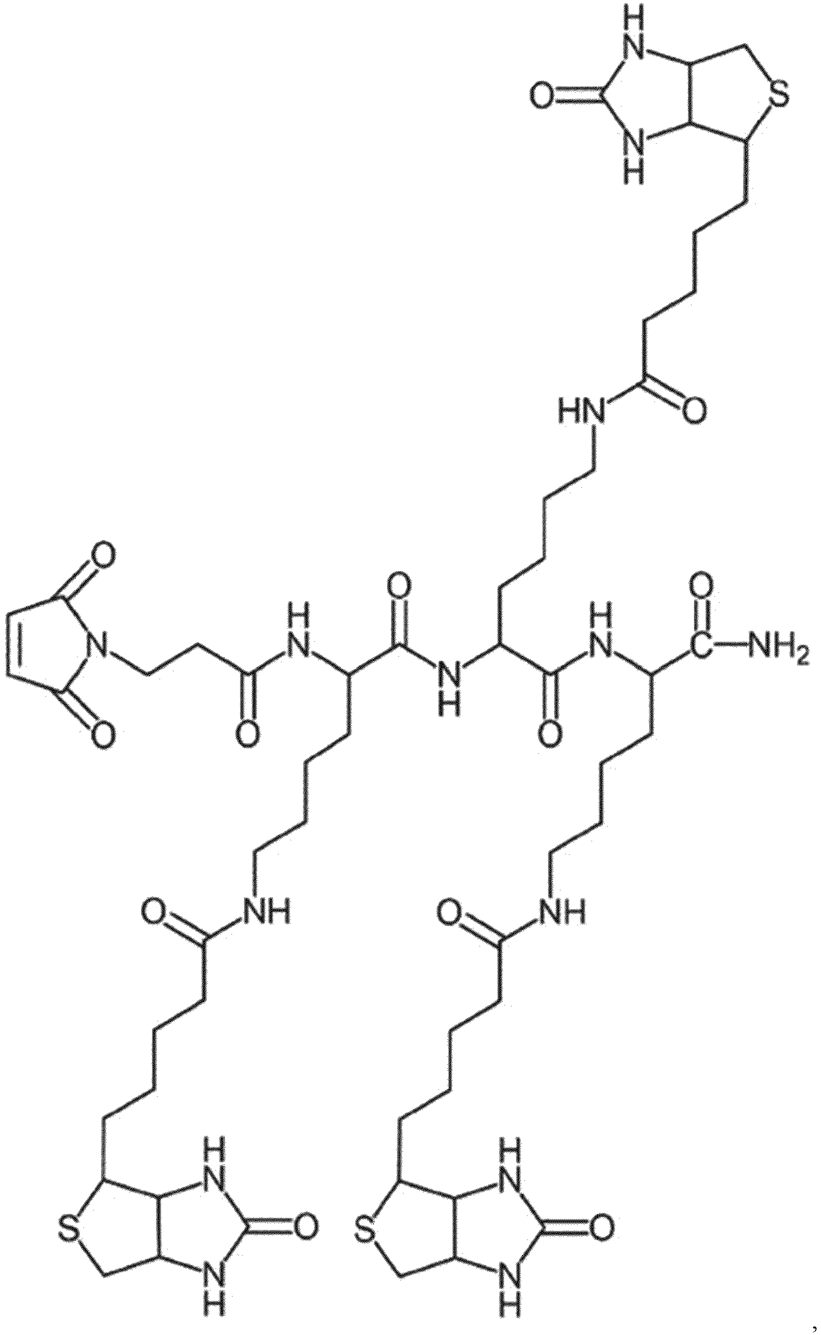

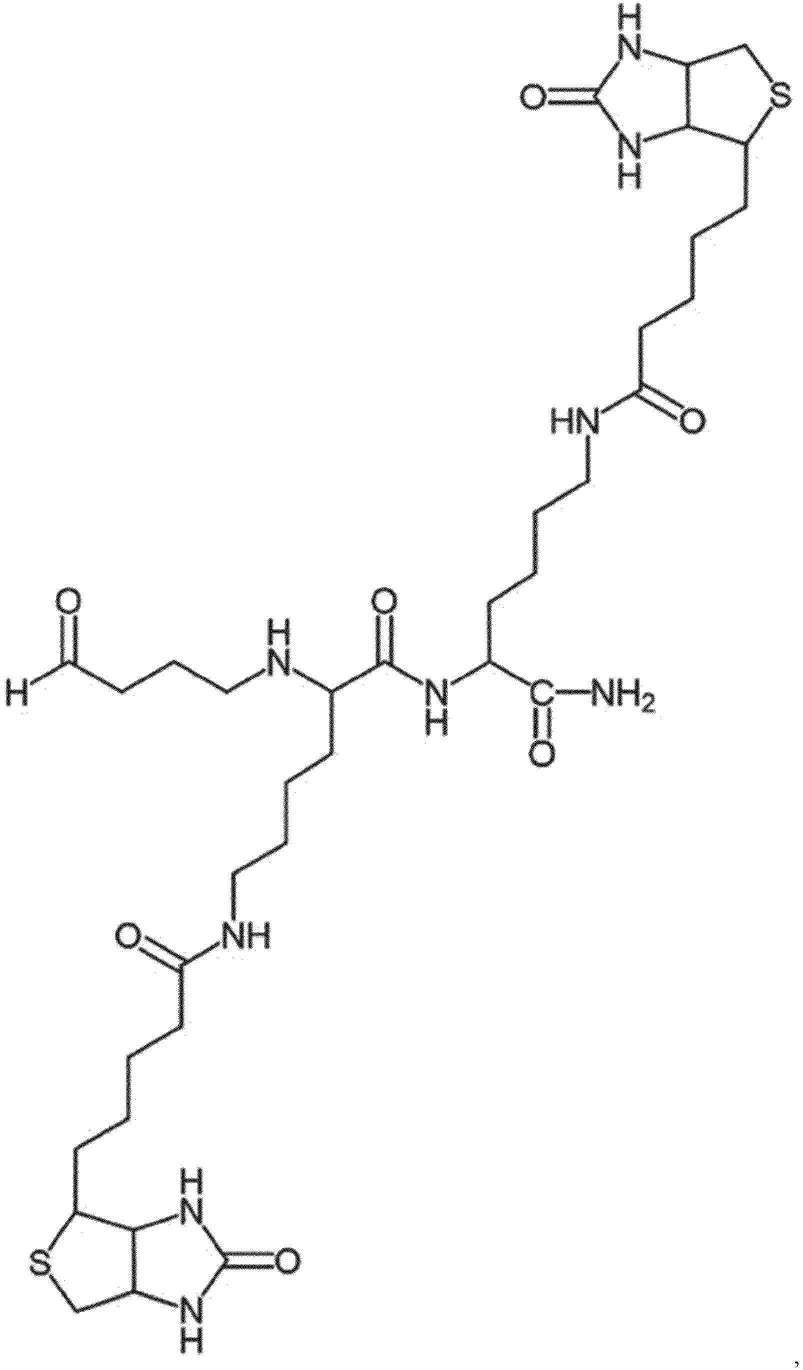

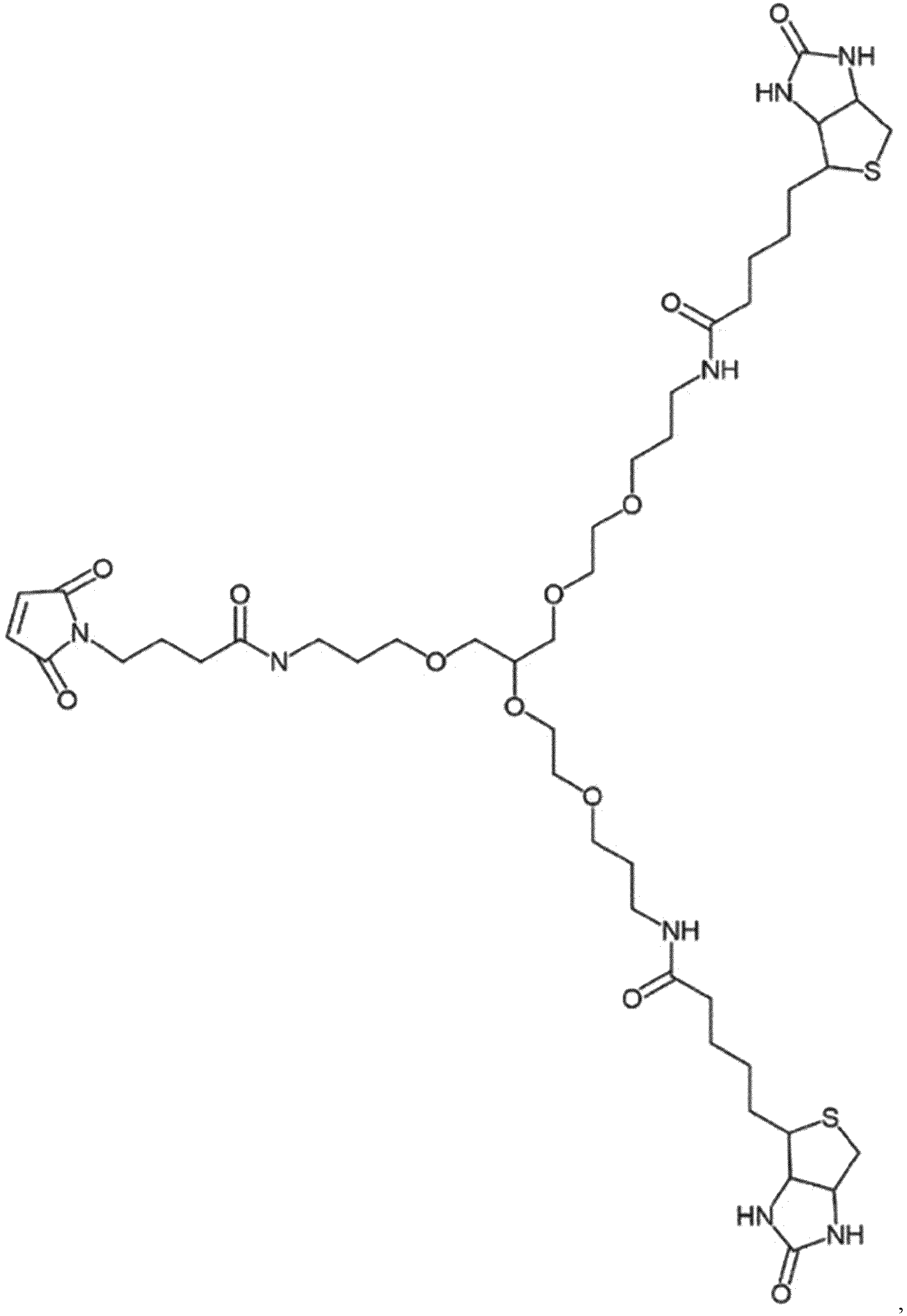

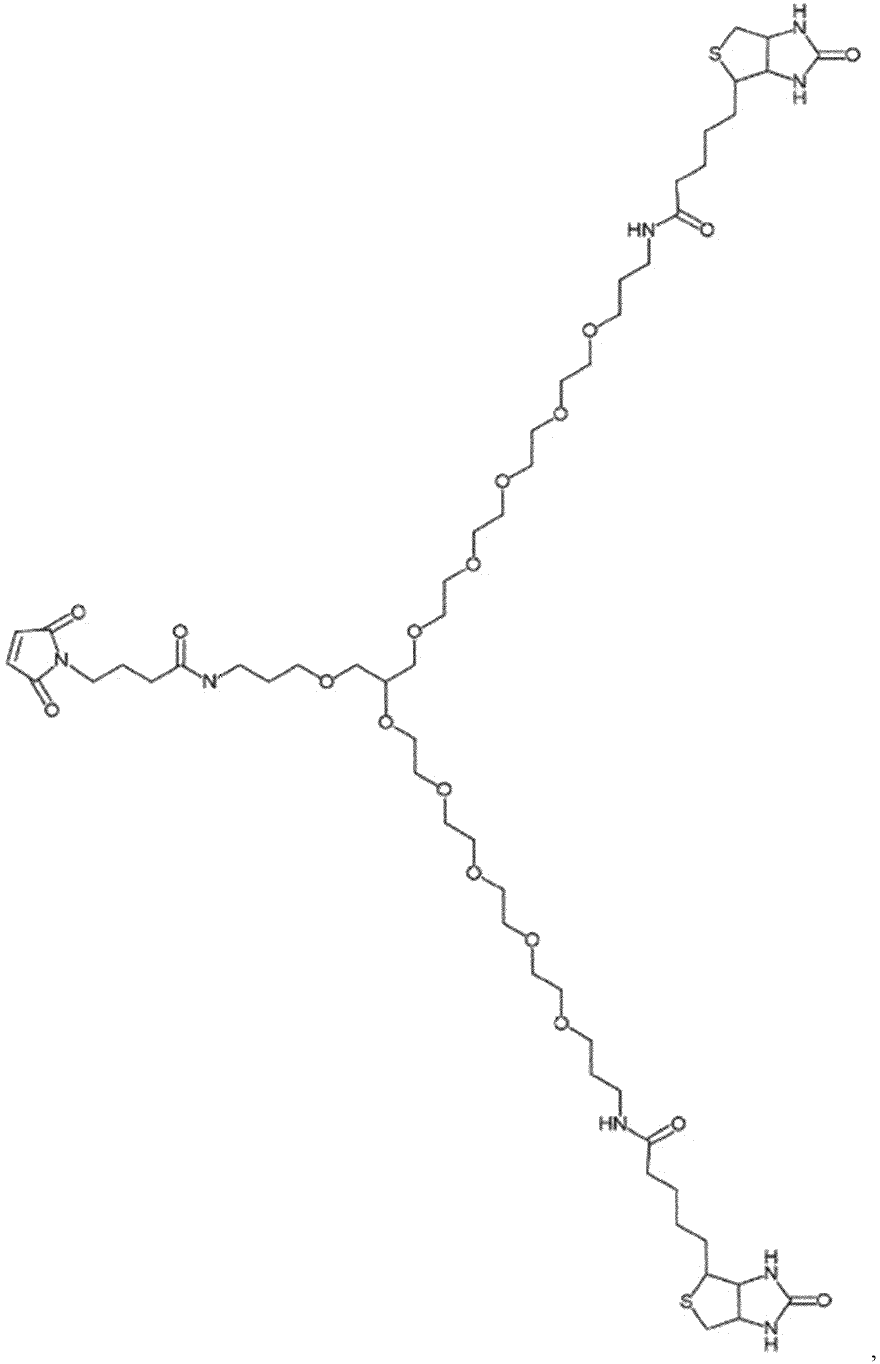

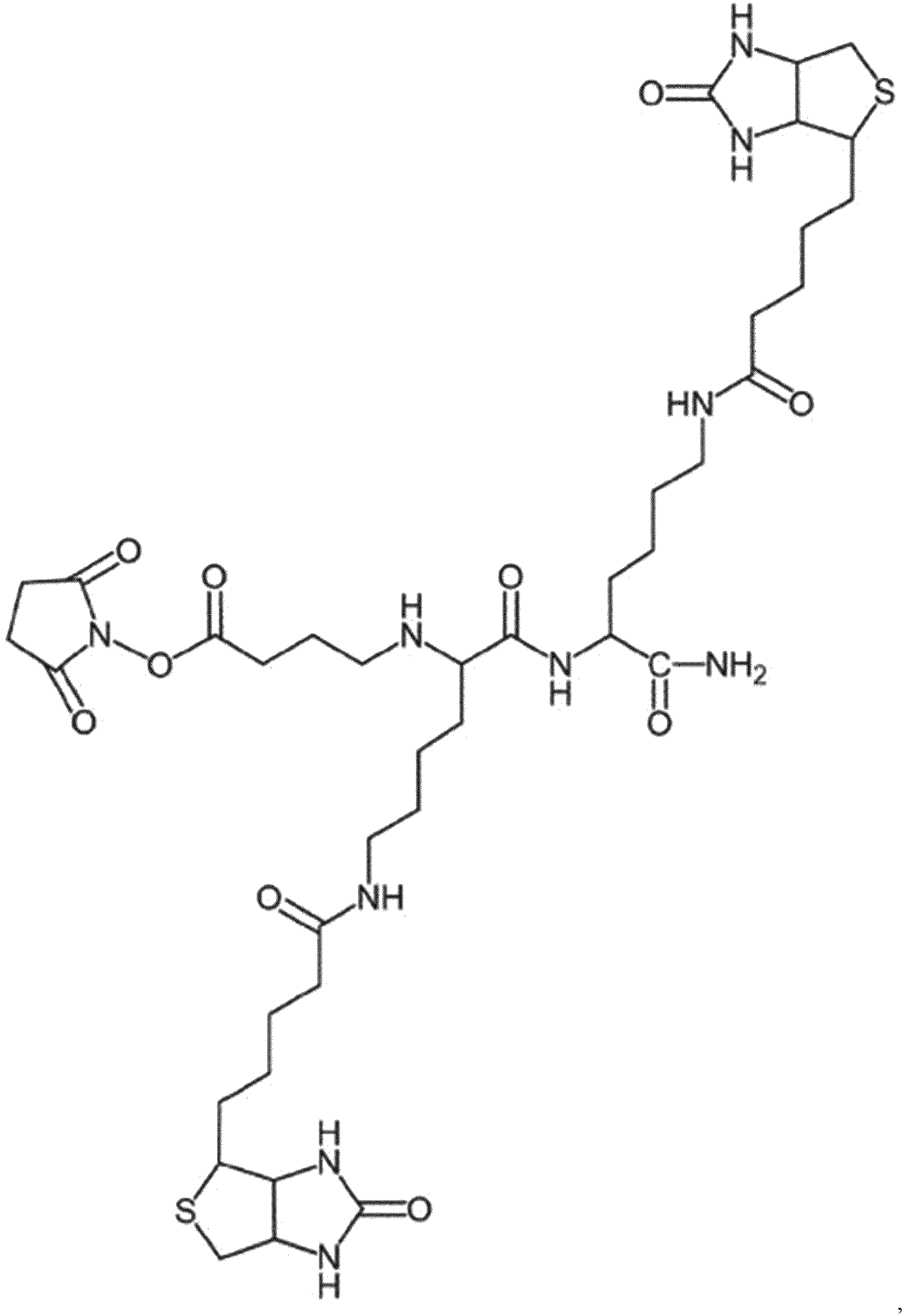

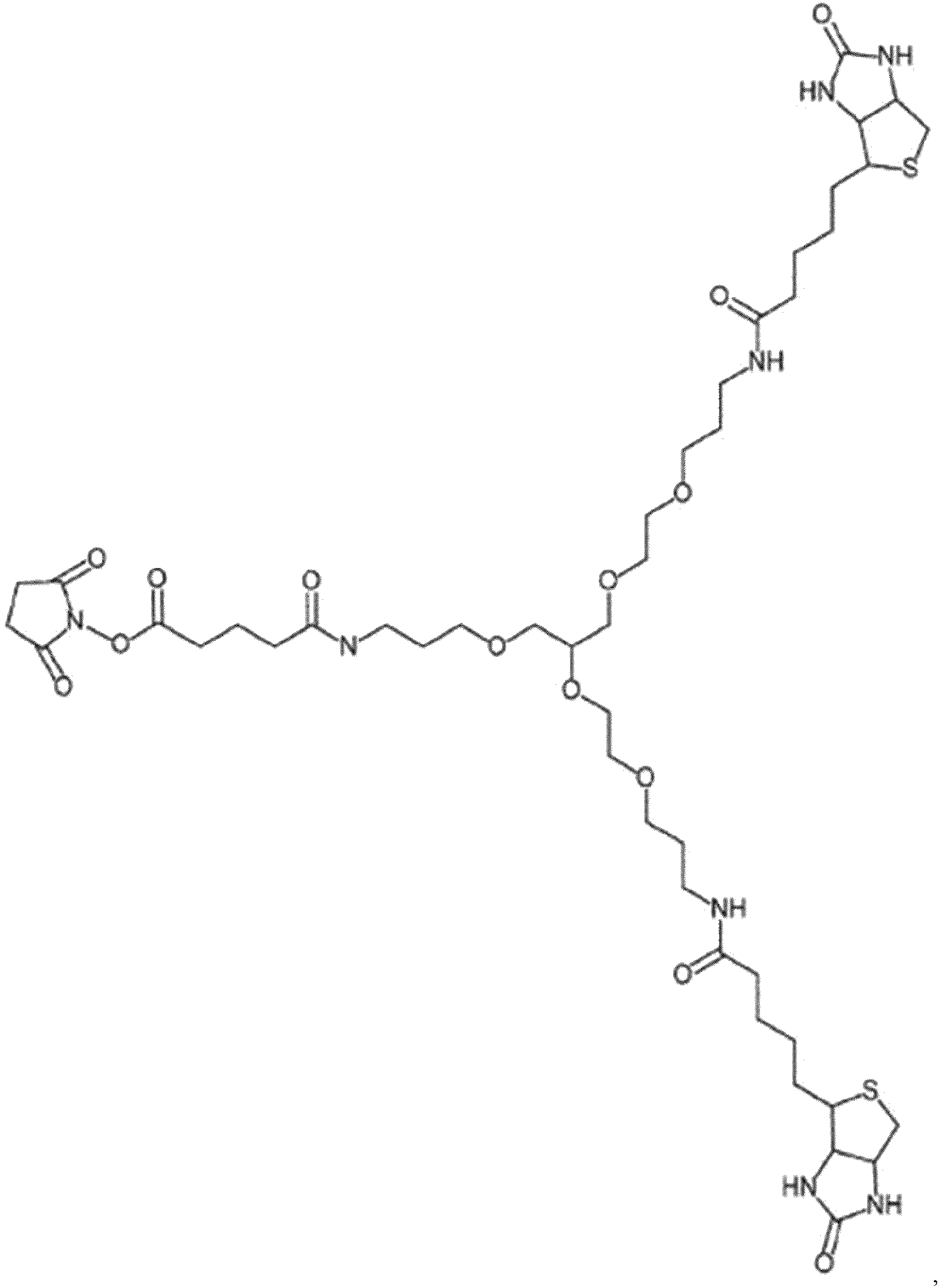

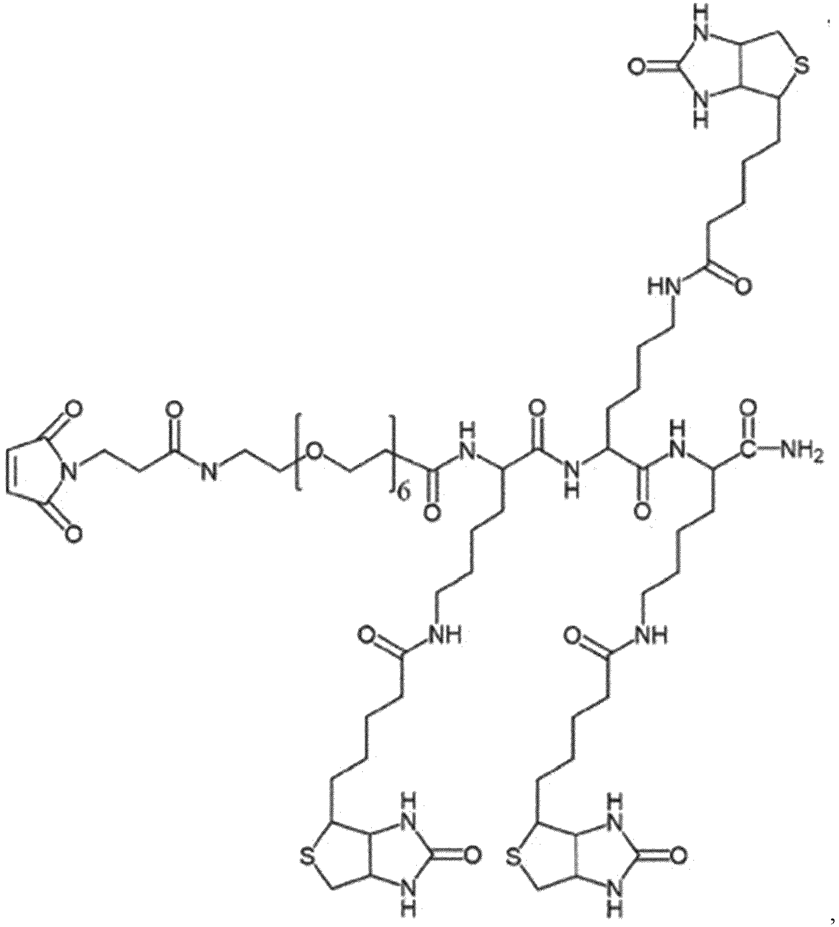

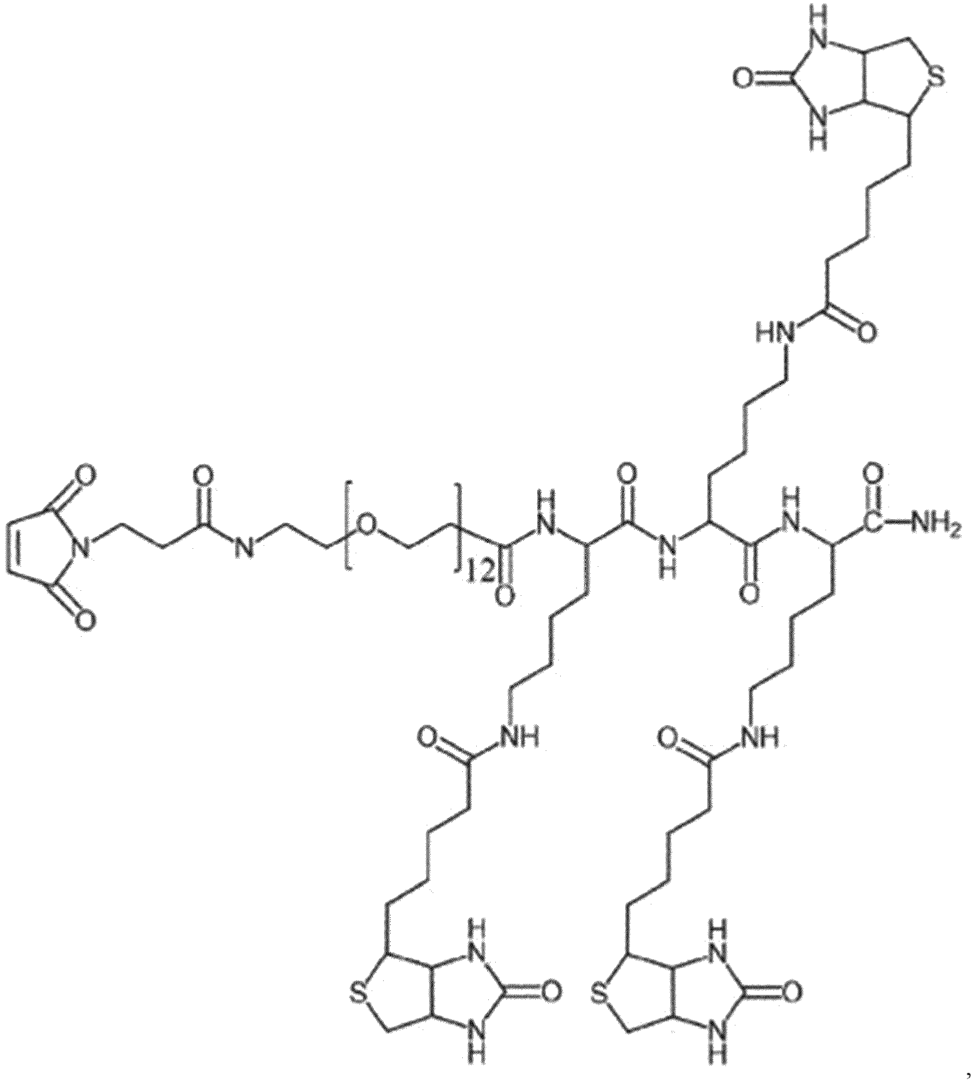

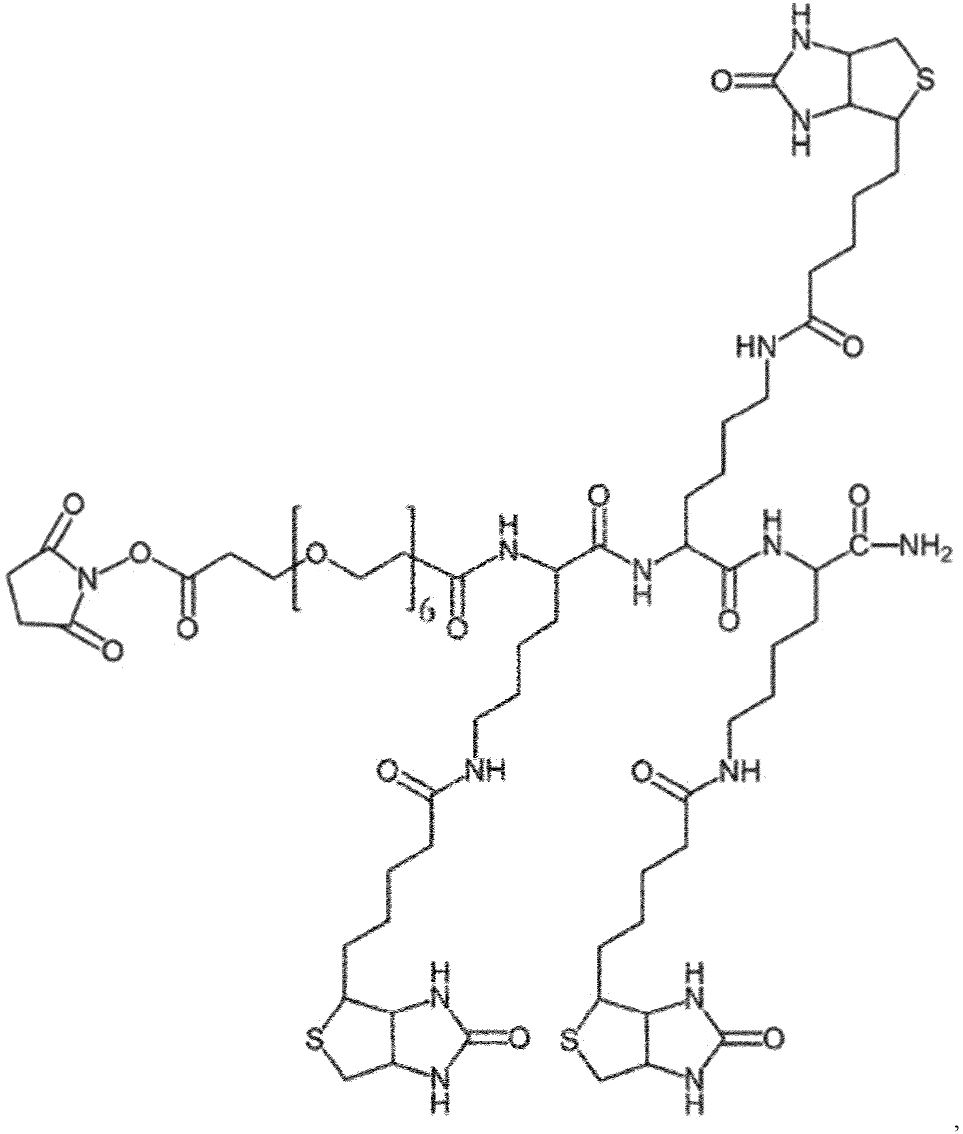

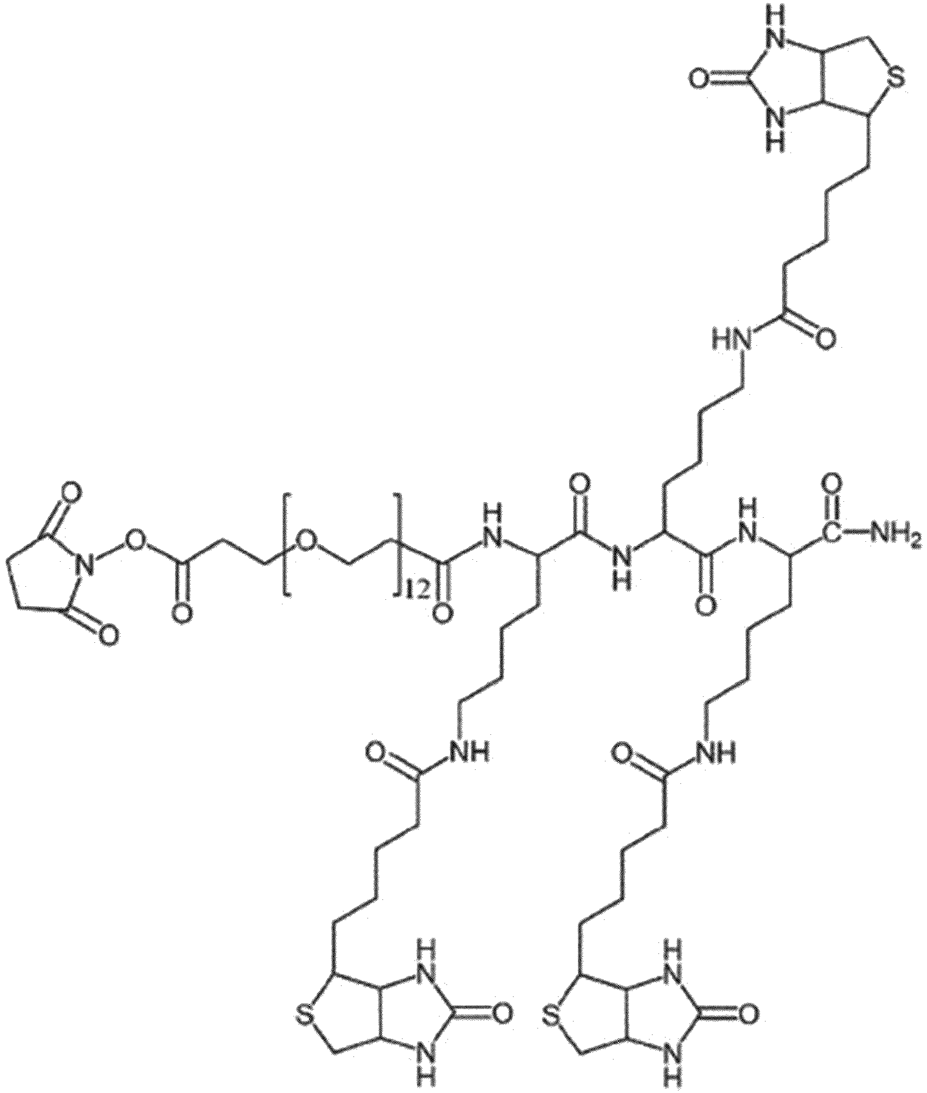

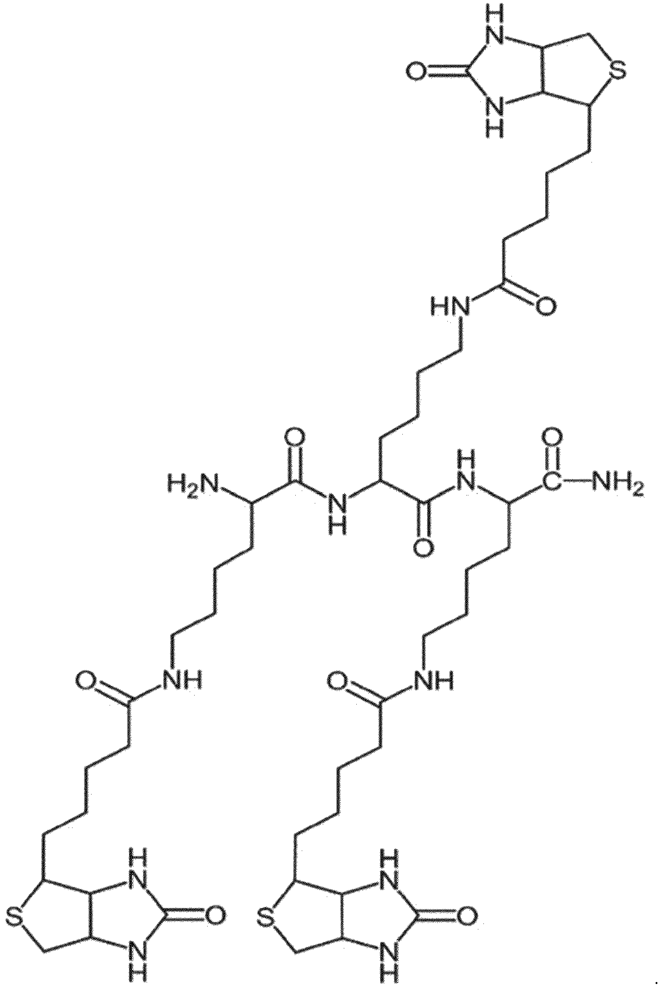

53. A composition orally bioavailable in mammals, the composition comprising biotin moiety according to any one of Claims 34 through 52.
